# EUROPEAN PATENT SPECIFICATION

(11) **EP 2 568 979 B1**
(45) Date of publication and mention of the grant of the patent: **05.04.2017**
(21) Application number: 11781133.1
(22) Date of filing: 10.05.2011
(51) Int. Cl.: A61K 31/337, A61K 47/50, A61K 47/30, A61K 9/24, A61K 9/16, A61P 35/00

(54) **RATIOMETRIC COMBINATORIAL DRUG DELIVERY**
RATIOMETRISCHE KOMBINATORISCHE ARZNEIMITTELVERABREICHUNG
ADMINISTRATION DE MÉDICAMENTS COMBINATOIRE RATIOMÉTRIQUE

(30) Priority: 10.05.2010 US 333138 P
(43) Date of publication of application: 20.03.2013
(73) Proprietor: The Regents of the University of California, Oakland, CA 94607-5200 (US)
(72) Inventor: ZHANG, Liangfang, San Diego CA 62130 (US); ARYAL, Santosh, San Diego CA 92122 (US); HU, Che-ming, La Jolla CA 92092 (US)
(74) Representative: Weickmann & Weickmann PartmbB
(86) International application number: PCT/US2011/035903
(87) International publication number: WO 2011/143201

(56) References cited:
- WO-A1-2008/124632
- WO-A2-2008/051565
- US-A1- 2003 147 945
- US-A1- 2006 046 967
- SONG ET AL: "PLGA nanoparticles simultaneously loaded with vincristine sulfate and verapamil hydrochloride: Systematic study of particle size and drug entrapment efficiency", INTERNATIONAL JOURNAL OF PHARMACEUTICS, ELSEVIER BV, NL, vol. 350, no. 1-2, 28 January 2008 (2008-01-28), pages 320-329, XP022435790, ISSN: 0378-5173, DOI: 10.1016/J.IJPHARM.2007.08.034
- LAMMERS T ET AL: "Simultaneous delivery of doxorubicin and gemcitabine to tumors in vivo using prototypic polymeric drug carriers", BIOMATERIALS, ELSEVIER SCIENCE PUBLISHERS BV., BARKING, GB, vol. 30, no. 20, 1 July 2009 (2009-07-01), pages 3466-3475, XP026104378, ISSN: 0142-9612, DOI: 10.1016/J.BIOMATERIALS.2009.02.040 [retrieved on 2009-03-21]
- SONG X R ET AL: "Reversion of multidrug resistance by co-encapsulation of vincristine and verapamil in PLGA nanoparticles", EUROPEAN JOURNAL OF PHARMACEUTICAL SCIENCES, ELSEVIER, AMSTERDAM, NL, vol. 37, no. 3-4, 28 June 2009 (2009-06-28), pages 300-305, XP026160257, ISSN: 0928-0987, DOI: 10.1016/J.EJPS.2009.02.018 [retrieved on 2009-03-09]
- SOMA C E; DUBERNET C; BENTOLILA D; BENITA S; COUVREUR P: "Reversion of multidrug resistance by co-encapsulation of doxorubicin and cyclosporin A in polyalkylcyanoacrylate nanoparticles.", BIOMATERIALS, vol. 21, 1999, - 1999, pages 1-7, XP002699520,

## Description

### CROSS-REFERENCE TO RELATED APPLICATIONS

This application claims priority from U.S. Provisional Application Serial No. 61/333,138 filed on May 10, 2010.

### STATEMENT REGARDING FEDERALLY SPONSORED RESEARCH OR DEVELOPMENT

This invention was made with Government support under National Institutes of Health Grant No. U54CA119335 and National Science Foundation Grant No. CMMI-1031239. The Government has certain rights in the invention.

### FIELD

The present teachings relate to nanoparticles, drug conjugates, and controlled release of drug conjugates from the nanoparticles. Methods of making the nanoparticles and drug conjugates are contemplated.

### INTRODUCTION

Combinatorial drug delivery, or combination therapy, refers to the use of multiple drugs to treat diseases or disorders in patients such as various cancers. For example, gemicitabine and paclitaxel are concurrently administered for treating breast cancer; docetaxel and carboplatin for lung cancer; and doxorubicin and ifosfamide for soft tissue sarcoma. Combination chemotherapy is usually more effective than individual chemotherapy as drugs with similar mechanisms act synergistically to enhance therapeutic efficacy whereas drugs with different mechanisms give cancer cells a higher hurdle in developing resistance. However, because of the different therapeutic indices, cellular uptake mechanisms, and *in vivo* clearance time among drugs, it is difficult to ensure that the tumors receive the optimal dosage of each therapeutic agent. Compositions and methods for precisely controlling the molar ratio among multiple drugs and their concentration taken up by the same target diseased cells would therefore be beneficial in optimizing combination chemotherapy regimens.

Nanoparticulate drug delivery systems have become increasingly attractive in systemic drug delivery because of their ability to prolong drug circulation half-life, reduce non-specific uptake, and better accumulate at the tumors through enhanced permeation and retention (EPR) effect. As a result, several therapeutic nanoparticles such as Doxil® and Abraxane® are used as the frontline therapies in clinics. But despite the advancement in nanoparticle drug delivery, most research efforts focus on single drug encapsulation. Several strategies have been employed to co-encapsulate multiple drugs into a single nanocarrier, including physical loading into the particle core (see, e.g., X. R. Song, et al. Eur J Pharm Sci 2009, 37, 300-305; C. E. Soma, et al. Biomaterials 2000, 21, 1-7), chemical conjugation to the particle surface (*see, e.g.,* L. Zhang, et al. ChemMedChem 2007, 2, 1268-1271), and covalent linkage to the polymer backbone prior to nanoparticle synthesis (*see, e.g.,* T. Lammers, et al. Biomaterials 2009, 30, 3466-3475; Y. Bae, et al. J Control Release 2007, 122, 324-330; N. Kolishetti, et al. Proc Natl Acad Sci U S A 2010, 107, 17939-17944). However, controlling the ratios of different types of drugs in the same nanoparticles remains a major challenge because of factors such as steric hindrance between the different drug molecules and the polymer backbones, batch-to-batch heterogeneity in conjugation chemistry, and variability in drug-to-drug and drug-to-polymer interactions.

Many pharmaceutically active agents possess multiple functional groups that are readily modified chemically. Several prodrugs have been synthesized based on these functional groups. For instance, gemcitabine has been acylated through its primary amine to improve its stability in blood; paclitaxel has been pegylated through its hydroxyl groups to improve its water solubility; and doxorubicin has been conjugated to polymers through hydrazone linkage to its ketonic group for nanoparticle encapsulation. It has been demonstrated that modifications through the aforementioned functional groups do not reduce the therapeutic efficacy of chemotherapy drugs as the modified drugs either retain their chemical activities or release the drug content intracellularly through pH- or enzyme-sensitive response.

Therefore, what is needed are compositions comprising ratiometrically controlled drug combinations, methods of synthesizing such ratiometric compositions, and combination therapy methods of using such compositions.

### SUMMARY

The invention relates to a method for nanoencapsulation of a plurality of drugs comprising:
separately linking each of the plurality of drugs with a corresponding polymer backbone with nearly 100% loading efficiency by forming the corresponding polymer backbone by ring opening polymerization beginning with the corresponding drug, wherein each of the corresponding polymer backbones has the same or similar physicochemical properties and has approximately the same chain length;
mixing the plurality of linked drugs and polymers at selectively predetermined ratios at selectively and precisely controlled drug ratios; and
synthesizing the mixed plurality of linked drugs and polymers into a nanoparticle.

The present teachings include ratiometric combinatorial drug delivery including nanoparticles, multi-drug conjugates, pharmaceutical compositions and methods of producing such compositions. In one embodiment, a nanoparticle is provided that includes an inner sphere and an outer surface, the inner sphere containing a combination of conjugated drugs connected by a stimuli-sensitive bond and having a predetermined ratio, wherein the conjugated drugs have the following formula:

(X-Y-Z)ₙ

wherein X is a pharmaceutically active agent, Y is a stimuli-sensitive linker, and Z is not X, and is a pharmaceutically active agent or hydrogen. In various aspects, n is an integer greater than or equal to 2. In another aspect, each individual conjugated drug of the combination comprises a predetermined molar weight percentage from about 1% to about 99%, provided that the sum of all individual conjugated drug molar weight percentages of the combination is 100%. In various aspects of the present embodiment, about 100% of drugs contained in the inner sphere are conjugated.

In various aspects, X can independently be an antibiotic, antimicrobial, growth factor, chemotherapeutic agent, and combinations thereof. For instance, X can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof. In various aspects, Z can independently be an antibiotic, antimicrobial, growth factor, chemotherapeutic agent, hydrogen, and combinations thereof. For instance, Z can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, pharmaceutically acceptable salts thereof, and hydrogen.

In various aspects, Y is a pH-sensitive linker. For instance, Y can include C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

In various aspects, the outer surface of the nanoparticle can include a cationic or anionic functional group.

In yet another aspect, the conjugated drug of the combination contained in the nanoparticle inner sphere has Formula I: 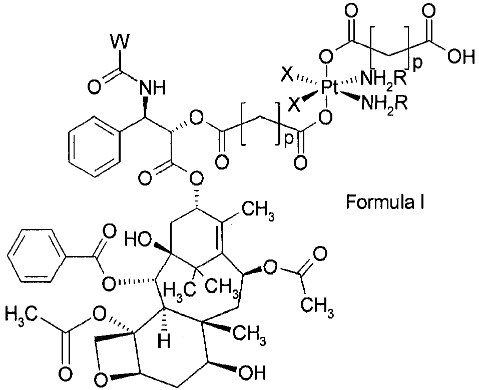 and pharmaceutically acceptable salts thereof, wherein 'p' is an integer from 1 to 10; 'X' is selected from the group consisting of halogen, sulfate, phosphate, nitrate, and water; 'W' is phenyl or tert-butyl oxy; and 'R' is hydrogen or alkyl. For instance, 'p' can be 3; 'X' can be chloride; 'W' can be phenyl and 'R' can be hydrogen.

In yet another aspect, the conjugated drug of the combination contained in the nanoparticle inner sphere has Formula II: 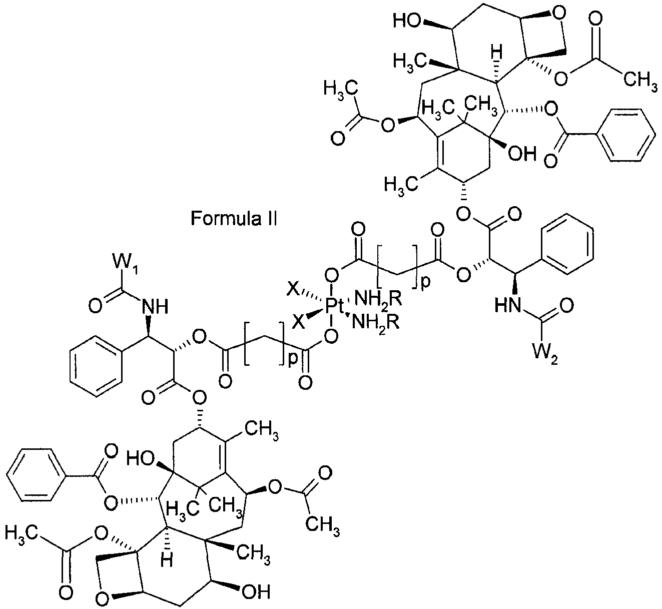 and pharmaceutically acceptable salts thereof, wherein 'p' is an integer from 1 to 10; 'X' is selected from the group consisting of halogen, sulfate, phosphate, nitrate, and water; 'W₁' and 'W₂' are independently selected from phenyl or tert-butyl oxy; and 'R' is hydrogen or alkyl. For instance, 'p' can be 3; 'X' is chloride; 'W₁' and 'W₂' can be phenyl and 'R' can be hydrogen.

In yet another aspect, the conjugated drug of the combination contained in the nanoparticle inner sphere has Formula III: 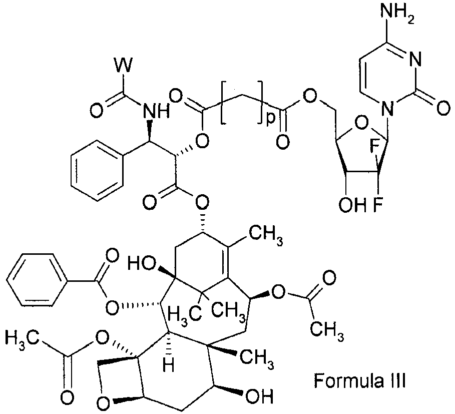 and pharmaceutically acceptable salts thereof, wherein 'p' is an integer from 1 to 10; and 'W' is slected from phenyl or tert-butyl oxy. For instance, 'p' can be 3; and 'W' can be phenyl.

In yet another aspect, the conjugated drug of the combination contained in the nanoparticle inner sphere has Formula IV: 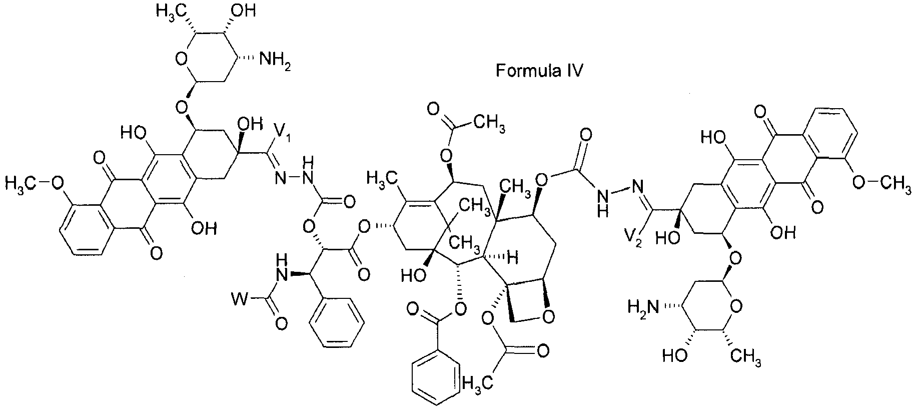 and pharmaceutically acceptable salts thereof, wherein 'W' is phenyl or tert-butyl oxy; and 'V₁' and 'V₂' are independently selected from -CH₃ or -CH₂OH. For instance,'W' can be phenyl; and 'V₁' and 'V₂' can be -CH₂OH.

In yet another aspect, the conjugated drug of the combination contained in the nanoparticle inner sphere has Formula V: 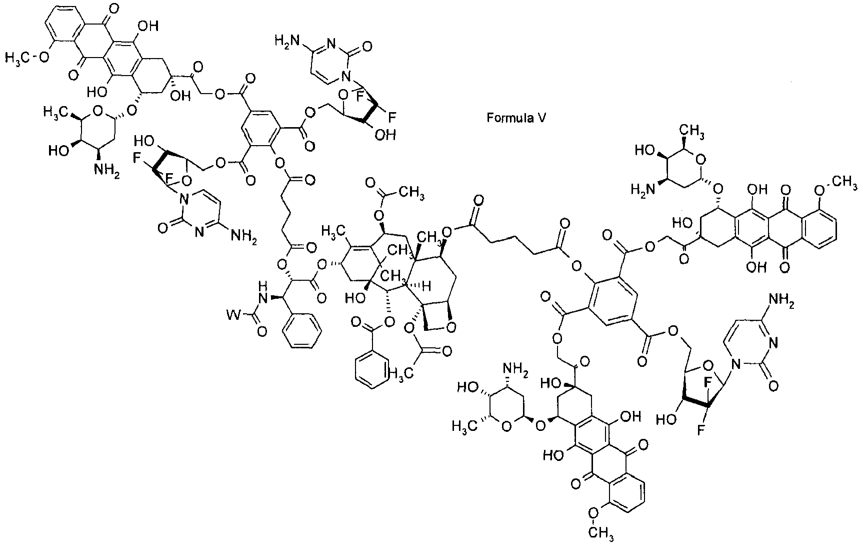 and pharmaceutically acceptable salts thereof, wherein 'W' is phenyl or tert-butyl oxy. For instance, 'W' can be phenyl.

In yet another aspect, the conjugated drug of the combination contained in the nanoparticle inner sphere has Formula VI: 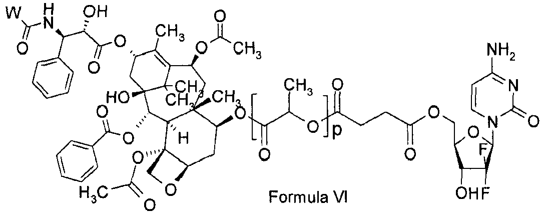 and pharmaceutically acceptable salts thereof, wherein 'p' is an integer from 5 to 20; and 'W' is phenyl or tert-butyl oxy. For instance, 'p' can be 10; and 'W' can be phenyl.

In yet another aspect, the conjugated drug of the combination contained in the nanoparticle inner sphere has Formula VII: 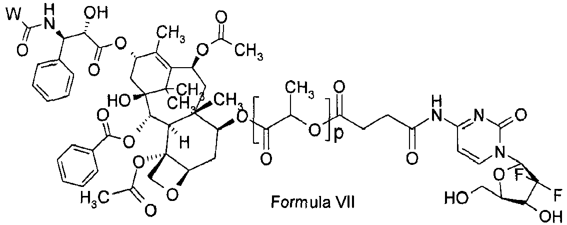 and pharmaceutically acceptable salts thereof, wherein 'p' is an integer from 5 to 20; and 'W' is phenyl or tert-butyl oxy. For instance, 'p' can be 10; and 'W' can be phenyl.

In various aspects, the nanoparticle is about 10 nm to about 10 µm in diameter, and in certain aspects about 30 nm to about 300 nm in diameter.

In another embodiment, a multi-drug conjugate is provided having the following formula:

X-Y-Z

wherein X and Z are pharmaceutically active agents independently selected from the group consisting of an antibiotic, antimicrobial, growth factor, and chemotherapeutic agent; and Y is a stimuli-sensitive linker, wherein the conjugate releases at least one pharmaceutically active agent upon delivery of the conjugate to a target cell.

In various aspects of the present embodiment, Y is a C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof. For instance, Y can be a C₃ straight chain alkyl or a ketone. In various aspects, the pharmaceutically active agent comprises an anticancer chemotherapy agent. For instance, X and Y can independently be doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, or pharmaceutically acceptable salts thereof.

In yet another aspect, the conjugate has Formula I: 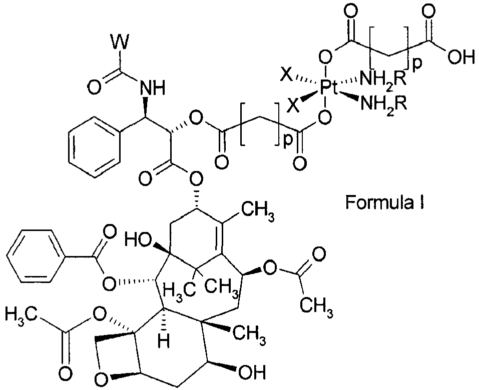 and pharmaceutically acceptable salts thereof, wherein 'p' is an integer from 1 to 10; 'X' is selected from the group consisting of halogen, sulfate, phosphate, nitrate, and water; 'W' is phenyl or tert-butyl oxy; and 'R' is hydrogen or alkyl. For instance, 'p' can be 3; 'X' can be chloride; 'W' can be phenyl and 'R' can be hydrogen.

In another aspect, the conjugate has Formula II: 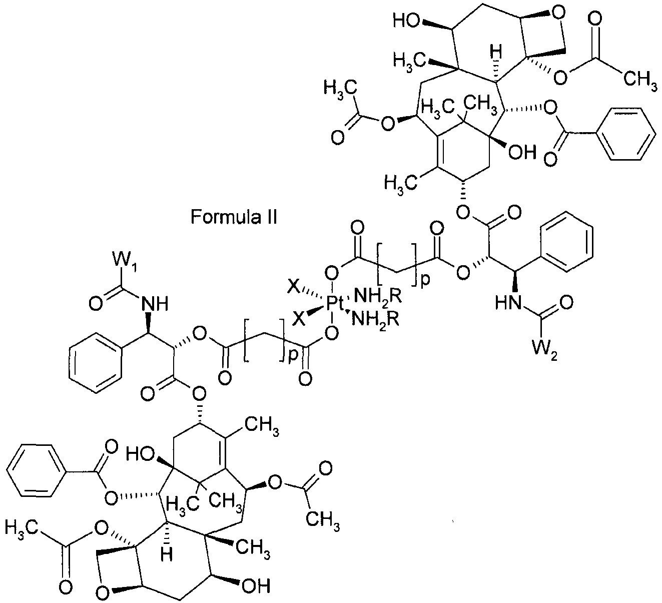 and pharmaceutically acceptable salts thereof, wherein 'p' is an integer from 1 to 10; 'X' is selected from the group consisting of halogen, sulfate, phosphate, nitrate, and water; 'W₁' and 'W₂' are independently selected from phenyl or tert-butyl oxy; and 'R' is hydrogen or alkyl. For instance, 'p' can be 3; 'X' can be chloride; 'W₁' and 'W₂' can be phenyl and 'R' can be hydrogen.

In another aspect, the conjugate has Formula III: 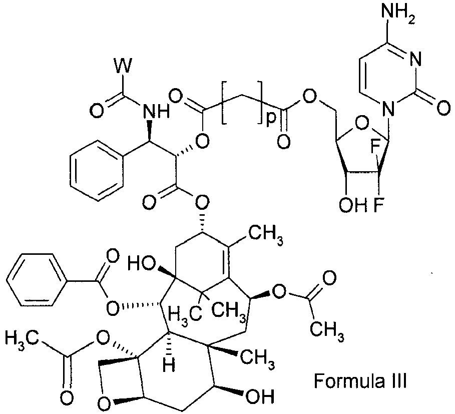 and pharmaceutically acceptable salts thereof, wherein 'p' is an integer from 1 to 10; and 'W' is slected from phenyl or tert-butyl oxy. For instance, 'p' can be 3; and 'W' can be phenyl.

In another aspect, the conjugate has Formula IV: 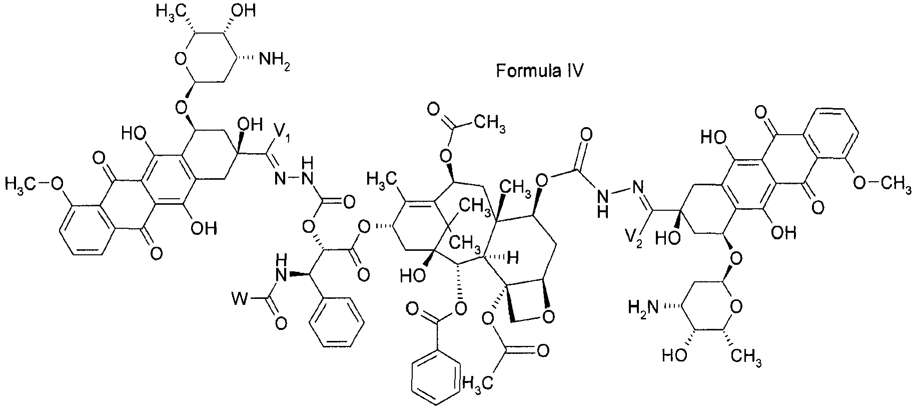 and pharmaceutically acceptable salts thereof, wherein 'W' is phenyl or tert-butyl oxy; and 'V₁' and 'V₂' are independently selected from -CH₃ or -CH₂OH. For instance, 'W' can be phenyl; and 'V₁' and 'V₂' can be --CH₂OH.

In another aspect, the conjugate has Formula V: 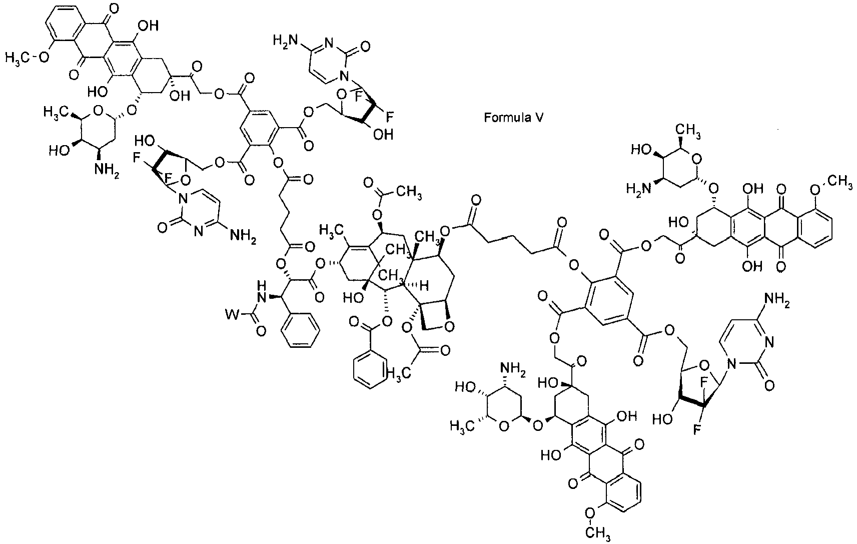 and pharmaceutically acceptable salts thereof, wherein 'W' is phenyl or tert-butyl oxy. For instance, 'W' can be phenyl.

In another aspect, the conjugate has Formula VI: 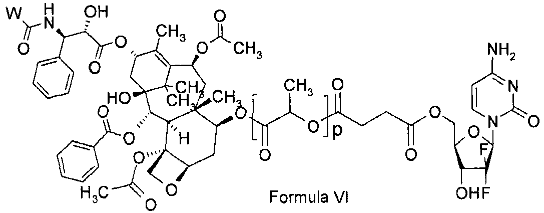 and pharmaceutically acceptable salts thereof, wherein 'p' is an integer from 5 to 20; and 'W' is phenyl or tert-butyl oxy. For instance, 'p' can be 10; and 'W' can be phenyl.

In another aspect, the conjugate has Formula VII: 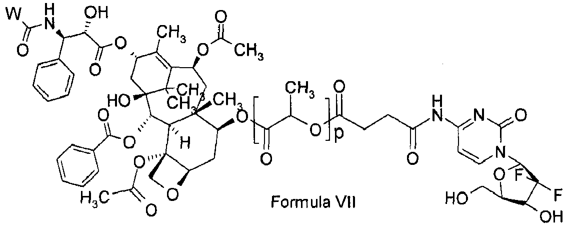 and pharmaceutically acceptable salts thereof, wherein 'p' is an integer from 5 to 20; and 'W' is phenyl or tert-butyl oxy. For instance, 'p' can be 10; and 'W' can be phenyl.

In yet another embodiment, a multi-drug conjugate is provided comprising a pharmaceutically active agent covalently bound to a plurality of stimuli-sensitive linkers, wherein each linker is covalently bound to at least one additional pharmaceutically active agent, wherein the conjugate releases at least one pharmaceutically active agent upon delivery to a target cell. In one aspect, the stimuli-sensitive linker can be a C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, or combinations thereof. For instance, the linker can be a C₃ straight chain alkyl. In yet another instance, the linker can comprise a ketone.

In yet another aspect, the pharmaceutically active agent comprises anticancer chemotherapy agents. For instance, the pharmaceutically active agent can include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In another embodiment, a pharmaceutical composition is provided comprising the multi-drug conjugate above, or a pharmaceutically acceptable salt thereof, in a pharmaceutically acceptable vehicle.

In yet another embodiment, a method is provided for controlling ratios of conjugated drugs contained in a nanoparticle inner sphere, the method comprising: a) synthesizing a combination of a first drug independently conjugated to a stimuli-sensitive linker, and a second drug independently conjugated to a linker having the same composition, wherein the first drug conjugate and second drug conjugate have a predetermined ratio; b) adding the combination to an agitated solution comprising a polar lipid; and c) adding water to the agitated solution, wherein nanoparticles are produced having a controlled ratio of conjugated drugs contained in the inner sphere. In various aspects of the present embodiment, about 100% of drugs contained in the inner sphere are conjugated.

In one aspect, the first drug and the second drug can independently include an antibiotic, antimicrobial, antiviral, growth factor, chemotherapeutic agent, and combinations thereof. For instance, the first drug and the second drug are independently selected from the group consisting of doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In another aspect, the stimuli-sensitive linker is a pH-sensitive linker. For instance, the stimuli-sensitive linker is selected from the group consisting of C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

In various aspects of the present embodiment, the combination of conjugated drugs having a predetermined ratio further comprises at least one additional drug independently conjugated to a stimuli-sensitive linker having the same composition.

In yet another embodiment, a method is provided for controlling ratios of conjugated drugs contained in a nanoparticle inner sphere, the method comprising: a) synthesizing a combination of (i) a first drug and a second drug conjugated by a first stimuli-sensitive linker, and (ii) a first drug and a second drug conjugated by a second stimuli-sensitive linker, wherein the first drug conjugate and second drug conjugate have a predetermined ratio; b) adding the combination to an agitated solution comprising a polar lipid; and c) adding water to the agitated solution, wherein nanoparticles are produced having a controlled ratio of conjugated drugs contained in the inner sphere. In various aspects of the present embodiment, about 100% of drugs contained in the inner sphere are conjugated.

In one aspect, the first drug and the second drug are independently selected from the group consisting of an antibiotic, antimicrobial, antiviral, growth factor, chemotherapeutic agent, and combinations thereof. For instance, the first drug and the second drug can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In another aspect, the stimuli-sensitive linker is a pH-sensitive linker. For instance, the first stimuli-sensitive linker and the second stimuli-sensitive linker can independently include C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

In various aspects of the present embodiment, the combination of conjugated drugs having a predetermined ratio further comprises at least one additional conjugate of a first drug and a second drug conjugated by a stimuli-sensitive linker other than those present in the combination.

In another embodiment, a method is provided for producing a combination of conjugated drugs having a predetermined ratio in a nanoparticle, said nanoparticle comprising an inner sphere, the method comprising: a) adding to an agitated solution comprising a polar lipid a combination of a first drug independently conjugated to a stimuli-sensitive linker, and a second drug independently conjugated to a linker having the same composition, wherein the first drug conjugate and the second drug conjugate have a predetermined ratio; and b) adding water to the agitated solution, wherein nanoparticles are produced containing in the inner sphere the conjugated drugs having a predetermined ratio. In various aspects, the method can further comprise: c) isolating nanoparticles having a diameter less than about 300 nm. In various aspects of the present embodiment, about 100% of drugs contained in the inner sphere are conjugated.

In various aspects, the first drug and the second drug are independently selected from the group consisting of an antibiotic, antimicrobial, growth factor, chemotherapeutic agent, and combinations thereof. For instance, the first drug and the second drug can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In yet another aspect, the stimuli-sensitive linker is a pH-sensitive linker. For instance, the stimuli-sensitive linker can be C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, or combinations thereof.

In yet another aspect, the combination of conjugated drugs having a predetermined ratio further comprise a third drug independently conjugated to a stimuli-sensitive linker having the same composition. In various aspects, the solution comprising a polar lipid further comprises a functionlalized polar lipid.

In yet another embodiment, a method is provided for producing a combination of conjugated drugs having a predetermined ratio in a nanoparticle, said nanoparticle comprising an inner sphere, the method comprising: a) adding to an agitated solution comprising a polar lipid a combination of (i) a first drug and second drug conjugated by a first stimuli-sensitive linker, and (ii) a first drug and a second drug conjugated by a second stimuli-sensitive linker, wherein the first drug conjugate and second drug conjugate have a predetermined ratio; and b) adding water to the agitated solution, wherein nanoparticles are produced containing in the inner sphere the conjugated drugs having a predetermined ratio. In various aspects, the method can further comprise: c) isolating nanoparticles having a diameter less than about 300 nm. In various aspects of the present embodiment, about 100% of drugs contained in the inner sphere are conjugated.

In one aspect, the first drug and the second drug can independently include an antibiotic, antimicrobial, growth factor, chemotherapeutic agent, and combinations thereof. For instance, the first drug and the second drug are independently selected from the group consisting of doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In another aspect, the stimuli-sensitive linker is a pH-sensitive linker. For instance, the first stimuli-sensitive linker and the second stimuli-sensitive linker can independently be C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

In various aspects of the present embodiment, the combination of conjugated drugs having a predetermined ratio further comprises at least one additional conjugate of a first drug and a second drug conjugated by a stimuli-sensitive linker other than those present in the combination. In various aspects, the solution comprising a polar lipid further comprises a functionlalized polar lipid.

In yet another disclosure, a method is provided for treating a disease or condition, the method comprising administering a therapeutically effective amount of the nanoparticle above to a subject in need thereof. In one aspect, the disease is a proliferative disease including lymphoma, renal cell carcinoma, prostate cancer, lung cancer, pancreatic cancer, melanoma, colorectal cancer, ovarian cancer, breast cancer, glioblastoma multiforme and leptomeningeal carcinomatosis. In another aspect, the disease is a heart disease including Atherosclerosis, Ischemic heart disease, Rheumatic heart disease, Hypertensive heart disease, Infective endocarditis, Coronary heart disease, and Constrictive pericarditis. In another aspect, the disease is an ocular disease selected from the group consisting of macular edema, retinal ischemia, macular degeneration, uveitis, blepharitis, keratitis, rubeosis iritis, iridocyclitis, conjunctivitis, and vasculitis. In another aspect, the disease is a lung disease including asthma, Chronic Bronchitis, Cystic Fibrosis, Emphysema, Pneumonia, lung cancer, Primary Pulmonary Hypertension, Pulmonary Arterial Hypertension, and Tuberculosis. In yet another aspect, the disease includes bacterial infection, viral infection, fungal infection, and parasitic infection.

In various aspects of the present disclosure, the nanoparticle is administered systemically. In another aspect, the nanoparticle is administered locally. In yet another aspect, the local administration is via implantable metronomic infusion pump.

In yet another disclosure, a method is provided for treating a disease or condition, the method comprising administering a therapeutically effective amount of the multi-drug conjugate above to a subject in need thereof. In one aspect, the disease is a proliferative disease including lymphoma, renal cell carcinoma, prostate cancer, lung cancer, pancreatic cancer, melanoma, colorectal cancer, ovarian cancer, breast cancer, glioblastoma multiforme and leptomeningeal carcinomatosis. In one aspect, the disease is a heart disease including Atherosclerosis, Ischemic heart disease, Rheumatic heart disease, Hypertensive heart disease, Infective endocarditis, Coronary heart disease, and Constrictive pericarditis. In one aspect, the disease is an ocular disease including macular edema, retinal ischemia, macular degeneration, uveitis, blepharitis, keratitis, rubeosis iritis, iridocyclitis, conjunctivitis, and vasculitis. In one aspect, the disease is a lung disease including asthma, Chronic Bronchitis, Cystic Fibrosis, Emphysema, Pneumonia, lung cancer, Primary Pulmonary Hypertension, Pulmonary Arterial Hypertension, and Tuberculosis. In yet another aspect, the disease is selected from the group consisting of bacterial infection, viral infection, fungal infection, and parasitic infection.

In various aspects of the present disclosure, the multi-drug conjugate is administered systemically. In another aspect, the multi-drug conjugate is administered locally. In yet another aspect, the local administration is via implantable metronomic infusion pump.

In yet another disclosure, a method is provided for sequentially delivering a drug conjugate to a target cell, the method comprising administering a nanoparticle above to the target cell and triggering multi-drug conjugate release. In various aspects of the present embodiment, the nanoparticle is administered systemically. In another aspect, the nanoparticle is administered locally. In yet another aspect, the local administration is via implantable metronomic infusion pump.

In yet another embodiment, a method is provided for nanoencapsulation of a plurality of drugs comprising separately linking each of the plurality of drugs with a corresponding polymer backbone with nearly 100% loading efficiency by forming the corresponding polymer backbone by ring opening polymerization beginning with the corresponding drug, wherein each of the corresponding polymer backbones has the same or similar physicochemical properties and has approximately the same chain length; mixing the plurality of linked drugs and polymers at selectively predetermined ratios at selectively and precisely controlled drug ratios; and synthesizing the mixed plurality of linked drugs and polymers into a nanoparticle.

In various aspects, the plurality of drugs can independently include an antibiotic, antimicrobial, growth factor, chemotherapeutic agent, and combinations thereof. For instance, the plurality of drugs can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In various aspects, the polymer backbone is a stimuli-sensitive linker. For instance, the stimuli-sensitive linker can include a C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

These and other features, aspects and advantages of the present teachings will become better understood with reference to the following description, examples and appended claims.

### DRAWINGS

Those of skill in the art will understand that the drawings, described below, are for illustrative purposes only. The drawings are not intended to limit the scope of the present teachings in any way.
Figure 1. Schematic illustration of a dual-drug loaded lipid-polymer hybrid nanoparticle, of which the polymeric core consists of two distinct drug-polymer conjugates with ratiometric control over drug loading.
Figure 2. Chemical characterization of the drug-polymer conjugates. (A) Schematic description of the living ring-opening polymerization of 1-lactide catalyzed by an activated metal alkoxide complex. (B) Qualitative ¹H-NMR spectra showing the characteristic proton resonance peaks of DOX-PLA (upper panel) and CPT-PLA (lower panel). (C) Gel permeation chromatograms of DOX-PLA (red dashed line) and CPT-PLA (black solid line).
Figure 3. Scanning electron microscopy (SEM) and dynamic light scattering (DLS) measurements showing the morphology and size of lipid-polymer hybrid nanoparticles with the polymer cores consisting of: (A) DOX-PLA conjugates, (B) CPT-PLA conjugates, or (C) DOX-PLA and CPT-PLA conjugates with a molar ratio of 1:1.
Figure 4. Quantification of DOX and CPT loading efficiency in dual-drug loaded nanoparticles (containing both DOX-PLA and CAP-PLA) and single-drug loaded nanoparticles (containing DOX-PLA or CPT-PLA), respectively. NPs: nanoparticles.

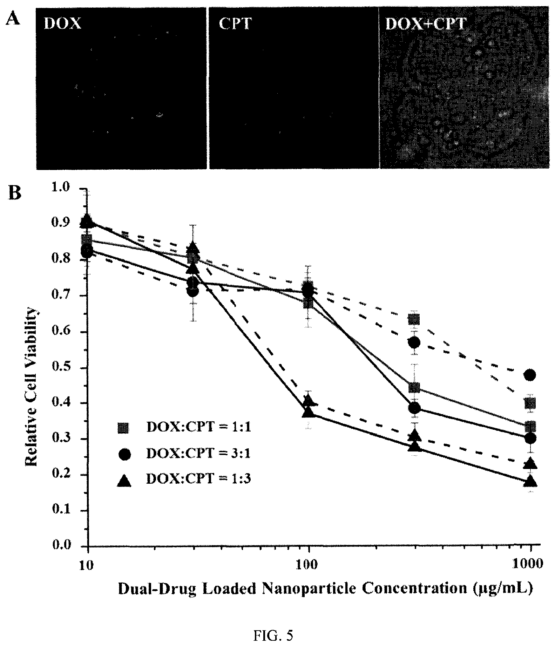

Figure 5. Cellular colocalization and cytotoxicity studies of the DOX-PLA and CPT-PLA loaded dual-drug nanoparticles. (A) Fluorescence microscopy images showing the colocalization of DOX and CPT in the cellular compartment of MDB-MB-435 breast cancer cells. (B) A comparative study of cellular cytotoxicity of the DOX-PLA and CPT-PLA loaded dual-drug nanoparticles against the MDB-MB-435 breast cancer cells. The ratios shown in figure legends are the molar ratios of DOX-PLA to CPT-PLA. Solid lines represent the dual-drug loaded nanoparticles and dashed lines represent the cocktail mixture of DOX-PLA loaded and CPT-PLA loaded single-drug nanoparticles. All samples were incubated with cells for 24 h, and the cells were subsequently washed and incubated in media for a total of 72 h prior to MTT assay (n=4).

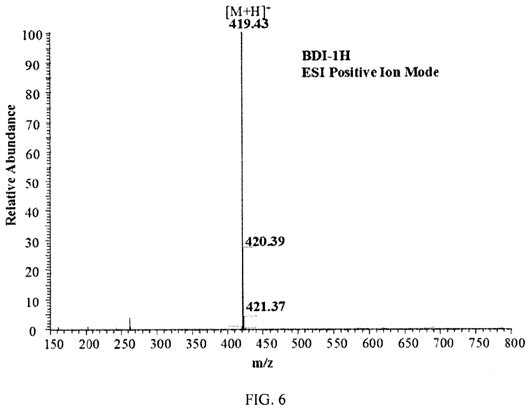

Figure 6. Mass spectrum (ESI-positive ion mode) of 2-((2,6-diisopropylphenyl)amido)-4-((2,6diisopropylphenyl)-imino)-2-pentene (BDI).

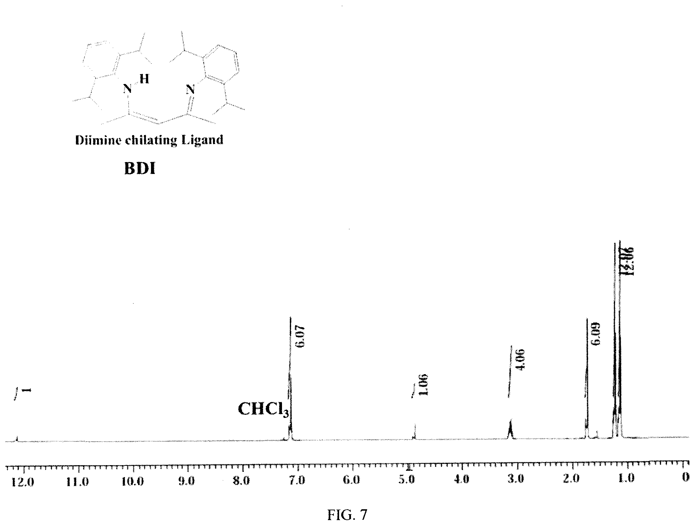

Figure 7. ¹H-NMR characterization of 2-((2,6-diisopropylphenyl)amido)-4-((2,6diisopropylphenyl) -imino)-2-pentene (BDI).

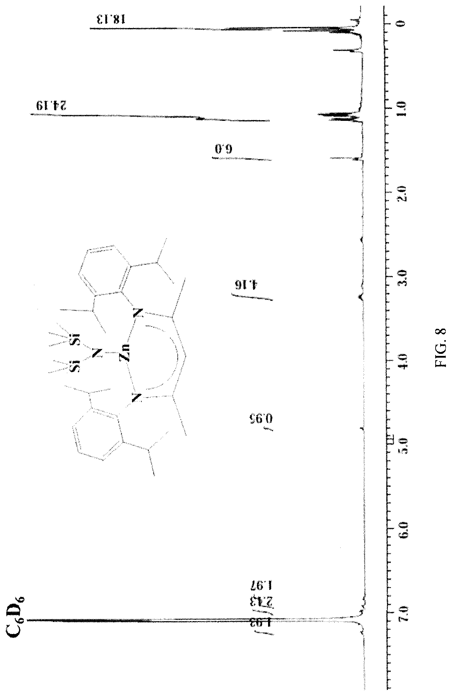

Figure 8. ¹H-NMR characterization of (BDI)ZnN(SiMe₃)₂ complex catalyst.
Figure 9. Synthesis scheme of paclitaxel (PTXL) and gemcitabine hydrochloride (GEM) conjugate (PTXL-GEM conjugate, compound 2).
Figure 10. Characterization of PTXL-GEM conjugates using (A) ¹H-NMR spectroscopy showing the characteristic protons, and **(B)** high resolution mass spectrum determining the exact mass and corresponding molecular formula of the drug conjugates.
Figure 11. Hydrolysis and cellular cytotoxicity of PTXL-GEM conjugates. (A) HPLC chromatograms of PTXL-GEM conjugates **(a)** before and **(b)** after 24 hrs of incubation in water/acetonitrile (75/25, v/v) solution at pH = 7.4. **(B)** Hydrolysis kinetics of PTXL-GEM conjugates at pH = 6.0 and pH = 7.4. (C) Time dependent comparative cytotoxicity of PTXL-GEM conjugates with the corresponding mixture of free PTXL and free GEM drugs at 100 nM concentration against XPA3 human pancreatic cancer cell line (n = 8).
Figure 12. Characterization of PTXL-GEM conjugates loaded lipid-coated polymeric nanoparticles (NPs). **(A)** Schematic illustration of a PTXL-GEM conjugates loaded nanoparticle. **(B)** Representative scanning electron microscopy (SEM) image of PTXL-GEM conjugates loaded nanoparticles. **(C)** Diameter and surface zeta-potential of PTXL-GEM conjugates loaded nanoparticles and empty nanoparticles measured by dyanamic light scattering (DLS).
Figure 13. **(A)** PTXL-GEM conjugates loading yield at various initial weight ratios of PTXL-GEM conjugates/excipient (PLGA polymer). **(B)** Cellular cytotoxicity of PTXL-GEM conjugates loaded nanoparticles and free PTXL-GEM conjugates (compound 2) at various drug conjugate concentrations against XPA3 human pancreatic cancer cell line. All samples were incubated with cells for 24 hrs, and the cells were subsequently washed and incubated in media for a total of 72 hrs before assessing cell viability in each group (n = 8).
Figure 14. ¹H NMR spectrum of paclitaxel.
Figure 15. ¹H NMR spectrum of compound 1.
Figure 16. ESI-MS (positive) mass spectrum of compound 1.
Figure 17. ESI-MS (positive) mass spectrum of paclitaxel recovered from the hydrolyzed PTXL-GEM conjugates with an HPLC retention time of 6.2 min.
Figure 18. ESI-MS (positive) mass spectrum of gemcitabine recovered from the hydrolyzed PTXL-GEM conjugates with an HPLC retention time of 1.8 min.
Figure 19. Synthesis scheme of paclitaxel (Ptxl) and cisplatin conjugate (Ptxl-Pt(IV) conjugate) as a representative hydrophobic-hydrophilic drug conjugate.
Figure 20. Characterization of Ptxl-Pt(IV) conjugate using (A) ¹H-NMR spectroscopy showing the characteristic protons, and (B) high resolution mass spectrum determining the exact mass and corresponding molecular formula of the Ptxl-Pt(IV) conjugate.
Figure 21. Characterization of Ptxl-Pt(IV) conjugates loaded nanoparticles. (A) Schematic illustration of Ptxl-Pt(IV) conjugates loaded lipid coated polymeric nanoparticles. (B) Dynamic light scattering (DLS) measurement of Ptxl-Pt(IV) loaded nanoparticles. (C) Representative scanning electron microscopy (SEM) image of Ptxl-Pt(IV) loaded nanoparticles. Inset: high-resolution SEM image of Ptxl-Pt(IV) loaded nanoparticles

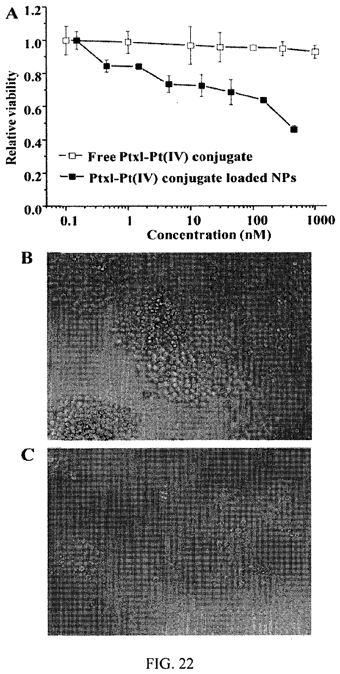

Figure 22. (A) Cellular cytotoxicity of free Ptxl-Pt(IV) conjugates and Ptxl-Pt(IV) conjugates loaded nanoparticles (NPs) at various drug concentration against A2780 human ovarian cancer cell line. All samples were incubated with cells for 24 hrs, and the cells were subsequently washed and incubated in fresh media for a total of 72 hrs before cell viability using the ATP assay (n=8). (B,C) Representative phase contrast microscopy images of A2780 cells treated with (B) free Ptxl-Pt(IV) drug conjugates and (C) Ptxl-Pt(IV) conjugates loaded nanoparticles, respectively, at a drug concentration of 300 nM.

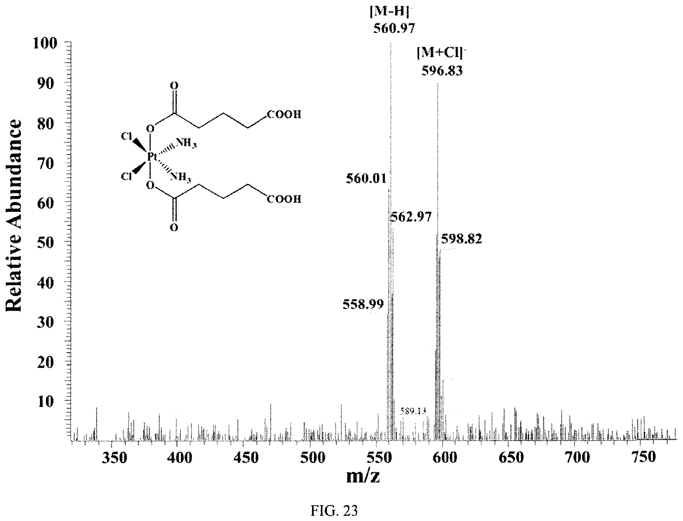

Figure 23. ¹H NMR spectrum of cis-trans-cis PtCl₂(OCOCH₂CH₂CH₂COOH)₂(NH₃)₂.
Figure 24. Drug loading yield of PTXL conjugates.

### DETAILED DESCRIPTION

### Abbreviations and Definitions

To facilitate understanding of the invention, a number of terms and abbreviations as used herein are defined below as follows:

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

The term "and/or" when used in a list of two or more items, means that any one of the listed items can be employed by itself or in combination with any one or more of the listed items. For example, the expression "A and/or B" is intended to mean either or both of A and B, *i.e.* A alone, B alone or A and B in combination. The expression "A, B and/or C" is intended to mean A alone, B alone, C alone, A and B in combination, A and C in combination, B and C in combination or A, B, and C in combination.

In the descriptions of molecules and substituents, molecular descriptors can be combined to produce words or phrases that describe substituents. Such descriptors are used in this document. Examples include such terms as aralkyl (or arylalkyl), heteroaralkyl, heterocycloalkyl, cycloalkylalkyl, aralkoxyalkoxycarbonyl and the like. A specific example of a compound encompassed with the latter descriptor aralkoxyalkoxycarbonyl is C₆H₅-CH₂-CH₂-O-CH₂-O-C(O) wherein C₆H₅ is phenyl. It is also to be noted that a substituents can have more than one descriptive word or phrase in the art, for example, heteroaryloxyalkylcarbonyl can also be termed heteroaryloxyalkanoyl. Such combinations are used herein in the description of the compounds and methods of this invention and further examples are described herein.

Alkyl: The term "alkyl" as used herein describes substituents which are preferably lower alkyl containing from one to eight carbon atoms in the principal chain and up to about 20 carbon atoms. The principal chain may be straight or branched chain or cyclic and include methyl, ethyl, propyl, isopropyl, butyl, hexyl and the like.

Analog: The term "analog" as used herein may refer to a compound in which one or more atoms are replaced with a different atom or group of atoms. The term may also refer to compounds with an identity of atoms but of different isomeric configuration. Such isomers may be constitutional isomers, *i.e.* structural isomers having different bonding arrangements of their atoms or stereoisomers having identical bonding arrangements but different spatial arrangements of the constituent atoms.

Anionic: The term "anionic" as used herein refers to substances capable of forming ions in aqueous media with a net negative charge.

Anionic functional group: The term "anionic functional group" as used herein refers to functional group as defined herein which possesses a net negative charge. Representative anionic functional groups include carboxylic, sulfonic, phosphonic, their alkylated derivatives, and so on.

Cationic: The term "cationic" as used herein refers to substances capable of forming ions in aqueous media with a net positive charge.

Functional group: The term "functional group" as used herein, refers to a chemical group that imparts a particular function to an article (*e.g.*, nanoparticle) bearing the chemical group. For example, functional groups can include substances such as antibodies, oligonucleotides, biotin, or streptavidin that are known to bind particular molecules; or small chemical groups such as amines, carboxylates, and the like.

Halogen: The terms "halogen" or "halo" as used herein, alone or as part of a group of atoms, refer to chlorine, bromine, fluorine, and iodine.

Nanoparticle: The term "nanoparticle" as used herein refers to unilamellar or multilamellar lipid vesicles which enclose a fluid space and has a diameter of between about 1 nm and about 1000 nm. Similarly, by the term "nanoparticles" is meant a plurality of particles having an average diameter of between about 1 nm and about 1000 nm. The term can also include vesicles as large as 10,000 nm depending on the environment such nanoparticles are administered to a subject, for example, locally to a tumor *in situ* via implantable pump or via syringe. For systemic use, an average diameter of about 30 nm to about 300 nm is preferred. The walls of the vesicles, also referred to as a membrane, are formed by a bimolecular layer of one or more lipid components (*e*.*g*., multiple phospholipids and cholesterol) having polar heads and non-polar tails, such as a phospholipid. In an aqueous (or polar) solution, and in a unilamellar nanoparticle, the polar heads of one layer orient outwardly to extend into the surrounding medium, and the non-polar tail portions of the lipids associate with each other, thus providing a polar surface and a non-polar core in the wall of the vesicle. In a multilamellar nanoparticle, the polar surface of the vesicle also extends to the core of the liposome and the wall is a bilayer. The wall of the vesicle in either of the unilamellar or multilamellar nanoparticles can be saturated or unsaturated with other lipid components, such as cholesterol, free fatty acids, and phospholipids. In such cases, an excess amount of the other lipid component can be added to the vesicle wall which will shed until the concentration in the vesicle wall reaches equilibrium, which can be dependent upon the nanoparticle environment. Nanoparticles may also comprise other agents that may or may not increase an activity of the nanoparticle. For example, polyethylene glycol (PEG) can be added to the outer surface of the membrane to enhance bioavailability. In other examples, functional groups such as antibodies and aptamers can be added to the outer surface of the membrane to enhance site targeting, such as to cell surface epitopes found in cancer cells. The membrane of the nanoparticles can also comprise particles that can be biodegradable, cationic nanoparticles including, but not limited to, gold, silver, and synthetic nanoparticles. An example of a biocompatible synthetic nanoparticle includes polystyrene and the like.

Pharmaceutically active: The terms "pharmaceutically active" as used herein refer to the beneficial biological activity of a substance on living matter and, in particular, on cells and tissues of the human body. A "pharmaceutically active agent" or "drug" is a substance that is pharmaceutically active and a "pharmaceutically active ingredient" (API) is the pharmaceutically active substance in a drug.

Pharmaceutically acceptable: The terms "pharmaceutically acceptable" as used herein means approved by a regulatory agency of the Federal or a state government or listed in the U.S. Pharmacopoeia, other generally recognized pharmacopoeia in addition to other formulations that are safe for use in animals, and more particularly in humans and/or non-human mammals.

Pharmaceutically acceptable salt: The terms "pharmaceutically acceptable salt" as used herein refer to acid addition salts or base addition salts of the compounds, such as the multi-drug conjugates, in the present disclosure. A pharmaceutically acceptable salt is any salt which retains the activity of the parent compound and does not impart any deleterious or undesirable effect on a subject to whom it is administered and in the context in which it is administered. Pharmaceutically acceptable salts include, but are not limited to, metal complexes and salts of both inorganic and carboxylic acids. Pharmaceutically acceptable salts also include metal salts such as aluminum, calcium, iron, magnesium, manganese and complex salts. In addition, pharmaceutically acceptable salts include, but are not limited to, acid salts such as acetic, aspartic, alkylsulfonic, arylsulfonic, axetil, benzenesulfonic, benzoic, bicarbonic, bisulfuric, bitartaric, butyric, calcium edetate, camsylic, carbonic, chlorobenzoic, citric, edetic, edisylic, estolic, esyl, esylic, formic, fumaric, gluceptic, gluconic, glutamic, glycolic, glycolylarsanilic, hexamic, hexylresorcjnoic, hydrabamic, hydrobromic, hydrochloric, hydroiodic, hydroxynaphthoic, isethionic, lactic, lactobionic, maleic, malic, malonic, mandelic, methanesulfonic, methylnitric, methylsulfuric, mucic, muconic, napsylic, nitric, oxalic, p-nitromethanesulfonic, pamoic, pantothenic, phosphoric, monohydrogen phosphoric, dihydrogen phosphoric, phthalic, polygalactouronic, propionic, salicylic, stearic, succinic, sulfamic, sulfanlic, sulfonic, sulfuric, tannic, tartaric, teoclic, toluenesulfonic, and the like. Pharmaceutically acceptable salts may be derived from amino acids including, but not limited to, cysteine. Methods for producing compounds as salts are known to those of skill in the art (see, for example, Stahl et al., Handbook of Pharmaceutical Salts: Properties, Selection, and Use, Wiley-VCH; Verlag Helvetica Chimica Acta, Zürich, 2002; Berge et al., J Pharm. Sci. 66: 1, 1977).

Pharmaceutically acceptable carrier: The terms "pharmaceutically acceptable carrier" as used herein refers to an excipient, diluent, preservative, solubilizer, emulsifier, adjuvant, and/or vehicle with which a compound, such as a multi-drug conjugate, is administered. Such carriers may be sterile liquids, such as water and oils, including those of petroleum, animal, vegetable or synthetic origin, such as peanut oil, soybean oil, mineral oil, sesame oil and the like, polyethylene glycols, glycerine, propylene glycol or other synthetic solvents. Water is a preferred carrier when a compound is administered intravenously. Saline solutions and aqueous dextrose and glycerol solutions may also be employed as liquid carriers, particularly for injectable solutions. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. A compound, if desired, may also combine minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; chelating agents such as ethylenediaminetetraacetic acid; and agents for the adjustment of tonicity such as sodium chloride or dextrose may also be a carrier. Methods for producing compounds in combination with carriers are known to those of skill in the art.

Phospholipid: The term "phospholipid", as used herein, refers to any of numerous lipids contain a diglyceride, a phosphate group, and a simple organic molecule such as choline. Examples of phospholipids include, but are not limited to, Phosphatidic acid (phosphatidate) (PA), Phosphatidylethanolamine (cephalin) (PE), Phosphatidylcholine (lecithin) (PC), Phosphatidylserine (PS), and Phosphoinositides which include, but are not limited to, Phosphatidylinositol (PI), Phosphatidylinositol phosphate (PIP), Phosphatidylinositol bisphosphate (PIP2) and Phosphatidylinositol triphosphate (PIP3). Additional examples of PC include DDPC, DLPC, DMPC, DPPC, DSPC, DOPC, POPC, DRPC, and DEPC as defined in the art.

Stimuli-Sensitive Linker: As used herein, the term "stimuli-sensitive linker" refers to a carbon chain that can contain heteroatoms (*e.g*., nitrogen, oxygen, sulfur, etc.) and which may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 atoms long. Stimuli-sensitive linkers may be substituted with various substituents including, but not limited to, hydrogen atoms, alkyl, alkenyl, alkynl, amino, alkylamino, dialkylamino, trialkylamino, hydroxyl, alkoxy, halogen, aryl, heterocyclic, aromatic heterocyclic, cyano, amide, carbamoyl, carboxylic acid, ester, thioether, alkylthioether, thiol, and ureido groups. Those of skill in the art will recognize that each of these groups may in turn be substituted. Examples of stimuli-sensitive linkers include, but are not limited to, pH sensitive linkers, protease cleavable peptide linkers, nuclease sensitive nucleic acid linkers, lipase sensitive lipid linkers, glycosidase sensitive carbohydrate linkers, hypoxia sensitive linkers, photo-cleavable linkers, heat-labile linkers, enzyme cleavable linkers (*e*.*g*., esterase cleavable linker), ultrasound-sensitive linkers, x-ray cleavable linkers, and so forth.

Substituted: The term "substituted" as used herein refers to one or more substitutions that are common in the art. The terms "optionally substituted" means that a group may be unsubstituted or substituted with one or more substituents. Suitable substituents for any of the groups defined above may include moieties such as alkyl, cycloalkyl, alkenyl, alkylidenyl, aryl, heteroaryl, heterocyclyl, halo (e.g., chloro, bromo, iodo and fluoro), cyano, hydroxy, alkoxyl, aroxyl, sulfhydryl (mercapto), alkylthio, arylthio, amino, substituted amino, nitro, carbamyl, keto (oxo), acyl, glycolyl, glycyl, hydrazino, guanyl, sulfamyl, sulfonyl, sulfinyl, thioalkyl-C(O)--, thioalkyl-CO₂--, and the like.

Therapeutically Effective Amount: As used herein, the term "therapeutically effective amount" refers to those amounts that, when administered to a particular subject in view of the nature and severity of that subject's disease or condition, will have a desired therapeutic effect, *e.g*., an amount which will cure, prevent, inhibit, or at least partially arrest or partially prevent a target disease or condition. More specific embodiments are included in the Pharmaceutical Preparations and Methods of Administration section below.

### Ratiometric Combinatorial Drug Delivery

The present teachings include ratiometric combinatorial drug delivery including nanoparticles, multi-drug conjugates, pharmaceutical compositions, methods of producing such compositions and methods of using such compositions, including in the treatment of diseases and conditions using drug combinations.

A combinatorial drug conjugation approach is provided to enable multi-drug delivery. In one example, hydrophobic and hydrophilic drugs were covalently conjugated using a hydrolysable linker and then encapsulated into lipid-polymer hybrid nanoparticles for combined delivery. In one non-limiting example, the ratio between two drugs co-delivered, some with drastically different properties, included various ratios including a 1:1 drug-drug ratio, and in other examples 3:1 and 1:3 ratios. As disclosed herein, such ratios can be controlled by the different molar amounts of the drugs in combination which results in versatile multi-drug encapsulation schemes.

In one aspect, each different drug molecule is linked to an individual linker backbone that has the same physicochemical properties and nearly the same chain length (*i.e.* a drug-linker). These drug-linker conjugates can be subsequently mixed at predetermined ratios prior to or during nanoparticle synthesis. The long and sharply distributed linker, in some examples a polymer chain, can provide each drug molecule a predominant and uniform hydrophobic property, and yield near 100% drug loading efficiency upon nanoparticle formation. In various aspects, the linkers can be stimuli-sensitive such that the linked drug is cleaved upon a change in the nanoparticle or multi-drug conjugate environment, such as a difference in pH.

In another aspect, an individual drug molecule is linked to another individual drug molecule, each being linked through different linkers. These drug-drug conjugates can be subsequently mixed or created at predetermined ratios prior to or during nanoparticle synthesis. The hydrophobic properties of these conjugates can be different and the linkers can have different stimuli-sensitive activities. This can result in sequential drug delivery as one linker can be cleaved to release a drug at a certain environmental state, and a second linker can release the same or different drug upon a change in environmental state, such as a different pH.

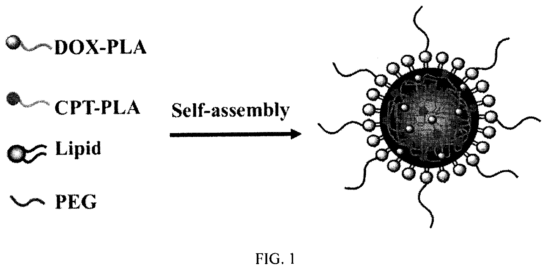

As provided in one non-limiting example, the synthesis of a drug-linker conjugate with two different pharmaceutically active agents, doxorubicin (DOX) and camptothecin (CPT), is provided. Utilizing ring-opening polymerization of 1-lactide, DOX and CPT polymer conjugates were synthesized using metal-amido catalyst, which reacts selectively with hydroxyl groups of the drug molecules to initiate polymerization (R. Tong, J. Cheng, Angew Chem Int Ed Engl 2008, 47, 4830-4834; R. Tong, J. Cheng, Angew Chem 2008, 120, 4908-4912; R. Tong, J. Cheng, Bioconjug Chem 2010, 21, 111-121; R. Tong, J. Cheng, J Am Chem Soc 2009, 131, 4744-4754). Using a nanoprecipitation technique (Figure 1), the drug-polymer conjugates were quantitatively loaded into lipid-polymer hybrid nanoparticles at high loading efficiency and precisely controlled drug ratios. *See* B. M. Chamberlain, et al. J Am Chem Soc 2001, 123, 3229-3238; L. Zhang, et al. ACS Nano 2008, 2, 1696-1702. The combinatorial treatment provided herein shows superior efficacy to cocktail therapy *in vitro* and offers a solution to the aforementioned limitations in multi-drug encapsulation into the same nanoparticles.

### Ratiometrically Controlled Nanoparticles

Therefore, in one embodiment, a nanoparticle is provided that includes an inner sphere and an outer surface, the inner sphere containing a combination of conjugated drugs connected by a stimuli-sensitive bond and having a predetermined ratio, wherein the conjugated drugs have the following formula:

(X-Y-Z)ₙ

wherein X is a pharmaceutically active agent, Y is a stimuli-sensitive linker, and Z is not X, and Z is a pharmaceutically active agent or hydrogen.

In various aspects, X and Z can independently be an antibiotic, antimicrobial, growth factor, chemotherapeutic agent, and combinations thereof. A listing of classes and specific drugs suitable for use in the present invention may be found in Pharmaceutical Drugs: Syntheses, Patents, Applications by Axel Kleemann and Jurgen Engel, Thieme Medical Publishing, 1999 and the Merck Index: An Encyclopedia of Chemicals, Drugs and Biologicals, Ed. by Budavari et al., CRC Press, 1996, both of which are incorporated herein by reference. Examples of such pharmaceutically active agents are provided in the Tables appended hereto. Such pharmaceutically active agents can be delivered to particular organs, tissues, cells, extracellular matrix components, and/or intracellular compartments via any suitable method, including the use of a functional group such as an antibody, antibody fragment, aptamer, and so on.

For instance, X can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

These and other pharmaceutically active agents can be covalently conjugated by a suitable chemical linker through environmentally cleavable bonds. Any of a variety of methods can be used to associate a linker with a pharmaceutically active agent including, but not limited to, passive adsorption (*e.g*., via electrostatic interactions), multivalent chelation, high affinity non-covalent binding between members of a specific binding pair, covalent bond formation, etc. In some embodiments, click chemistry can be used to associate a linker with a particle (e.g. Diels-Alder reaction, Huigsen 1,3-dipolar cycloaddition, nucleophilic substitution, carbonyl chemistry, epoxidation, dihydroxylation, etc.). In various aspects, drug conjugates including a plurality of pharmaceutically active agents, each of which is covalently bound to a linker, wherein the conjugate releases the pharmaceutically active agent upon delivery to target cells, are provided.

Some chemical bonds such as hydrazone, ester and amide bonds are sensitive to acidic pH values, for example, of the intracellular environment of tumor cells. At acidic pH, hydrogen ions catalyze the hydrolysis of these bonds which in turn releases the drug from its conjugate format. Therefore, different pharmaceutically active agents, such as but not limited to paclitaxel, gemcitabin, doxorubicine, cisplatin, docetaxel, etc, having -OH, -NH₂, and/or ketonic groups may be covalently linked together with a suitable spacer with alkyl chains of variable lengths. These spacers may be easily introduced to the drug conjugates by reacting different acid anhydrides and any organic compounds having monofunctional or bifunctional or hetero functional groups with the drugs.

For the pharmaceutically active agents without functional groups such as-OH, -NH₂, or ketonic groups, they may be covalently linked with other pharmaceutically active agents by creating such functional groups. For example, cisplatin can first be oxidized to its hydroxyl derivative which then can react with carboxylic acid aldehyde or acid anhydride to create an aldehydic and carboxylic functional group. This functional group can be covalently linked with other drugs with -OH and/or -NH₂. Many pharmaceutically active agents can be linked together to form combinatorial drug conjugates for combination therapy. Those of skill in the art are able to recognize other conjugation methods which are well known in the art. Such conjugation methods may be used to link various pharmaceutically active agents, including small molecules, polypeptides, and polynucleotides, via linkers, including stimuli-sensitive linkers.

In various aspects, the variable 'n' of the formula (X-Y-Z)ₙ is an integer greater than or equal to 2. In various aspects, this numeral represents 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 and even greater numbers of drug-linker and drug-drug conjugates can be contained in the nanoparticle.

In another aspect, each individual conjugated drug of the combination comprises a predetermined molar weight percentage from about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, to about 99%, provided that the sum of all individual conjugated drug molar weight percentages of the combination is 100%. For example, a first drug-linker conjugate can comprise 70 weight percent (70% w/w) and a second drug-linker conjugate can comprise 30 weight percent (30% w/w) as contained in the nanoparticle. In another example, a first drug-drug conjugate can comprise 40 weight percent (40% w/w) and a second drug-linker conjugate can comprise 60 weight percent (60% w/w) as contained in the nanoparticle. In yet another example, a first drug-linker conjugate can comprise 10 weight percent (10% w/w), a second drug-linker conjugate can comprise 30 weight percent (30% w/w), and a third drug-linker conjugate can comprise 60 weight percent (60% w/w) as contained in the nanoparticle. As another example, a first drug-drug conjugate can comprise 10 weight percent (10% w/w), a second drug-drug conjugate can comprise 30 weight percent (30% w/w), and a third drug-drug conjugate can comprise 60 weight percent (60% w/w) as contained in the nanoparticle.

By using predetermined molar weight percentages, precise ratios among conjugated drugs in the nanoparticle can be provided. For example, among two-drug conjugate combinations, ratios including 1:1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, and 1:500 are provided. In another example having three-drug conjugate combinations ratios of 1:1:1, 1:2:1, 1:3:1, 1:1:2, 1:1:3, and so forth are provided. Those of skill in the art will recognize that other ratios can be provided with different numbers of drugs and different molar weight percentages are utilized.

In various aspects, Z can independently be an antibiotic, antimicrobial, growth factor, chemotherapeutic agent, hydrogen, and combinations described above. In addition, Z can be hydrogen (e.g., a drug-linker conjugate).

In various aspects, Y is a pH-sensitive linker. For instance, Y can include C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

In various aspects, the outer surface of the nanoparticle can include a cationic or anionic functional group.

In yet another aspect, the conjugated drug of the combination contained in the nanoparticle inner sphere has Formula I: 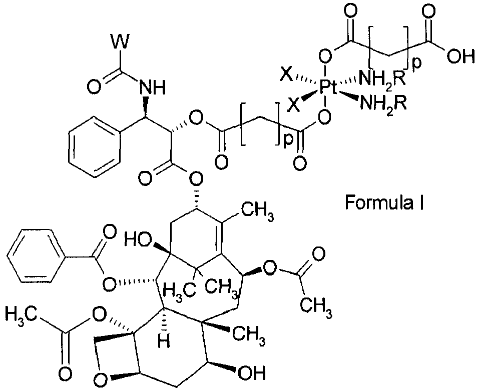 and pharmaceutically acceptable salts thereof, wherein 'p' is an integer from 1 to 10; 'X' is selected from the group consisting of halogen, sulfate, phosphate, nitrate, and water; 'W' is phenyl or tert-butyl oxy; and 'R' is hydrogen or alkyl. For instance, 'p' can be 3; 'X' can be chloride; 'W' can be phenyl and 'R' can be hydrogen.

In yet another aspect, the conjugated drug of the combination contained in the nanoparticle inner sphere has Formula II: 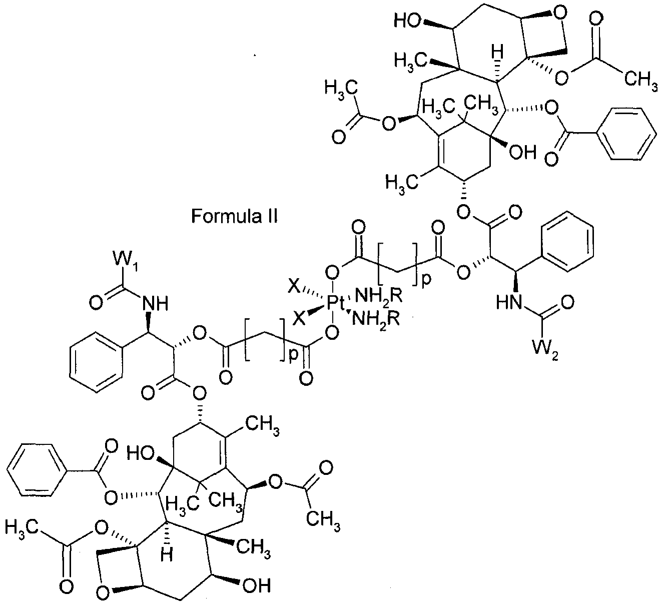 and pharmaceutically acceptable salts thereof, wherein 'p' is an integer from 1 to 10; 'X' is selected from the group consisting of halogen, sulfate, phosphate, nitrate, and water; 'W₁' and 'W₂' are independently selected from phenyl or tert-butyl oxy; and 'R' is hydrogen or alkyl. For instance, 'p' can be 3; 'X' is chloride; 'W₁' and 'W₂' can be phenyl and 'R' can be hydrogen.

In yet another aspect, the conjugated drug of the combination contained in the nanoparticle inner sphere has Formula III: 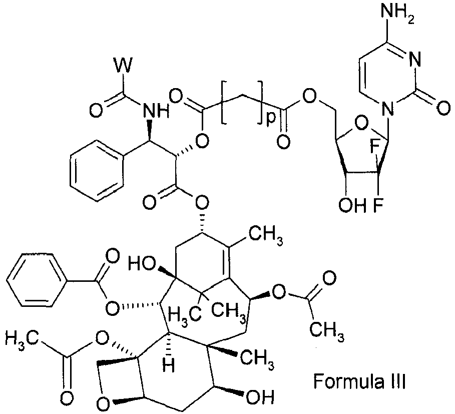 and pharmaceutically acceptable salts thereof, wherein 'p' is an integer from 1 to 10; and 'W' is slected from phenyl or tert-butyl oxy. For instance, 'p' can be 3; and 'W' can be phenyl.

In yet another aspect, the conjugated drug of the combination contained in the nanoparticle inner sphere has Formula IV: 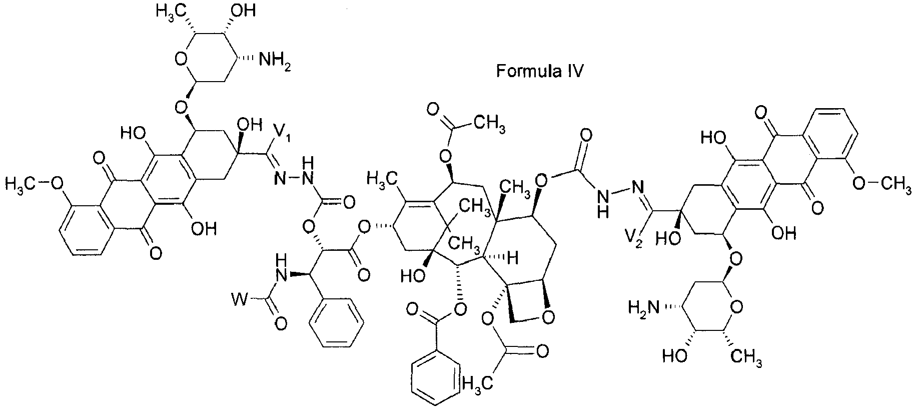 and pharmaceutically acceptable salts thereof, wherein 'W' is phenyl or tert-butyl oxy; and 'V₁' and 'V₂' are independently selected from -CH₃ or -CH₂OH. For instance, 'W' can be phenyl; and 'V₁' and 'V₂' can be -CH₂OH.

In yet another aspect, the conjugated drug of the combination contained in the nanoparticle inner sphere has Formula V: 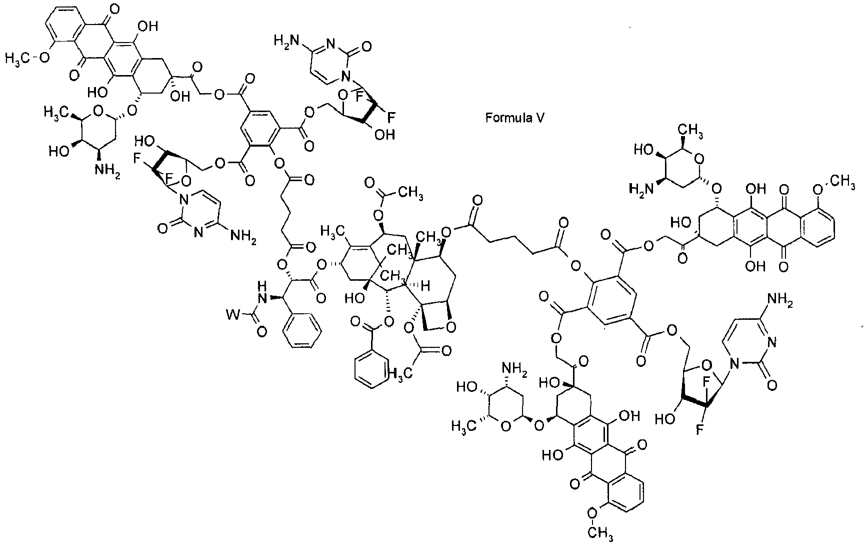 and pharmaceutically acceptable salts thereof, wherein 'W' is phenyl or tert-butyl oxy. For instance, 'W' can be phenyl.

In yet another aspect, the conjugated drug of the combination contained in the nanoparticle inner sphere has Formula VI: 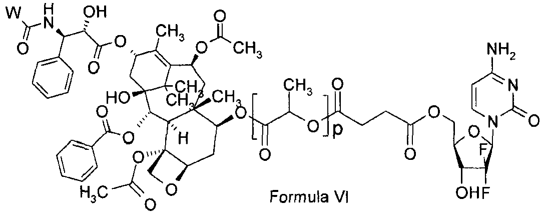 and pharmaceutically acceptable salts thereof, wherein 'p' is an integer from 5 to 20; and'W' is phenyl or tert-butyl oxy. For instance, 'p' can be 10; and 'W' can be phenyl.

In yet another aspect, the conjugated drug of the combination contained in the nanoparticle inner sphere has Formula VII: 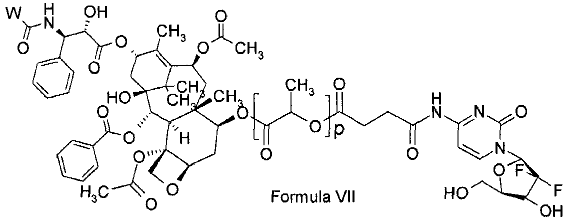 and pharmaceutically acceptable salts thereof, wherein 'p' is an integer from 5 to 20; and 'W' is phenyl or tert-butyl oxy. For instance, 'p' can be 10; and 'W' can be phenyl.

In various aspects, the nanoparticle can be about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 600, 700, 800, 900, 1000, 1100, 1200, 1300, 1400, 1500, 1600, 1700, 1800, 1900, 2000, 3000, 4000, 5000, 6000, 7000, 8000, 9000, and about 10000 nm in diameter. In various aspects, and particularly depending on the route of administration in a subject, the nanoparticle can have a diameter from about 30 nm to about 300 nm. In general, larger nanoparticles are acceptable when administered locally or topically where the nanoparticle is not required to traverse a subject vasculature to contact a target cell, tissue or organ. Likewise, smaller nanoparticles are acceptable when administered systemically in a subject, in particular nanoparticles from about 30 nm to about 300 nm.

### Multi-Drug Conjugates

In another embodiment, a multi-drug conjugate is provided having the following formula:

X-Y-Z

wherein X and Z are pharmaceutically active agents independently selected from the group consisting of an antibiotic, antimicrobial, growth factor, and chemotherapeutic agent; and Y is a stimuli-sensitive linker, wherein the conjugate releases at least one pharmaceutically active agent upon delivery of the conjugate to a target cell. Such conjugated drugs are provided above as contained in the nanoparticle of the present invention.

In various aspects of the present embodiment, Y is a C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof. For instance, Y can be a C₃ straight chain alkyl or a ketone. In various aspects, the pharmaceutically active agent comprises an anticancer chemotherapy agent. For instance, X and Y can independently be doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, or pharmaceutically acceptable salts thereof.

In yet another aspect, the conjugate has Formula I: 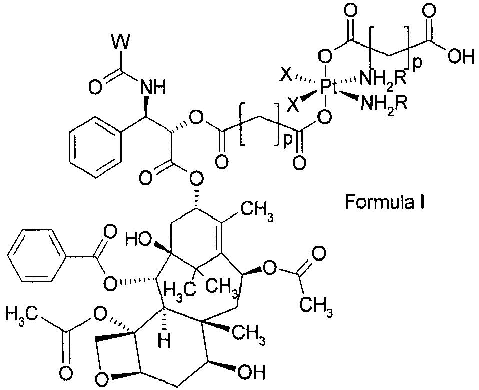 and pharmaceutically acceptable salts thereof, wherein 'p' is an integer from 1 to 10; 'X' is selected from the group consisting of halogen, sulfate, phosphate, nitrate, and water; 'W' is phenyl or tert-butyl oxy; and 'R' is hydrogen or alkyl. For instance, 'p' can be 3; 'X' can be chloride; 'W' can be phenyl and 'R' can be hydrogen.

In another aspect, the conjugate has Formula II: 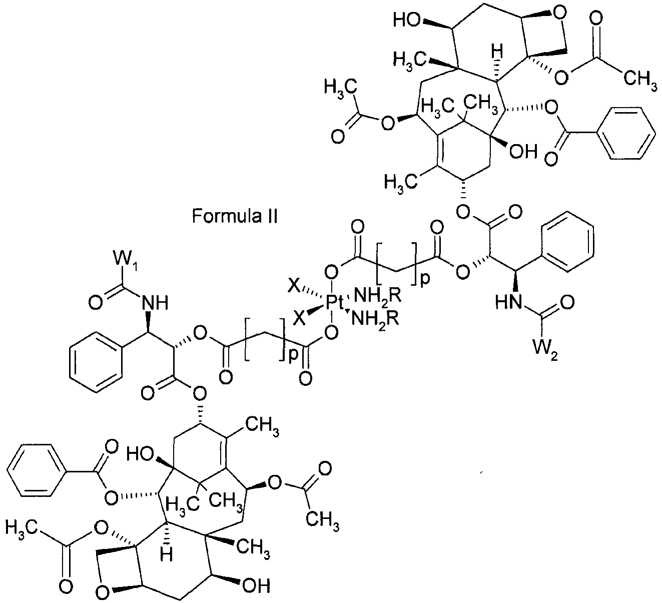 and pharmaceutically acceptable salts thereof, wherein 'p' is an integer from 1 to 10; 'X' is selected from the group consisting of halogen, sulfate, phosphate, nitrate, and water; 'W₁' and 'W₂' are independently selected from phenyl or tert-butyl oxy; and 'R' is hydrogen or alkyl. For instance, 'p' can be 3; 'X' can be chloride; 'W₁' and 'W₂' can be phenyl and 'R' can be hydrogen.

In another aspect, the conjugate has Formula III: 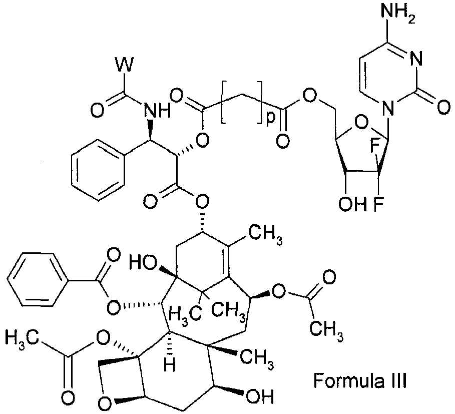 and pharmaceutically acceptable salts thereof, wherein 'p' is an integer from 1 to 10; and 'W' is slected from phenyl or tert-butyl oxy. For instance, 'p' can be 3; and 'W' can be phenyl.

In another aspect, the conjugate has Formula IV: 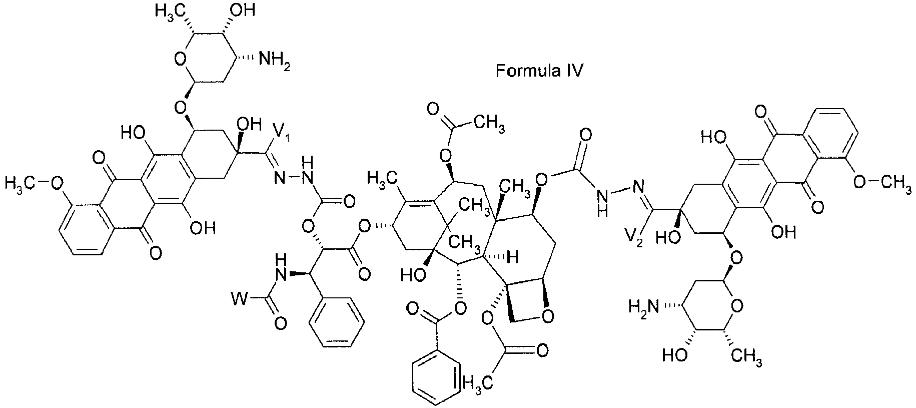 and pharmaceutically acceptable salts thereof, wherein 'W' is phenyl or tert-butyl oxy; and 'V₁' and 'V₂' are independently selected from -CH₃ or -CH₂OH. For instance, 'W' can be phenyl; and 'V₁' and 'V₂' can be --CH₂OH.

In another aspect, the conjugate has Formula V: 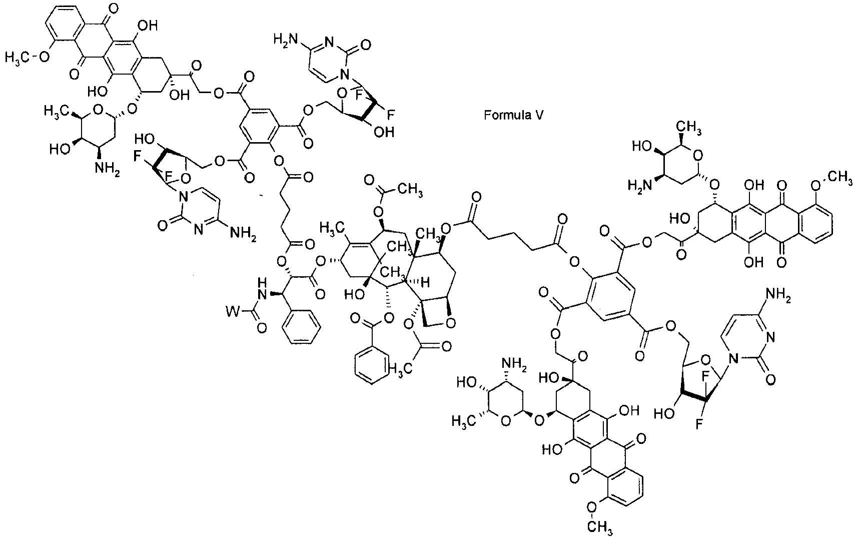 and pharmaceutically acceptable salts thereof, wherein 'W' is phenyl or tert-butyl oxy. For instance, 'W' can be phenyl.

In another aspect, the conjugate has Formula VI: 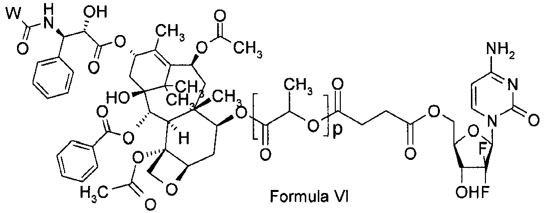 and pharmaceutically acceptable salts thereof, wherein 'p' is an integer from 5 to 20; and 'W' is phenyl or tert-butyl oxy. For instance, 'p' can be 10; and 'W' can be phenyl.

In another aspect, the conjugate has Formula VII: 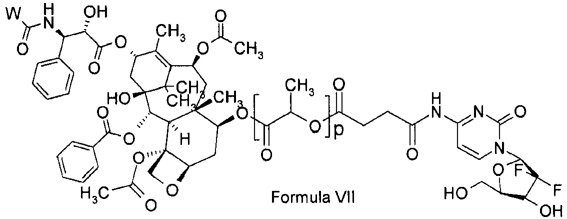 and pharmaceutically acceptable salts thereof, wherein 'p' is an integer from 5 to 20; and 'W' is phenyl or tert-butyl oxy. For instance, 'p' can be 10; and 'W' can be phenyl.

### Multi-Linked Drug Conjugates

In yet another embodiment, a multi-drug conjugate is provided comprising a pharmaceutically active agent covalently bound to a plurality of stimuli-sensitive linkers, wherein each linker is covalently bound to at least one additional pharmaceutically active agent, wherein the conjugate releases at least one pharmaceutically active agent upon delivery to a target cell. Such conjugates can have a conformation similar to a dendrimer, and can comprise a series of conjugates in a chain.

In one aspect, the stimuli-sensitive linker can be a C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, or combinations thereof. For instance, the linker can be a C₃ straight chain alkyl. In yet another instance, the linker can comprise a ketone.

In yet another aspect, the pharmaceutically active agent comprises anticancer chemotherapy agents. For instance, the pharmaceutically active agent can include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

### Pharmaceutical Preparations and Methods of Administration

In another embodiment, a pharmaceutical composition is provided comprising the multi-drug conjugate above, or a pharmaceutically acceptable salt thereof, in a pharmaceutically acceptable vehicle.

The identified nanoparticles and multi-drug conjugates (*i.e.* compounds) treat, inhibit, control and/or prevent, or at least partially arrest or partially prevent, diseases that are treatable by known pharmaceutically active agents and can be administered to a subject at therapeutically effective doses for the inhibition, prevention, prophylaxis or therapy for such diseases. The compounds of the present invention comprise a therapeutically effective dosage of a nanoparticle and/or multi-drug conjugate, a term which includes therapeutically, inhibitory, preventive and prophylactically effective doses of the compounds of the present invention and is more particularly defined below. The subjects treated by administration of the compounds is preferably an animal, including, but not limited to, mammals, reptiles and avians, more preferably horses, cows, dogs, cats, sheep, pigs, and chickens, and most preferably human.

### Therapeutically Effective Dosage

Toxicity and therapeutic efficacy of such compounds can be determined by standard pharmaceutical procedures in cell cultures or experimental animals for determining the LD₅₀ (the dose lethal to 50% of the population) and the ED₅₀, (the dose therapeutically effective in 50% of the population). The dose ratio between toxic and therapeutic effects is the therapeutic index that can be expressed as the ratio LD₅₀/ED₅₀. Compounds that exhibit large therapeutic indices are preferred. While compounds exhibiting toxic side effects may be used, care should be taken to design a delivery system that targets such compounds to the site affected by the disease or disorder in order to minimize potential damage to unaffected cells and reduce side effects.

The data obtained from the cell culture assays and animal studies can be used in formulating a range of dosages for use in humans and other mammals. The dosage of such compounds lies preferably within a range of circulating plasma or other bodily fluid concentrations that include the ED₅₀ with little or no toxicity. The dosage may vary within this range depending upon the dosage form employed and the route of administration utilized. For any compound of the invention, the therapeutically effective dose can be estimated initially from cell culture assays. A dosage may be formulated in animal models to achieve a circulating plasma concentration range that includes the IC₅₀ (the concentration of the test compound that achieves a half-maximal inhibition of symptoms) as determined in cell culture. Such information can be used to more accurately determine useful dosages in humans and other mammals. Compound levels in plasma may be measured, for example, by high performance liquid chromatography.

The amount of a compound that may be combined with a pharmaceutically acceptable carrier to produce a single dosage form will vary depending upon the host treated and the particular mode of administration. It will be appreciated by those skilled in the art that the unit content of a compound contained in an individual dose of each dosage form need not in itself constitute a therapeutically effective amount, as the necessary therapeutically effective amount could be reached by administration of a number of individual doses. The selection of dosage depends upon the dosage form utilized, the condition being treated, and the particular purpose to be achieved according to the determination of those skilled in the art.

The dosage regime for treating a disease or condition with the compounds of the invention is selected in accordance with a variety of factors, including the type, age, weight, sex, diet and medical condition of the patient, the route of administration, pharmacological considerations such as activity, efficacy, pharmacokinetic and toxicology profiles of the particular compound employed, and whether a compound delivery system is utilized. Thus, the dosage regime actually employed may vary widely from subject to subject.

### Formulations and Use

The compounds of the present invention may be formulated by known methods for administration to a subject using several routes which include, but are not limited to, parenteral, oral, topical, intradermal, intramuscular, intraperitoneal, intravenous, subcutaneous, intranasal, epidural, and ophthalmic routes. The individual compounds may also be administered in combination with one or more additional compounds of the present invention and/or together with other pharmaceutically active or inert agents. Such pharmaceutically active or inert agents may be in fluid or mechanical communication with the compound(s) or attached to the compound(s) by ionic, covalent, Van der Waals, hydrophobic, hydrophillic or other physical forces. It is preferred that administration is localized in a subject, but administration may also be systemic.

The compounds of the present invention may be formulated by any conventional manner using one or more pharmaceutically acceptable carriers. Thus, the compounds and their pharmaceutically acceptable salts and solvates may be specifically formulated for administration, *e*.*g*., by inhalation or insufflation (either through the mouth or the nose) or oral, buccal, parenteral or rectal administration. The compounds may take the form of charged, neutral and/or other pharmaceutically acceptable salt forms. Examples of pharmaceutically acceptable carriers include, but are not limited to, those described in REMINGTON'S PHARMACEUTICAL SCIENCES (A.R. Gennaro, Ed.), 21st edition, ISBN: 0781746736 (2005), incorporated herein by reference in its entirety.

The compounds may also take the form of solutions, suspensions, emulsions, tablets, pills, capsules, powders, and the like. Such formulations will contain a therapeutically effective amount of the compound, preferably in purified form, together with a suitable amount of carrier so as to provide the form for proper administration to the patient. The formulation should suit the mode of administration.

### Parenteral Administration

The compound may be formulated for parenteral administration by injection, *e.g.,* by bolus injection or continuous infusion. Formulations for injection may be presented in unit dosage form in ampoules or in multi-dose containers with an optional preservative added. The parenteral preparation can be enclosed in ampoules, disposable syringes or multiple dose vials made of glass, plastic or the like. The formulation may take such forms as suspensions, solutions or emulsions in oily or aqueous vehicles, and may contain formulatory agents such as suspending, stabilizing and/or dispersing agents.

For example, a parenteral preparation may be a sterile injectable solution or suspension in a nontoxic parenterally acceptable diluent or solvent (*e.g*., as a solution in 1,3-butanediol). Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid may be used in the parenteral preparation.

Alternatively, the compound may be formulated in powder form for constitution with a suitable vehicle, such as sterile pyrogen-free water, before use. For example, a compound suitable for parenteral administration may comprise a sterile isotonic saline solution containing between 0.1 percent and 90 percent weight per volume of the compound. By way of example, a solution may contain from about 5 percent to about 20 percent, more preferably from about 5 percent to about 17 percent, more preferably from about 8 to about 14 percent, and still more preferably about 10 percent of the compound. The solution or powder preparation may also include a solubilizing agent and a local anesthetic such as lignocaine to ease pain at the site of the injection. Other methods of parenteral delivery of compounds will be known to the skilled artisan and are within the scope of the invention.

### Oral Administration

For oral administration, the compound may take the form of tablets or capsules prepared by conventional means with pharmaceutically acceptable excipients such as binding agents, fillers, lubricants and disintegrants:

### A. Binding Agents

Binding agents include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, *(e.g.,* Nos. 2208, 2906, 2910), microcrystalline cellulose, and mixtures thereof. Suitable forms of microcrystalline cellulose include, for example, the materials sold as AVICEL-PH-101, AVICEL-PH-103 and AVICEL-PH-105 (available from FMC Corporation, American Viscose Division, Avicel Sales, Marcus Hook, Pennsylvania, USA). An exemplary suitable binder is a mixture of microcrystalline cellulose and sodium carboxymethyl cellulose sold as AVICEL RC-581 by FMC Corporation.

### B. Fillers

Fillers include, but are not limited to, talc, calcium carbonate (e.g., granules or powder), lactose, microcrystalline cellulose, powdered cellulose, dextrates, kaolin, mannitol, silicic acid, sorbitol, starch, pre-gelatinized starch, and mixtures thereof.

### C. Lubricants

Lubricants include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laurate, agar, and mixtures thereof. Additional lubricants include, for example, a syloid silica gel (AEROSIL 200, manufactured by W.R. Grace Co. of Baltimore, Maryland, USA), a coagulated aerosol of synthetic silica (marketed by Deaussa Co. of Plano, Texas, USA), CAB-O-SIL (a pyrogenic silicon dioxide product sold by Cabot Co. of Boston, Massachusetts, USA), and mixtures thereof.

### D. Disintegrants

Disintegrants include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

The tablets or capsules may optionally be coated by methods well known in the art. If binders and/or fillers are used with the compounds of the invention, they are typically formulated as about 50 to about 99 weight percent of the compound. In one aspect, about 0.5 to about 15 weight percent of disintegrant, and particularly about 1 to about 5 weight percent of disintegrant, may be used in combination with the compound. A lubricant may optionally be added, typically in an amount of less than about 1 weight percent of the compound. Techniques and pharmaceutically acceptable additives for making solid oral dosage forms are described in Marshall, SOLID ORAL DOSAGE FORMS, Modern Pharmaceutics (Banker and Rhodes, Eds.), 7:359-427 (1979). Other less typical formulations are known in the art.

Liquid preparations for oral administration may take the form of solutions, syrups or suspensions. Alternatively, the liquid preparations may be presented as a dry product for constitution with water or other suitable vehicle before use. Such liquid preparations may be prepared by conventional means with pharmaceutically acceptable additives such as suspending agents (*e*.*g*., sorbitol syrup, cellulose derivatives or hydrogenated edible fats); emulsifying agents (*e.g*., lecithin or acacia); non-aqueous vehicles (*e.g*., almond oil, oily esters, ethyl alcohol or fractionated vegetable oils); and/or preservatives (*e.g*., methyl or propyl-p-hydroxybenzoates or sorbic acid). The preparations may also contain buffer salts, flavoring, coloring, perfuming and sweetening agents as appropriate. Preparations for oral administration may also be formulated to achieve controlled release of the compound. Oral formulations preferably contain 10% to 95% compound. In addition, the compounds of the present invention may be formulated for buccal administration in the form of tablets or lozenges formulated in a conventional manner. Other methods of oral delivery of compounds will be known to the skilled artisan and are within the scope of the invention.

### Controlled-Release Administration

Controlled-release (or sustained-release) preparations may be formulated to extend the activity of the compound and reduce dosage frequency. Controlled-release preparations can also be used to effect the time of onset of action or other characteristics, such as blood levels of the compound, and consequently affect the occurrence of side effects.

Controlled-release preparations may be designed to initially release an amount of a compound that produces the desired therapeutic effect, and gradually and continually release other amounts of the compound to maintain the level of therapeutic effect over an extended period of time. In order to maintain a near-constant level of a compound in the body, the compound can be released from the dosage form at a rate that will replace the amount of compound being metabolized and/or excreted from the body. The controlled-release of a compound may be stimulated by various inducers, *e.g*., change in pH, change in temperature, enzymes, water, or other physiological conditions or molecules.

Controlled-release systems may include, for example, an infusion pump which may be used to administer the compound in a manner similar to that used for delivering insulin or chemotherapy to specific organs or tumors. Typically, using such a system, the compound is administered in combination with a biodegradable, biocompatible polymeric implant that releases the compound over a controlled period of time at a selected site. Examples of polymeric materials include polyanhydrides, polyorthoesters, polyglycolic acid, polylactic acid, polyethylene vinyl acetate, and copolymers and combinations thereof. In addition, a controlled release system can be placed in proximity of a therapeutic target, thus requiring only a fraction of a systemic dosage.

As an example, an implantable metronomic infusion pump can be used for local delivery of the nanoparticles and multi-drug conjugates of the present invention. *See,* e.g., U.S. Patent Nos. 7,799,016, 7,799,012, 7,588,564, 7,575,574, and 7,569,051, each of which is incorporated herein by reference in its entirety. In this example, a magnetically controlled pump can be implanted into the brain of a patient and deliver the nanoparticles and multi-drug conjugates at a controlled rate corresponding to the specific needs of the patient. A flexible double walled pouch that is formed from two layers of polymer can be alternately expanded and contracting by magnetic solenoid. When contracted, the nanoparticles and multi-drug conjugates can be pushed out of the pouch through a plurality of needles. When the pouch is expanded, surrounding cerebral fluid is drawn into the space between the double walls of the pouch from which it is drawn through a catheter to an analyzer. Cerebral fluid drawn from the patient can be analyzed. The operation of the apparatus and hence the treatment can be remotely controlled based on these measurements and displayed through an external controller.

The compounds of the invention may be administered by other controlled-release means or delivery devices that are well known to those of ordinary skill in the art. These include, for example, hydropropylmethyl cellulose, other polymer matrices, gels, permeable membranes, osmotic systems, multilayer coatings, or a combination of any of the above to provide the desired release profile in varying proportions. Other methods of controlled-release delivery of compounds will be known to the skilled artisan and are within the scope of the invention.

### Inhalation Administration

The compound may also be administered directly to the lung by inhalation. For administration by inhalation, a compound may be conveniently delivered to the lung by a number of different devices. For example, a Metered Dose Inhaler ("MDI") which utilizes canisters that contain a suitable low boiling point propellant, *e.g*., dichlorodifluoromethane, trichlorofluoromethane, dichlorotetrafluoroethane, carbon dioxide or other suitable gas may be used to deliver a compound directly to the lung. MDI devices are available from a number of suppliers such as 3M Corporation, Aventis, Boehringer Ingleheim, Forest Laboratories, Glaxo-Wellcome, Schering Plough and Vectura.

Alternatively, a Dry Powder Inhaler (DPI) device may be used to administer a compound to the lung. DPI devices typically use a mechanism such as a burst of gas to create a cloud of dry powder inside a container, which may then be inhaled by the patient. DPI devices are also well known in the art and may be purchased from a number of vendors which include, for example, Fisons, Glaxo-Wellcome, Inhale Therapeutic Systems, ML Laboratories, Qdose and Vectura. A popular variation is the multiple dose DPI ("MDDPI") system, which allows for the delivery of more than one therapeutic dose. MDDPI devices are available from companies such as AstraZeneca, GlaxoWellcome, IVAX, Schering Plough, SkyePharma and Vectura. For example, capsules and cartridges of gelatin for use in an inhaler or insufflator may be formulated containing a powder mix of the compound and a suitable powder base such as lactose or starch for these systems.

Another type of device that may be used to deliver a compound to the lung is a liquid spray device supplied, for example, by Aradigm Corporation. Liquid spray systems use extremely small nozzle holes to aerosolize liquid compound formulations that may then be directly inhaled into the lung. For example, a nebulizer device may be used to deliver a compound to the lung. Nebulizers create aerosols from liquid compound formulations by using, for example, ultrasonic energy to form fine particles that may be readily inhaled. Examples of nebulizers include devices supplied by Sheffield/Systemic Pulmonary Delivery Ltd., Aventis and Batelle Pulmonary Therapeutics.

In another example, an electrohydrodynamic ("EHD") aerosol device may be used to deliver a compound to the lung. EHD aerosol devices use electrical energy to aerosolize liquid compound solutions or suspensions. The electrochemical properties of the compound formulation are important parameters to optimize when delivering this compound to the lung with an EHD aerosol device. Such optimization is routinely performed by one of skill in the art. Other methods of intra-pulmonary delivery of compounds will be known to the skilled artisan and are within the scope of the invention.

Liquid compound formulations suitable for use with nebulizers and liquid spray devices and EHD aerosol devices will typically include the compound with a pharmaceutically acceptable carrier. In one exemplary embodiment, the pharmaceutically acceptable carrier is a liquid such as alcohol, water, polyethylene glycol or a perfluorocarbon. Optionally, another material may be added to alter the aerosol properties of the solution or suspension of the compound. For example, this material may be a liquid such as an alcohol, glycol, polyglycol or a fatty acid. Other methods of formulating liquid compound solutions or suspensions suitable for use in aerosol devices are known to those of skill in the art.

### Depot Administration

The compound may also be formulated as a depot preparation. Such long-acting formulations may be administered by implantation (e.g., subcutaneously or intramuscularly) or by intramuscular injection. Accordingly, the compounds may be formulated with suitable polymeric or hydrophobic materials such as an emulsion in an acceptable oil or ion exchange resins, or as sparingly soluble derivatives such as a sparingly soluble salt. Other methods of depot delivery of compounds will be known to the skilled artisan and are within the scope of the invention.

### Topical Administration

For topical application, the compound may be combined with a carrier so that an effective dosage is delivered, based on the desired activity ranging from an effective dosage, for example, of 1.0 µM 1.0 mM. In one aspect of the invention, a topical compound can be applied to the skin. The carrier may be in the form of, for example, and not by way of limitation, an ointment, cream, gel, paste, foam, aerosol, suppository, pad or gelled stick.

A topical formulation may also consist of a therapeutically effective amount of the compound in an ophthalmologically acceptable excipient such as buffered saline, mineral oil, vegetable oils such as corn or arachis oil, petroleum jelly, Miglyol 182, alcohol solutions, or liposomes or liposome-like products. Any of these compounds may also include preservatives, antioxidants, antibiotics, immunosuppressants, and other biologically or pharmaceutically effective agents which do not exert a detrimental effect on the compound. Other methods of topical delivery of compounds will be known to the skilled artisan and are within the scope of the invention.

### Suppository Administration

The compound may also be formulated in rectal formulations such as suppositories or retention enemas containing conventional suppository bases such as cocoa butter or other glycerides and binders and carriers such as triglycerides, microcrystalline cellulose, gum tragacanth or gelatin. Suppositories can contain the compound in the range of 0.5% to 10% by weight. Other methods of suppository delivery of compounds will be known to the skilled artisan and are within the scope of the invention.

### Other Systems of Administration

Various other delivery systems are known in the art and can be used to administer the compounds of the invention. Moreover, these and other delivery systems may be combined and/or modified to optimize the administration of the compounds of the present invention.

### Ratiometric Control of Drug-Linker and Drug-Drug Compositions in a Nanoparticle

In yet another embodiment, a method is provided for controlling ratios of conjugated drugs contained in a nanoparticle inner sphere, the method comprising: a) synthesizing a combination of a first drug independently conjugated to a stimuli-sensitive linker, and a second drug independently conjugated to a linker having the same composition, wherein the first drug conjugate and second drug conjugate have a predetermined ratio; b) adding the combination to an agitated solution comprising a polar lipid; and c) adding water to the agitated solution, wherein nanoparticles are produced having a controlled ratio of conjugated drugs contained in the inner sphere. Unlike other methods that require several additional steps to create nanoparticles, the present self assembly of the nanoparticles containing combinations of conjugated drugs is highly efficient.

In one aspect, the first drug and the second drug can independently include an antibiotic, antimicrobial, antiviral, growth factor, chemotherapeutic agent, and combinations thereof. For instance, the first drug and the second drug are independently selected from the group consisting of doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In another aspect, the stimuli-sensitive linker is a pH-sensitive linker. For instance, the stimuli-sensitive linker is selected from the group consisting of C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

In various aspects of the present embodiment, the combination of conjugated drugs having a predetermined ratio further comprises at least one additional drug independently conjugated to a stimuli-sensitive linker having the same composition.

In yet another embodiment, a method is provided for controlling ratios of conjugated drugs contained in a nanoparticle inner sphere, the method comprising: a) synthesizing a combination of (i) a first drug and a second drug conjugated by a first stimuli-sensitive linker, and (ii) a first drug and a second drug conjugated by a second stimuli-sensitive linker, wherein the first drug conjugate and second drug conjugate have a predetermined ratio; b) adding the combination to an agitated solution comprising a polar lipid; and c) adding water to the agitated solution, wherein nanoparticles are produced having a controlled ratio of conjugated drugs contained in the inner sphere.

In one aspect, the first drug and the second drug are independently selected from the group consisting of an antibiotic, antimicrobial, antiviral, growth factor, chemotherapeutic agent, and combinations thereof. For instance, the first drug and the second drug can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In another aspect, the stimuli-sensitive linker is a pH-sensitive linker. For instance, the first stimuli-sensitive linker and the second stimuli-sensitive linker can independently include C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

In various aspects of the present embodiment, the combination of conjugated drugs having a predetermined ratio further comprises at least one additional conjugate of a first drug and a second drug conjugated by a stimuli-sensitive linker other than those present in the combination.

### Methods of Synthesizing Drug-Linker and Drug-Drug Conjugate Containing Nanoparticles

In another embodiment, a method is provided for producing a combination of conjugated drugs having a predetermined ratio in a nanoparticle, said nanoparticle comprising an inner sphere, the method comprising: a) adding to an agitated solution comprising a polar lipid a combination of a first drug independently conjugated to a stimuli-sensitive linker, and a second drug independently conjugated to a linker having the same composition, wherein the first drug conjugate and the second drug conjugate have a predetermined ratio; and b) adding water to the agitated solution, wherein nanoparticles are produced containing in the inner sphere the conjugated drugs having a predetermined ratio. In various aspects, the method can further comprise: c) isolating nanoparticles having a diameter less than about 300 nm.

In various aspects, the first drug and the second drug are independently selected from the group consisting of an antibiotic, antimicrobial, growth factor, chemotherapeutic agent, and combinations thereof. For instance, the first drug and the second drug can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In yet another aspect, the stimuli-sensitive linker is a pH-sensitive linker. For instance, the stimuli-sensitive linker can be C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, or combinations thereof.

In yet another aspect, the combination of conjugated drugs having a predetermined ratio further comprise a third drug independently conjugated to a stimuli-sensitive linker having the same composition. In various aspects, the solution comprising a polar lipid further comprises a functionlalized polar lipid.

In yet another embodiment, a method is provided for producing a combination of conjugated drugs having a predetermined ratio in a nanoparticle, said nanoparticle comprising an inner sphere, the method comprising: a) adding to an agitated solution comprising a polar lipid a combination of (i) a first drug and second drug conjugated by a first stimuli-sensitive linker, and (ii) a first drug and a second drug conjugated by a second stimuli-sensitive linker, wherein the first drug conjugate and second drug conjugate have a predetermined ratio; and b) adding water to the agitated solution, wherein nanoparticles are produced containing in the inner sphere the conjugated drugs having a predetermined ratio. In various aspects, the method can further comprise: c) isolating nanoparticles having a diameter less than about 300 nm.

In one aspect, the first drug and the second drug can independently include an antibiotic, antimicrobial, growth factor, chemotherapeutic agent, and combinations thereof. For instance, the first drug and the second drug are independently selected from the group consisting of doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In another aspect, the stimuli-sensitive linker is a pH-sensitive linker. For instance, the first stimuli-sensitive linker and the second stimuli-sensitive linker can independently be C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

In various aspects of the present embodiment, the combination of conjugated drugs having a predetermined ratio further comprises at least one additional conjugate of a first drug and a second drug conjugated by a stimuli-sensitive linker other than those present in the combination. In various aspects, the solution comprising a polar lipid further comprises a functionlalized polar lipid. An example of a polar lipid is a phospholipid as defined herein.

### Methods of Treating Diseases and Conditions in a Subject

The pharmaceutically active agents used in the present invention are known to provide a certain response when administered to subjects. One of skill in the art will readily be able to choose particular pharmaceutically active agents to use with the nanoparticles and multi-drug conjugates to treat certain diseases or conditions, including those listed in the appended tables. In addition, the literature is replete with examples of administering pharmaceutically active agents to subjects, especially those regulated by the government.

Therefore, a method is provided for treating a disease or condition, the method comprising administering a therapeutically effective amount of the nanoparticle above to a subject in need thereof. In one aspect, the disease is a proliferative disease including lymphoma, renal cell carcinoma, prostate cancer, lung cancer, pancreatic cancer, melanoma, colorectal cancer, ovarian cancer, breast cancer, glioblastoma multiforme and leptomeningeal carcinomatosis. In another aspect, the disease is a heart disease including Atherosclerosis, Ischemic heart disease, Rheumatic heart disease, Hypertensive heart disease, Infective endocarditis, Coronary heart disease, and Constrictive pericarditis. In another aspect, the disease is an ocular disease selected from the group consisting of macular edema, retinal ischemia, macular degeneration, uveitis, blepharitis, keratitis, rubeosis iritis, iridocyclitis, conjunctivitis, and vasculitis. In another aspect, the disease is a lung disease including asthma, Chronic Bronchitis, Cystic Fibrosis, Emphysema, Pneumonia, lung cancer, Primary Pulmonary Hypertension, Pulmonary Arterial Hypertension, and Tuberculosis. In yet another aspect, the disease includes bacterial infection, viral infection, fungal infection, and parasitic infection.

In various aspects of the present embodiment, the nanoparticle is administered systemically. In another aspect, the nanoparticle is administered locally. In yet another aspect, the local administration is via implantable metronomic infusion pump.

In yet another embodiment, a method is provided for treating a disease or condition, the method comprising administering a therapeutically effective amount of the multi-drug conjugate above to a subject in need thereof. In one aspect, the disease is a proliferative disease including lymphoma, renal cell carcinoma, prostate cancer, lung cancer, pancreatic cancer, melanoma, colorectal cancer, ovarian cancer, breast cancer, glioblastoma multiforme and leptomeningeal carcinomatosis. In one aspect, the disease is a heart disease including Atherosclerosis, Ischemic heart disease, Rheumatic heart disease, Hypertensive heart disease, Infective endocarditis, Coronary heart disease, and Constrictive pericarditis. In one aspect, the disease is an ocular disease including macular edema, retinal ischemia, macular degeneration, uveitis, blepharitis, keratitis, rubeosis iritis, iridocyclitis, conjunctivitis, and vasculitis. In one aspect, the disease is a lung disease including asthma, Chronic Bronchitis, Cystic Fibrosis, Emphysema, Pneumonia, lung cancer, Primary Pulmonary Hypertension, Pulmonary Arterial Hypertension, and Tuberculosis. In yet another aspect, the disease is selected from the group consisting of bacterial infection, viral infection, fungal infection, and parasitic infection.

In various aspects of the present embodiment, the multi-drug conjugate is administered systemically. In another aspect, the multi-drug conjugate is administered locally. In yet another aspect, the local administration is via implantable metronomic infusion pump.

### Methods of Sequentially Delivering a Pharmaceutically Active Drug to a Target

In yet another embodiment, a method is provided for sequentially delivering a drug conjugate to a target cell. Preferably, a combination of drug-drug conjugates having individual linkers of varying sensitivities is administered in an environment whereby one individual linker is triggered first, followed by another individual linker triggered at another condition. Therefore, the method comprises administering a nanoparticle above to the target cell and triggering multi-drug conjugate release. In various aspects of the present embodiment, the nanoparticle is administered systemically. In another aspect, the nanoparticle is administered locally. In yet another aspect, the local administration is via implantable metronomic infusion pump.

### Methods of Nanoencapsulation with High Loading Efficiency

In yet another embodiment, a method is provided for nanoencapsulation of a plurality of drugs comprising: separately linking each of the plurality of drugs with a corresponding polymer backbone with nearly 100% loading efficiency by forming the corresponding polymer backbone by ring opening polymerization beginning with the corresponding drug, wherein each of the corresponding polymer backbones has the same or similar physicochemical properties and has approximately the same chain length; mixing the plurality of linked drugs and polymers at selectively predetermined ratios at selectively and precisely controlled drug ratios; and synthesizing the mixed plurality of linked drugs and polymers into a nanoparticle.

In various aspects, the plurality of drugs can independently include an antibiotic, antimicrobial, growth factor, chemotherapeutic agent, and combinations thereof. For instance, the plurality of drugs can independently include doxorubicin, camptothecin, gemicitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansanol, epothilone A, combretastatin, pharmaceutically active analogs thereof, and pharmaceutically acceptable salts thereof.

In various aspects, the polymer backbone is a stimuli-sensitive linker. For instance, the stimuli-sensitive linker can include a C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

### Kits

In various embodiments, the present invention can also involve kits. Such kits can include the compounds of the present invention and, in certain embodiments, instructions for administration. When supplied as a kit, different components of a compound formulation can be packaged in separate containers and admixed immediately before use. Such packaging of the components separately can, if desired, be presented in a pack or dispenser device which may contain one or more unit dosage forms containing the compound. The pack may, for example, comprise metal or plastic foil such as a blister pack. Such packaging of the components separately can also, in certain instances, permit long-term storage without losing activity of the components. In addition, if more than one route of administration is intended or more than one schedule for administration is intended, the different components can be packaged separately and not mixed prior to use. In various embodiments, the different components can be packaged in one combination for administration together.

Kits may also include reagents in separate containers such as, for example, sterile water or saline to be added to a lyophilized active component packaged separately. For example, sealed glass ampules may contain lyophilized compounds and in a separate ampule, sterile water, sterile saline or sterile each of which has been packaged under a neutral non-reacting gas, such as nitrogen. Ampules may consist of any suitable material, such as glass, organic polymers, such as polycarbonate, polystyrene, ceramic, metal or any other material typically employed to hold reagents. Other examples of suitable containers include bottles that may be fabricated from similar substances as ampules, and envelopes that may consist of foil-lined interiors, such as aluminum or an alloy. Other containers include test tubes, vials, flasks, bottles, syringes, and the like. Containers may have a sterile access port, such as a bottle having a stopper that can be pierced by a hypodermic injection needle. Other containers may have two compartments that are separated by a readily removable membrane that upon removal permits the components to mix. Removable membranes may be glass, plastic, rubber, and the like.

In certain embodiments, kits can be supplied with instructional materials. Instructions may be printed on paper or other substrate, and/or may be supplied as an electronic-readable medium, such as a floppy disc, mini-CD-ROM, CD-ROM, DVD-ROM, Zip disc, videotape, audio tape, and the like. Detailed instructions may not be physically associated with the kit; instead, a user may be directed to an Internet web site specified by the manufacturer or distributor of the kit, or supplied as electronic mail.

### EXAMPLES

Aspects of the present teachings may be further understood in light of the following examples, which should not be construed as limiting the scope of the present teachings in any way.

### Example 1- Ratiometric Combinatorial Drug and Nanoparticle Synthesis

### Materials.

L-lactide was purchased from Sigma-Aldrich Co. (Milwaukee, WI), recrystallized three times in ethylacetate and dried under vacuum. L-lactide crystals were further dried inside a glove box and sealed into a glass vial under dry argon and then stored at -20 °C prior to use. 2,6-di-iso-propylaniline (Sigma-Aldrich Co.) and 2,4-pentanedione (Alfa Aesar Co., Ward Hill, MA) were used as received. All other chemicals and anhydrous solvents were purchased from Sigma-Aldrich Co. unless otherwise specified. Anhydrous tetrahydrofuran (THF) and toluene were prepared by distillation under sodium benzophenone and were kept anhydrous by using molecular sieves. The 2-((2,6-diisopropylphenyl)amino)-4-((2,6-diisopropylphenyl)imino)-2-pentene (BDI) ligand and the corresponding metal catalysts (BDI)ZnN(SiMe₃)₂ were prepared inside a glove box following a published protocol and stored at -20 °C prior to use (B. M. Chamberlain, M. Cheng, D. R. Moore, T. M. Ovitt, E. B. Lobkovsky, G. W. Coates, J Am Chem Soc 2001, 123, 3229-3238). DOX·HCl was purchased from Jinan Wedo Co., Ltd. (Jinan, China) and used as received. Removal of HCl from DOX·HCl was achieved by neutralizing DOX·HCl solution in water with triethyleamine, after which the solution color changed from red to purple. The free base form of DOX was subsequently extracted with dichloromethane. The organic extract was filtered through anhydrous Na₂SO₄ and dried under vacuum to collect DOX crystals. *(S)*-*(*+*)-*Camptothecine (CPT) was purchased from TCI America and used as received.

### Synthesis of 2-((2,6-diisopropylphenyl)amino)-4-((2,6-diisopropylphenyl)imino)-2-pentene (BDI).

Ligand BDI was prepared following a previously published protocol with minor modification (B. M. Chamberlain, M. Cheng, D. R. Moore, T. M. Ovitt, E. B. Lobkovsky, G. W. Coates, J Am Chem Soc 2001, 123, 3229-3238). Briefly, 2,6-Di-*n-*propylaniline (13.0 mmol) and 2,4-pentanedione (6.5 mmol) in the ratio of 2:1 were dissolved in absolute ethanol (20 ml). The mixture solution was acidified with concentrated HCl (0.6 mL) and heated at reflux for 48 h, which resulted in white precipitates. After being cooled to room temperature, the white precipitates were dissolved with dichloromethane and saturated aqueous bicarbonate solution. The orange colored solution was then extracted and washed with brine three times and filtered through anhydrous Na₂SO₄, followed by being concentrated and precipitated in hexane. The resulting precipitates were collected by filtration, suspended in diethyl ether (20 mL), and washed with saturated aqueous bicarbonate followed by brine. The organic layer was then separated through filtration in the presence of Na₂SO₄ to absorb moisture and then precipitated in hexane as a light brown powder (yield ~ 60%). ¹H NMR (JEOL, CDCl₃, 500 MHz): *δ* 12.20 (br, 1H, NH), 7.12 (m, 6H, ArH), 4.83 (s, 1H, Hβ), 3.10 (m, 4H, CHMe₂), 1.72 (s, 6H, α-Me), 1.22 (d, 12H, CHMeMe), 1.12 (d, 12H, CHMeMe) ppm. ESI-MS (positive): m/z = 419.43 [M+H]⁺.

### Synthesis of (BDI)ZnN(SiMe3)2 catalyst.

Zinc bis-(trimethylsilyl)amide (463 mg, 1.19 mmol) in toluene (20 mL) was added into a solution of BDI (500 mg, 1.19 mmol) in toluene (20 mL). The mixture solution was stirred for 18 h at 80 °C and then the solvent was removed under vacuum to form (BDI)ZnN(SiMe₃)₂ as a light yellow solid, which was recrystallized from toluene at -30 °C to yield colorless blocks (yield ~ 70%).¹H NMR (JEOL, C₆D₆, 500 MHz): *δ* (br, 1H, NH), 6.9-7.13 (m, 6H, ArH), 4.85 (s, 1H,Hβ), 3.25 (m, 4H, CHMe₂), 1.67 (s, 6H, α-Me), 1.1-1.25 (d, 12H+12H=24H, CHMeMe), 0.08-0.1 (18H, s, SiCH₃) ppm.

### Ring opening polymerization of l-lactide.

Following previously published protocols, DOX-PLA and CPT-PLA polymers were synthesized through ring opening polymerization of l-lactide initiated by alkoxy complex of (BDI)ZnN(SiMe₃)₂ in a glove box under argon environment at room temperature. For the synthesis of DOX-PLA, (BDI)ZnN(SiMe₃)₂ (6.4 mg, 0.01 mmol) and DOX (5.4 mg, 0.01 mmol) were mixed in 0.5 mL of anhydrous THF. L-lactide (101.0 mg, 0.7 mmol) dissolved in 2 mL anhydrous THF was added dropwise. After the l-lactide was completely consumed, the crude product was precipitated in cold diethyl ether, yielding DOX-PLA conjugates. The CPT-PLA conjugates were synthesized in the same procedures as the DOX-PLA. These drug-polymer conjugates had a molecular weight of about 10,000 g/mole determined by gel permeation chromatography.

### Synthesis of lipid-coated drug-polymer conjugate nanoparticles.

Lipid-polymer hybrid nanoparticles with polymeric cores consisting of the synthesized drug-polymer conjugates were prepared through a nanoprecipitation method (L. Zhang, J. M. Chan, F. X. Gu, J. W. Rhee, A. Z. Wang, A. F. Radovic-Moreno, F. Alexis, R. Langer, O. C. Farokhzad, ACS Nano 2008, 2, 1696-1702). In detail, 200 ug of egg PC (Avanti Polar Lipids Inc.) and 260 ug of 1,2- distearoyl-sn-glycero-3- phosphoethanolamine-N-carboxy(polyethyleneglycol)-2000 (DSPE-PEG-COOH) (Avanti Polar Lipids Inc.) were dissolved in 4% ethanol and stirred and heated at 68 °C for 3 min. A total of 500 ug of DOX-PLA and CPT-PLA was dissolved in acetonitrile and added dropwise to the lipid solution while stirring. The solution was then vortexed for 3 min followed by the addition of deionized water (1 mL). Then the diluted solution was stirred at room temperature for 2 h, washed with PBS buffer using an Amicon Ultra centrifugal filter with a molecular weight cutoff of 100 kDa (Millipore, Billerica, MA), and resuspended in 1mL of PBS. Nanoparticles with different DOX/CPT drug ratios were prepared by adjusting the amount of each type of drug-polymer conjugates while keeping the total polymer weight at 500 ug. The nanoparticle size and surface zeta potential were obtained from three repeat measurements by dynamic light scattering (DLS) (Malvern Zetasizer, ZEN 3600) with a backscattering angle of 173°. The morphology of the particles was characterized by scanning electron microscopy (SEM) (Phillips XL30 ESEM). Samples for SEM were prepared by dropping nanoparticle solution (5 µL) onto a polished silicon wafer. After drying the droplet at room temperature overnight, the sample was coated with chromium and then imaged by SEM. The drug loading yield of the synthesized nanoparticles was determined by UV-spectroscopy (TECAN, infinite M200) using the maximum absorbance at 482 nm for DOX and 362 nm for CPT. No shift in the absorbance peak was observed between the free drugs and their polymer conjugates. Standard calibration curves of both DOX and CPT at various concentrations were obtained to quantify drug concentrations in the nanoparticles.

### Cellular colocalization and cytotoxicity studies.

The MDA-MB-435 cell line was maintained in Dulbecco's modification of Eagle's medium (DMEM, Mediatech, Inc.) supplemented with 10% fetal calf albumin, penicillin/streptomycin (GIBCO^{®}), L-glutamine (GIBCO^{®}), nonessential amino acids, sodium bicarbonate, and sodium pyruvate (GIBCO^{®}). The cells were cultured at 37°C and 5% CO₂. For the dual-drug colocalization and cellular internalization study, the cells were incubated with dual-drug loaded nanoparticles for 4 h, washed with PBS, and fixed on a chamber slide for fluorescence microscopy imaging. The cytotoxicity of the dual-drug loaded nanoparticles was assessed using the (3-(4,5-dimethylthiazol-2-yl)-2,5-diphenyltetrazolium bromide (MTT) assay (Promega, Madison, WI). Briefly, the cells were seeded at 25% confluency (∼4×10³ cells/well) in 96-well plates and incubated with different concentrations of drug loaded nanoparticles for 24 h. The cells were then washed with PBS three times and incubated in fresh media for an additional 72h. MTT assay was then applied to the samples to measure the viability of the cells following the manufacturer's instruction.

### Results

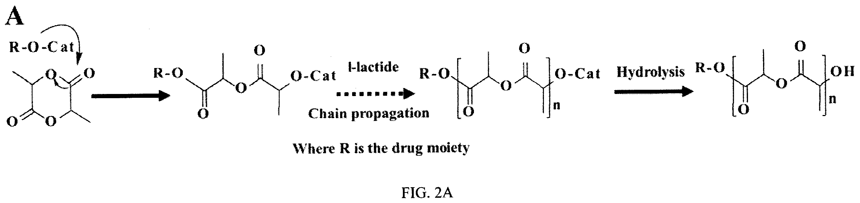

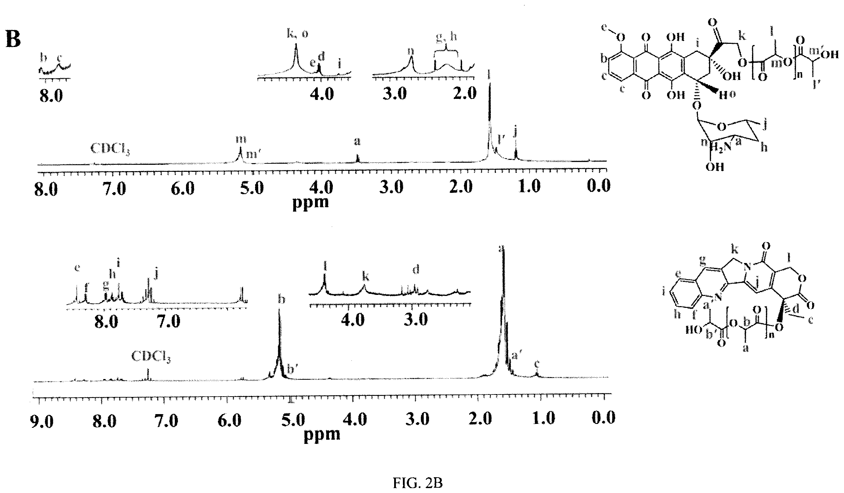

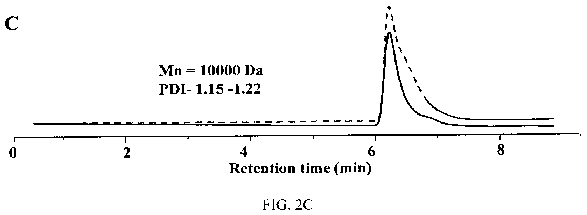

In the study, we used (BDI)ZnN(SiMe₃)₂, a metal-amido complex in which BDI refers to 2-((2,6-diisopropylphenyl)amido)-4-((2,6diisopropylphenyl) -imino)-2-pentene, as a catalyst for the in-situ formation of metal-alkoxide with the hydroxyl group of DOX and CPT to initiate the living polymerization of l-lactide and form drug-poly-l-lactide (drug-PLA) conjugates (Figure 2A). The formation of the drug-polymer conjugates was verified by the ¹H-NMR spectroscopy, which exhibits all the characteristic proton resonance peaks corresponding to the parent drug molecules. The appearance of the aromatic proton resonance at δ 7.5 to 8.0 ppm in DOX-PLA conjugates (Figure 2B, top panel) and δ 7.5 to 8.5 ppm in CPT-PLA conjugates (Figure 2B, bottom panel) along with the characteristic -CH3 proton of PLA at δ 1.5 ppm and -CH proton at δ 5.2 ppm confirms the formation of the drug-polymer conjugates. The desired drug-polymer conjugation products were further validated by gel permeation chromatography (GPC) which shows the molecular weight as 10,000 Dalton for both DOX-PLA and CPT-PLA conjugates (Figure 2C). The molecular weight is in accord with the monomer-to-initiator feed ratio which indicates near 100% conversion of the monomers to polymers. Since the formation of metal alkoxide complex is quantitative and the reaction is homogeneous, the reaction proceeded quantitatively such that all monomers were converted into products. Also the molecular weight of the polymer matches that from an earlier study conducted by Tong et al. who used (BDI)ZnN(SiMe₃)₂ to catalyze the ring opening polymerization of both DOX and CPT. (R. Tong, J. Cheng, Bioconjug Chem 2010, 21, 111-121; R. Tong, J. Cheng, J Am Chem Soc 2009, 131, 4744-4754).

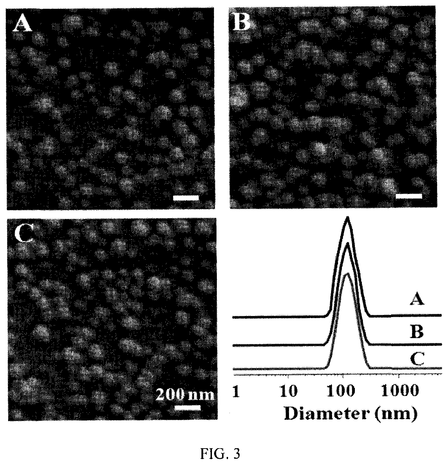

Upon successful synthesis of the drug-polymer conjugates, we used them to prepare lipid-polymer hybrid nanoparticles for dual-drug delivery. Using DSPE-PEG and phospholipids to coat the polymeric nanoparticle core, the resulting lipid-polymer hybrid nanoparticles are highly stable in water, PBS and serum and have high drug loading yield as the entire polymeric core consists of the drug-polymer conjugates. Moreover, by simply adjusting the DOX-PLA:CPT-PLA molar ratio, dual-drug loaded nanoparticles with ratiometric drug loading of DOX and CPT were prepared. Keeping the total drug-polymer conjugates weight constant at 1mg, we varied the DOX-PLA:CPT-PLA ratio to tune the ratiometric drug loading. The resulting drug-loaded nanoparticles exhibit a unimodal size distribution at ∼100 nm with low PDI values (Figure 3). In addition, the particles possess negative surface zeta potential, which is consistent with the DSPE-PEG-COOH coating and serves to prevent the particles from aggregation. The particle size measured by DLS was consistent with the SEM images of the particles (Figure 3).

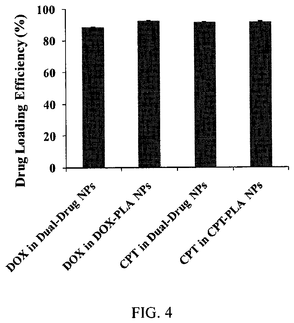

Following the physicochemical characterization of the particles, we next examined the drug loading efficiency in these drug-polymer conjugate nanoparticle systems. We prepared various formulations of the nanoparticles with different ratios of drug-polymer conjugates and found that, in all cases, over 90% of the conjugates were encapsulated into the nanoparticles (Figure 4). No change in loading efficiency was observed when DOX-PLA and CPT-PLA conjugates were loaded in combination or separately, presumably due to the fact that the long and sharply distributed PLA polymer chain gives each drug molecule a predominant and uniform hydrophobic property. Therefore, they were completely encapsulated and stabilized by the lipid and the lipid-PEG layers in the lipid-polymer hybrid nanoparticle system. Furthermore, we varied the DOX-PLA: CPT-PLA molar ratios from 1:1, to 3:1 and to 1:3, while keeping the total drug-polymer conjugates mass constant. It was found that the final loading yields of DOX and CPT in the dual-drug loaded nanoparticles were highly consistent with the initial DOX-PLA: CPT-PLA molar ratios (supporting information the following table).

**TABLE 1 - Characteristic features of the lipid-coated drug-polymer conjugate nanoparticles**

| | | | | | |
|---|---|---|---|---|---|
| DOX-PLA/CPT-PLA molar ratios | 1:0 | 0:1 | 1:1 | 3:1 | 1:3 |
| Particle size (nm) | | | 100±2 | | |
| Particle PDI | | | 0.17-0.22 | | |
| Particle zeta potential (mV) | | | -47±2 | | |
| DOX loading (µM) | 47.8±0.2 | 0 | 24.0+0.1 | 35.8±0.2 | 12.0±0.8 |
| CPT loading (µM) | 0 | 48.2±0.1 | 24.4±0.1 | 12.3±0.1 | 36.2±0.2 |

These results further confirm that this approach enables one to encapsulate different types of drugs to the same nanoparticles with ratiometric control over drug loading.

Upon verifying the excellent drug loading efficiency in the present system, we then examined whether the different drug-polymer conjugates are loaded into the same nanoparticles as opposed to forming two different particle populations. To this end, we studied the colocalization of the two drug molecules and their internalization into cells through fluorescence microscopy. Since DOX is also a highly fluorescent molecule, the DOX-PLA conjugates can be identified from DOX's characteristic fluorescence wavelength (excitation/emission = 540 nm/600 nm). To visualize CPT-PLA, we attached a fluorescent probe, 6-((7-amino-4-mehylcoumarin-3-acetyl) amino) hexanoic acid succinimidyl ester (excitation/emission = 353 nm/442 nm), to the hydroxyl end of the CPT-PLA. Figure 5A shows the fluorescence microscopy images that exhibit the colocalization of the DOX-PLA and the CPT-PLA-probe. The colocalization study indicates that no segregation between the two types of drug-polymer conjugates occurs and each particle contains both DOX and CPT.

After having confirmed that the nanoparticles contain a mixture of DOX and CPT, we next examined the cytotoxicity of these dual-drug loaded nanoparticles in comparison to the cocktail mixtures of the corresponding single-drug loaded nanoparticles against MDA-MB-435 breast cancer cells in vitro. The cocktail system was prepared by mixing DOX-PLA loaded nanoparticles and CPT-PLA loaded nanoparticles at a ratio that is equivalent to the DOX-PLA:CPT-PLA molar ratio in the dual-drug nanoparticles. Figure 5B shows the results of IC50 measurements of the dual-drug loaded nanoparticles and cocktail combination of single-drug loaded nanoparticles. It was found that the dual-drug loaded nanoparticles consistently showed higher potency as compared to the cocktail systems for the 3 different drug ratios. In the 3:1, 1:1, and 1:3 DOX-PLA:CPT-PLA combinations, the dual-drug loaded nanoparticles showed an enhancement in efficacy by 3.5, 2.5, and 1.1 times, respectively, compared to the cocktail particle mixtures. This enhanced cytotoxicity of the dual-drug delivery system can be explained, at least partially, by the fact that dual-drug loaded nanoparticles can deliver more consistent combination drug payloads when compared to cocktail nanoparticle systems and hence maximize their combinatorial effect. In the cocktail mixture, variations in the nanoparticle uptake and the random drug distribution in cells likely compromised the efficacy of the drug combinations. Figure 5 suggests that the dual-drug loaded nanoparticles enable concurrent combination drug delivery through particle endocytosis. Once engulfed by the plasma membrane, nanoparticles are transported by endosomal vesicles before unloading their drug payloads. This endocytic uptake mechanism is particularly favourable to the drug-polymer conjugate system used in the present combinatorial drug delivery scheme. The pH drop associated with endosome maturation subjects the nanoparticles to an acidic environment and enzymatic digestions, which facilitate the cleavage of the ester linkage between the drug and the polymers. In addition, the degradation of the polymer PLA releases lactic acid to further lower the pH surrounding the nanoparticles, thereby further accelerating the drug release.

In conclusion, a new and robust approach for combination chemotherapy was presented by incorporating two different types of drugs with ratiometric control over drug loading into a single polymeric nanoparticle. By adapting metal alkoxide chemistry, drug conjugated polymers were synthesized in a quantitative yield with 100% monomer conversion, resulting in the formation of highly hydrophobic drug-polymer conjugates. These drug-polymer conjugates were successfully encapsulated into lipid-coated polymeric nanoparticles with over 90% loading efficiency. Using DOX and CPT as two model chemotherapy drugs, various ratios of DOX-PLA and CPT-PLA were loaded into the nanoparticles, yielding particles that are uniform in size, size distribution and surface charge. The cytotoxicity of these dual-drug carrying nanoparticles was compared with their cocktail their cocktail mixtures of single-drug loaded nanoparticles and showed superior therapeutic effect. This strategy can also be exploited for various other chemotherapeutic agents containing hydroxyl groups as well as different types of combinations for combinatorial treatments of various diseases. While only two drugs (DOX and CPT) were used to demonstrate the concept of this combinatorial drug delivery approach, this method can be generalized to incorporate three or more different types of drugs into the same nanoparticles with ratiometro control over drug loading.

### Example 2 - Synthesis of Multi-Drug Conjugates

### Synthesis of PTXL-GEM Conjugates

Paclitaxel (PTXL) and Gemcitabine hydrochloride (GEM) were purchased from ChemiTek Company and used without further purification. All other materials including solvents were purchased from Sigma-Aldrich Company, USA. Single addition luminescence ATP detection assay for cytotoxicity measurement was purchased from PerkinElmer Inc. ¹H NMR spectra were recorded in CDCl₃ using a Varian Mercury 400 MHz spectrometer. Electrospray ionization mass spectrometry (ESI-MS, Thermo LCQdeca mass spectrometer) and Thermo Fisher Scientific LTQ-XL Orbitrap mass spectrometer were used to determine the mass and molecular formula of the compounds, respectively. Reversed phase HPLC purification was performed on an Varian HPLC system equipped with µ-bonapack C18 column (4.6 mm × 150 mm, Waters Associates, Inc.) using acetonitrile and water (50/50, v/v) as mobile phase. Thin-layer chromatography (TLC) measurements were carried out using pre-coated silica gel HLF250 plates (Advenchen Laboratories, LLC, USA). 4-(*N,N-*dimethylamino) pyridinium-4-toluenesulfonate (DPTS) was prepared by mixing saturated THF solutions of *N,N*-dimethylaminopyridine (DMAP) (1 equiv) and *p*-toluenesulfonic acid monohydrate (1 equiv) at room temperature. The precipitate was filtered, washed three times with tetrahydrofuran (THF), and dried under vacuum.

### Synthesis of compound 1.

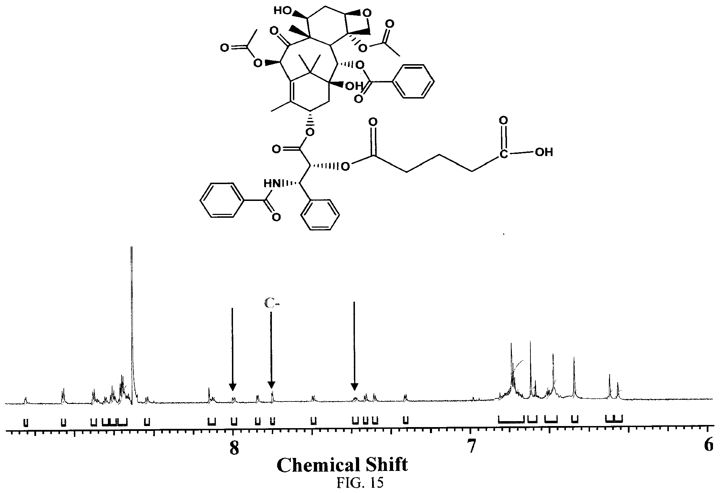

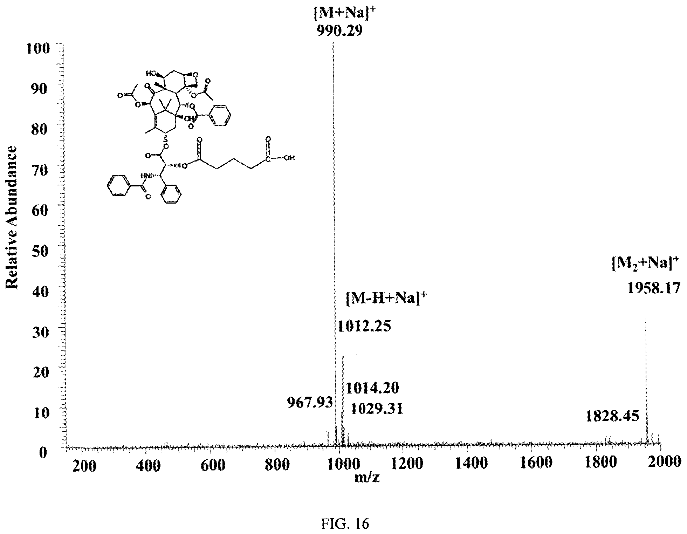

Paclitaxel (5 mg, 5.8 µmol) and glutaric anhydride (2 mg. 17.5 µmol) were dissolved in 200 µL dry pyridine. To this solution, DMAP (0.57 µmol) dissolved in 10 µL pyridine was added and the solution was stirred at room temperature for 3 hrs. The reaction was monitored by TLC using 9.2/0.8 (v/v) CHCl₃/MeOH as an eluent (product Rf = 0.42). The complete disappearance of the starting paclitaxel (Rf = 0.54) occurred after 3 hrs of reaction. Then the reaction was quenched by diluting the solution with dichloromethane (DCM), followed by extracting DMAP and pyridine with DI water. The remaining dichloromethane solution was concentrated and precipitated in hexane, resulting in 5.1 mg of the compound 1 as a white powder. The production yield was about 90%. ¹H NMR (CDCl₃, δ ppm) was carried out to characterize the produced compound 1 (Figure 15): 1.14 (s, 3H), 1.25 (s, 3H), 1.69 (s, 3H), 1.9-2.06 (broad, 7H), 2.16-2.27 (br, 4H), 2.2-2.7 (br, 14 H), 3.82 (d, 1H), 4.21 (d,1H), 4.32 (d-1H), 4.48 (t, 1H), 5.0 (d, 1H), 5.5 (d, 1H), 5.69 (d, 1H), 6.0 (d, 1H), 6.3 (br, 2H), 7.09 (d, 1H), 7.3-7.4(m, 7H), 7.5 (m, 3H), 7.6 (m, 1H), 7.74 (d, 2H), 8.13 (d, 2H), 8.6 (s, 1H). The mass of compound 1 was then determined by ESI-MS (positive) m/z 990.29 (M+Na)⁺ (Figure 16).

### Synthesis of PTXL-GEM conjugate (compound 2)

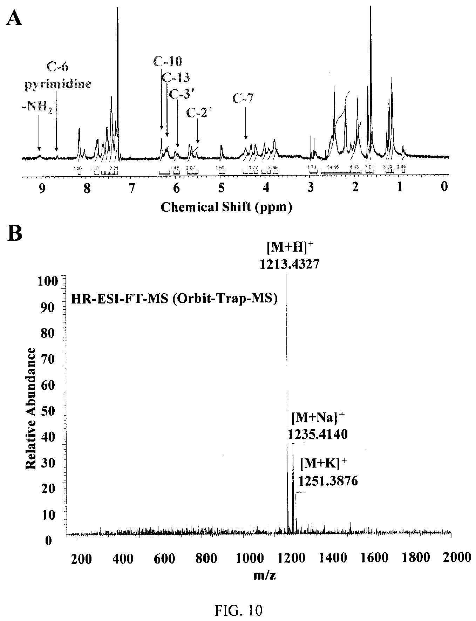

Compound 1 (5 mg, 5.2 µmol) was dissolve in 0.5 mL dry DCM containing DTPS (4.6 mg, 15.6 µmol). To the solution, a solution of GEM (1.5 mg, 5.2 µmol) dissolved in 0.5 mL dry N,N-dimethylformamide (DMF) was added and solution was stirred for 15 min. After 15 min of reaction, DIPC (5 mg, 39 µmol) in 0.1 mL pyridine was added slowly to the solution and reaction was carried on at room temperature for 24 hrs. The reaction was monitored by TLC using 9.2:0.8 (v/v) CHCl₃/MeOH as an eluent (product Rf = 0.22). The complete disappearance of the starting compound 1 (Rf = 0.42) occurred after 24 hrs of reaction. The reaction was then quenched by diluting the solution with dichloromethane (DCM), followed by extracting DPTS, DIPC, DMF, and pyridine with DI water. The remaining dichloromethane solution was concentrated and precipitated in hexane resulting in 6.1 mg of the compound 2 as a white powder. The production yield was about 86%. The resulting product was purified by HPLC using acetonitrile/water (50/50, v/v) as an eluent. Then ¹H NMR (CDCl₃, δ ppm) was carried out to characterize the produced compound 2 (Figure 10A): 0.91 (s, 1H), 1.14 (s, 3H), 1.22 (s,3H), 1.27(s,3H), 1.62 (s, 7H), 1.67 (s, 3H), 1.9-1.2 (br, 8H), 2.2-2.7 (br, 14H),2.89 (d, 2H), 3.7 (d, 2H), 3.85 (d, 2H), 3.9 (d, 1H), 4.32 (d, 1H), 4.48 (t, 1H), 5.0 (d, 1H), 5.5 (d, 1H), 5.69 (d, 1H), 6.0 (d, 1H), 6.3 (br, 3H) 7.28 (s, 3H), 7.4 (m, 5H), 7.5 (m, 3H), 7.6 (m, 1H), 7.74 (d, 2H), 8.13 (d, 2H), 8.75 (d, 1H), 9.1 (-NH₂, pyrimidine ring). The mass and molecular formula of compound 2 were then determined by HR-ESI-FT-MS (orbit-trap-MS, positive) m/z 1213.4327 [M+H]⁺, 1235.4140 [M+Na]⁺. Calcd for C₆₁H₆₆F₂N₄O₂₀ : 1213.4311. Found: 1213.4327 (Figure 10B).

### Hydrolysis of PTXL-GEM conjugate (compound 2)

Hydrolysis study of PTXL-GEM conjugates was performed to confirm that the conjugates can be hydrolyzed to free PTXL and free GEM and to measure its hydrolysis kinetics at different pH values. In the study, PTXL-GEM conjugates were incubated in aqueous solutions with a pH value of 6.0 or 7.4 at 37 °C. At each predefined time interval, an aliquot of the conjugate solutions was collected and run through HPLC (mobile phase: acetonitrile/water = 50/50, v/v) to determine the amount of free PTXL, free GEM and the remaining PTXL-GEM conjugates.

### Preperation of drug loaded nanoparticles

Drug loaded nanoparticles were prepared via nanoprecipitation process. In a typical experiment, 0.12 mg of lecithin (Alfa® Aesar Co.) and 0.259 mg of 1,2-distearoyl-*sn-*glycero-3-phosphoethanolamine-N-[carboxy(polyethylene glycol)-2000] (DSPE-PEG-COOH, Avinti® Polar lipids Inc.) was dissolve in 4% ethanol and homogenised to combine the components and heated at 68 °C for three minutes. To the solution 1 mg of poly(lactic-co-glycolic acid) (PLGA, Mₙ = 40 kDa) and calculated amount of drug dissolved in acetonitrile was added dropwise while heating and stirring. After the addition of PLGA and drug solution, the vial was vortexes for three minutes followed by the addition of 1 mL of water. The solution mixture was stirred at room temperature for 2 hrs and washed with Amicon Ultra centrifugal filter (Millipore, Billerica, MA) with a molecular weight cutoff of 10 kDa and 1 mL of drug loaded nanoparticles were collected. Bare nanoparticles were prepared similarly in the absence of drugs. The nanoparticle size and surface ξ-potential were obtained from three repeat measurements using a dynamic light scattering (Malvern Zetasizer, ZEN 3600) with backscattering angle of 173°. The morphology and particle size were further characterized using scanning electron microscopy (SEM). Samples for SEM were prepared by dropping 5 µL of nanoparticle solutions onto a polished silicon wafer. After drying the droplet at room temperature overnight, the sample was coated with chromium and then imaged by SEM. Drug loading yield was determined by using HPLC.

### Cellular viability assay

Cytotoxicity of compound 2 and PTXL-GEM conjugates loaded nanoparticles was assessed against XPA3 human pancreatic carcinoma cell lines using the ATP assay. First, cells were seeded (2x10⁴) in 96-well plates and incubated for 24 hrs. Next, the medium was replaced with 150 µL of fresh medium and incubated with different concentration of compound 2 dissolved in DMSO. The final concentration of DMSO in each well was kept constant at 2%. The plates were then incubated for 72 hrs and measured by ATP reagents following a protocol provided by the manufacturer. Fresh cell media with 2% DMSO were used as negative controls. Similar procedures were applied to compare the cytotoxicity of 100 nM of compound 2 with that of a mixture of free paclitaxel and gemcitabine at the corresponding drug concentrations at various incubation times including 24 hrs, 48 hrs, and 72 hrs. Here the use of DMSO is only for solubilizing the free drugs. For the measurement of the cytotoxicity of PTXL-GEM conjugates loaded nanoparticles, the experiments were carried out without using DMSO.

### Results

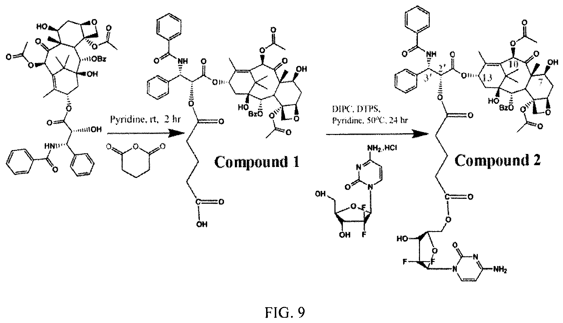

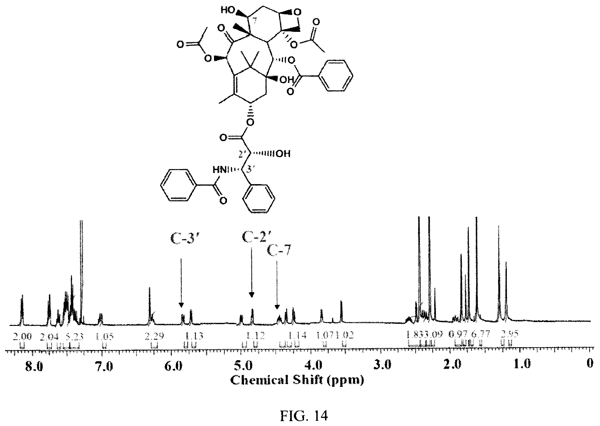

Figure 9 illustrates the synthesis scheme of PTXL-GEM conjugate (compound 2). We first took advantage of the steric hindrance structural chemistry of PTXL to selectively convert its 2' hydroxyl group (2'-OH) to a carboxyl moiety (compound 1). PTXL has three hydroxyl groups, of which two are secondary and one is tertiary. It has been reported that the tertiary hydroxyl group is highly hindered and unreactive. The secondary hydroxyl group at 7 position (7-OH) is less reactive than that at 2' position. Typically, one has to protect the 2'-OH in order to make any modification to the 7-OH group. Here we used glutaric anhydride (GA) to react with PTXL in the presence of catalytic amount of *N,N-*dimethylaminopyridine (DMAP) for 3 hrs at room temperature

to selectively modify the 2'-OH resulting in compound 1 as characterized in Figures 14-16. We observed that the reaction had to be limited for 3 hrs with a GA:PTXL molar ratio of 3:1 for 2'-OH reaction, otherwise (longer reaction time or higher GA:PTXL ratio) 7-OH reaction occurred. Compound 1 was then reacted with GEM using 1,3-diisopropyl carbodiimide (DIPC) and 4-(*N,N-*dimethylamino) pyridinium-4-toluenesulfonate (DPTS) resulting in the formation of compound 2. The formation of compound 2 was first confirmed by ¹H-NMR spectroscopy with all characteristic peaks and their integration values of PTXL and GEM, respectively, as indicated in Figure 10A. The 2'-OH reaction was confirmed by the integration value of 14H for the resonance peaks at δ 2.7-2.2 ppm. These peaks are corresponding to the methyl protons of acetate groups at C-4 and C-10, the methylene protons at C-14 position of the PTXL, and the methylene protons of GA linker. The resonance at δ 2.7-2.2 ppm of unmodified PTXL was integrated as 8H, which increased to 14H after the conjugation with GA because of the addition of 6H of the methylene group from GA moiety. In addition, the δ 4.4 ppm of the protons at C-7 position of PTXL remained intact during the conjugation. This further indicated the PTXL-GA reaction only occurred at the 2'-OH group as a downfield shifting of C-7 proton would have appeared if 7-OH reaction had happened. In contrast, a significant downfield shifting from δ 4.7 to δ 5.5 ppm was observed for the protons at the C-2' position. On the other hand, the use of GEM in its hydrochloride salt gives exclusive access to its hydroxyl group, which is thus prone to couple with the carboxyl group in the PTXL-GA to form an ester bond. In addition, it has been reported that DIPC and DTPS are effective esterification reagent with high reaction yield. Furthermore, the chemical shift associated with the -NH₂ protons of the pyrimidine ring at 9.0 ppm were intact after the reaction. This further confirms that the PTXL-GEM conjugation occurred via ester formation. The resulting compound 2 was further examined by high resolution mass spectrometry to determine its mass and molecular formula. As shown in Figure 10B, the results were precisely consistent with the expected formula of PTXL-GEM conjugates.

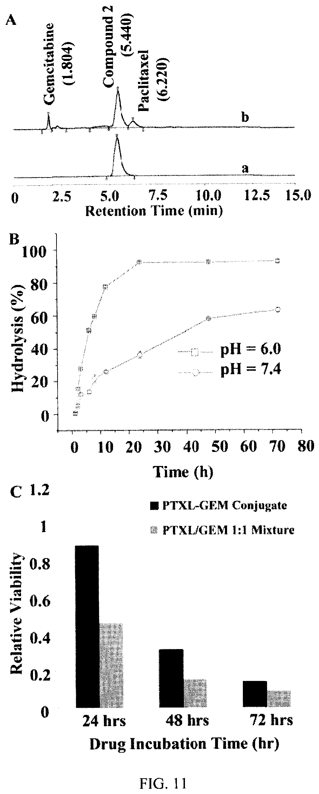

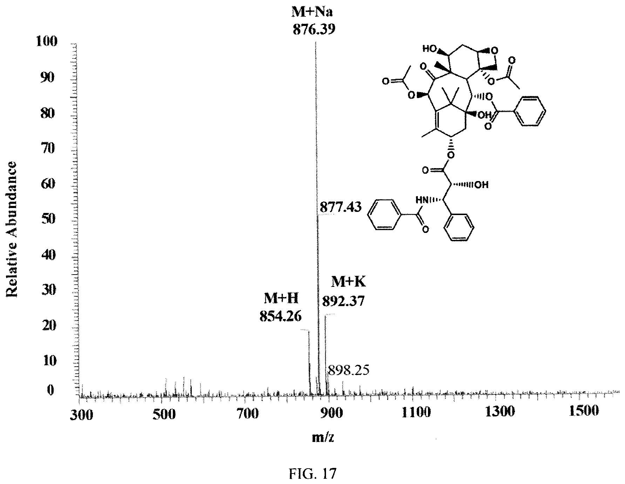

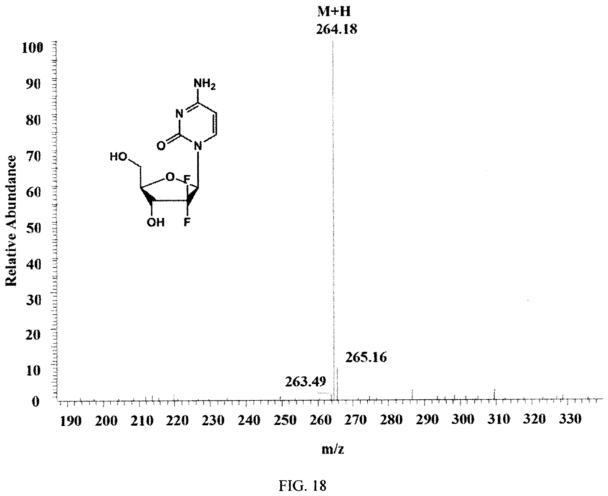

As the ultimate goal of this research is to concurrently deliver dual drugs to the same cancer cells for combinatorial therapy, it is crucial to ascertain that the linker bridging the two drugs can be effectively hydrolyzed, thereby releasing individual drugs to allow them to arrest cancer cells in their independent pathways. The hydrolysis of PTXL-GEM conjugates was evaluated and confirmed by high performance liquid chromatography (HPLC) and high resolution mass spectrometry. As shown in Figure 11A, the HPLC chromatogram clearly showed that after 24 hrs of incubation in water/acetonitrile (75/25, v/v) solution at pH = 7.4, a portion of the PTXL-GEM conjugates were hydrolyzed to free PTXL and free GEM with a characteristic HPLC retention time of 6.2 min and 1.8 min, respectively, which were confirmed by measuring the mass of the compounds collected at these two retention times (see Figure 17 and 18 for the corresponding mass spectra). The formation of free PTXL and free GEM upon hydrolysis further evidenced that the PTXL-GEM conjugation occurred via the coupling of hydroxyl and carboxyl group to form an ester bond. If the reaction had occurred via amide formation between the -NH₂ of the pyrimidine ring and the carboxyl group, free PTXL and free GEM would not have been released upon hydrolysis within only 24 hrs. We hypothesize that when these PTXL-GEM conjugates are delivered to target cells by a drug carrier through endocytosis, the hydrolysable PTXL-GEM conjugates can be hydrolyzed with a faster rate at the mild acidic endosomal environment (pH = ~6). To test this hypothesis, we measured the hydrolysis kinetics of the PTXL-GEM conjugates at pH = 6.0 and 7.4 respectively. As shown in Figure 11B, the hydrolysis rate was significantly faster at acidic environments (pH = 6.0) than at neutral solution (pH = 7.4). Near 80% of the drug conjugates were hydrolyzed to free PTXL and free GEM at pH = 6.0 within the first 10 hrs, while less than 25% were cleaved at pH = 7.4.

Next we examined the *in vitro* cellular cytotoxicity of free PTXL-GEM conjugates. As both PTXL and GEM are potent chemotherapy drugs against pancreatic cancer, we chose human pancreatic cancer cell line XPA3 for this study. Since it has been documented that the 2'-OH group is essential for high cytotoxicity of PTXL, it is natural to expect that the cytotoxicity profile of PTXL-GEM conjugates will rely on their hydrolysis process. To test this, we evaluated the cytotoxicity of the drug conjugates (100 nM concentration) at different hydrolysis duration, using a mixture of 100 nM free PTXL and 100 nM free GEM as a positive control. As shown in Figure 11C, large cytotoxicity difference was observed between the drug conjugates and the free drug mixtures after 24 hrs and 48 hrs incubation, during which the drug conjugates were partially hydrolyzed. For example, the drug conjugates killed ∼15% of XPA3 cells whereas the drug mixtures killed ∼55% of the cells after 24 hrs of incubation. However, after 72 hrs of incubation, the cytotoxicity of the PTXL-GEM conjugates was nearly at the same level as the free PTXL and free GEM mixtures; over 80% of the cells were killed for both systems. This time-dependent cytotoxicity is consistent with the temporal hydrolysis profile of the PTXL-GEM conjugates at pH = 7.4 measured by HPLC as shown in Figure 11B. It is worth noting that small molecule drugs such as PTXL, GEM and PTXL-GEM conjugate usually can diffuse across the cell membranes to the inside of the cells without going through the endocytosis mechanism. Therefore, the hydrolysis process of PTXL-GEM conjugates follows the pH = 7.4 profile when the drug conjugates are administered directly without using a drug delivery vehicle.

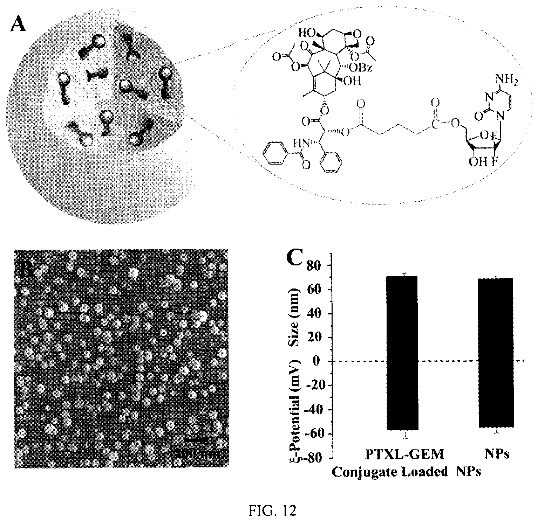

After having demonstrated the formation of PTXL-GEM drug conjugates, their spontaneous hydrolysis to individual drugs, and cytotoxicity against human pancreatic cancer cell line XPA3, we next loaded the PTXL-GEM conjugates into a recently developed lipid-coated polymeric nanoparticle to validate the feasibility of using this pre-conjugation approach to enable nanoparticle dual drug delivery. The PTXL-GEM conjugates were mixed with poly(lactic-co-glycolic acid) (PLGA) in an acetonitrile solution, which was subsequently added into an aqueous solution containing lipid and lipid-polyethylene glycol conjugates to prepare lipid-coated PLGA nanoparticles following a previously published protocol. L. Zhang, et al. *ACS Nano* 2008, 2, 1696. Figure 12A shows a schematic representation of PTXL-GEM conjugates loaded nanoparticles, which are spherical particles as imaged by scanning electron microscopy (SEM) (Figure 12B). Dynamic light scattering measurements showed that the resulting PTXL-GEM conjugates loaded nanoparticles had an unimodel size distribution with an average hydrodynamic diameter of 70 ± 1.5 nm (Figure 12C), which was consistent with the findings from the SEM image (Figure 11B). The surface zeta potential of the drug loaded nanoparticles in water was about -53 ± 2 mV (Figure 12C). We further found that the size and surface zeta potential of the PTXL-GEM conjugates loaded nanoparticles were similar to those of the corresponding empty nanoparticles, 70 ± 1 nm and -51 ± 2 mV, respectively. This suggests that the encapsulation of PTXL-GEM conjugates has negligible effect on the formation of the lipid-coated polymeric nanoparticles.

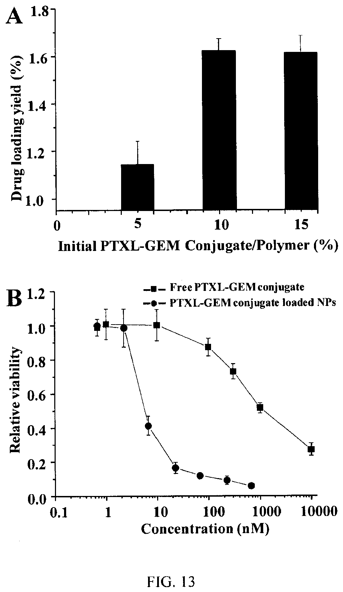

The encapsulation yield and loading yield of PTXL-GEM conjugates in the nanoparticles were quantified by HPLC after dissolving the particles in organic solvents to free all encapsulated drugs. When the initial PTXL-GEM conjugate input was 5 wt%, 10 wt%, and 15 wt% of the total nanoparticle weight, the drug encapsulation yield was 22.8 ± 2.0%, 16.2 ± 0.5%, 10.8 ± 0.7% respectively, which can be converted to the corresponding final drug loading yield of 1.1 wt%, 1.6 wt%, and 1.6 wt%, respectively (Figure 13A). Here the drug encapsulation yield is defined as the weight ratio of the encapsulated drugs to the initial drug input. The drug loading yield is defined as the weight ratio of the encapsulated drugs to the entire drug-loaded nanoparticles including both excipients and bioactive drugs. It seemed the maximum PTXL-GEM loading yield was about 1.6 wt% for the lipid-coated polymeric nanoparticles. This 1.6 wt% drug loading yield can be converted to roughly 1700 PTXL-GEM drug conjugate molecules per nanoparticle, calculating from the diameter of the nanoparticle (70 nm), PLGA density (1.2 g/mL) and the molecular weight of PTXL-GEM conjugate (1212 Da).

The cytotoxicity of PTXL-GEM conjugates loaded nanoparticles against XPA3 cell lines was then examined in comparison with free PTXL-GEM conjugates. Figure 13B summarized the results of IC₅₀ measurements of PTXL-GEM conjugates loaded nanoparticles and free PTXL-GEM conjugates for 24 hrs incubation with the cancer cells. It was found that the IC50 value of PTXL-GEM conjugates was decreased by a factor of 200 for XPA3 cells after loading the drug conjugates into the lipid-coated polymeric nanoparticles. This enhanced cytotoxicity of PTXL-GEM conjugates upon nanoparticle encapsulation can be explained, at least partially, by the fact that nanoparticle drug delivery can suppress cancer drug resistance. Small molecule chemotherapy drugs that enter cells through either passive diffusion or membrane translocators are rapidly vacuumed out of the cells before they can take an effect by transmembrane drug efflux pumps such as P-glycoprotein (P-gp). Drug loaded nanoparticles, however, can partially bypass the efflux pumps as they are internalized through endocytosis. Once being engulfed by the plasma membrane, nanoparticles are transported by endosomal vesicles before unloading their drug payloads. Thus drug molecules are released farther away from the membrane-bound drug efflux pumps and therefore are more likely to reach and interact with their targets. The endocytic uptake mechanism is particularly favourable to the combinatorial drug delivery system present in this study. The pH drop upon the endosomal maturation into lysosomes will subject the drug conjugates to more acidic environment and more hydrolase enzymes, which will facilitate the cleavage of the hydrolysable linkers. Moreover, the degradation of PLGA polymer will also contribute to lowering the pH value surrounding the nanoparticles which can accelerate the hydrolysis process of the drug conjugates as well. The enhanced hydrolysis of the conjugate linkers may also partially answer for the near 200-fold cytotoxicity increase of PTXL-GEM conjugates after being encapsulated into the nanoparticles.

While the focus of this article is to report a novel chemical approach to loading dual chemotherapy drugs into a single nanoparticle for combinatorial drug delivery, it would be interesting to compare the cytotoxicity of PTXL-GEM conjugates loaded nanoparticles with that of a cocktail mixture of the same type of nanoparticles containing either free PTXL or free GEM. However, the vast hydrophobicity (or solubility) difference between PTXL and GEM makes it practically undoable to load them into the same type of nanoparticles, such as the lipid-coated polymeric nanoparticles used in this study. These nanoparticles can encapsulate hydrophobic drugs such as PTXL with high encapsulation and loading yields but can barely encapsulate hydrophilic drugs such as GEM. In fact, the inability of loading different drugs to the same type of nanoparticles represents a generic challenge to many pairs of drugs for combination therapy. The work presented in this paper may offer a new way to overcome this challenge.

### Conclusions

In conclusion, we have demonstrated the conjugation of PTXL and GEM with a stoichiometric ratio of 1:1 via a hydrolysable ester linker and subsequently loaded the drug conjugates into lipid-coated polymeric nanoparticles. The cytotoxicity of the resulting combinatorial drug conjugates against human cancer cells was comparable to the corresponding free PTXL and GEM drug mixtures after the conjugates were hydrolyzed. The cytotoxicity of the drug conjugates was significantly improved after being encapsulated into drug delivery nanoparticles. This work provides a new method to load dual drugs to the same drug delivery vehicle in a precisely controllable manner, which holds great promise to suppress cancer drug resistance. Similar strategy may be generalized to other drug combinations. Synthesizing combinatorial drug conjugates with a broad range of stoichiometric ratios is described above.

### Synthesis of Ptxl-Pt(IV) drug conjugates loaded nanoparticles

Paclitaxel and cisplatin were purchased from ChemiTek Industries Co. (SX, China) and Sigma-Aldrich Company (St. Louis, MO, USA), respectively, and used without further purification. All other materials including solvents were purchased from Sigma-Aldrich Company, USA. Single addition luminescence ATP detection assay was purchased from PerkinElmer Inc. for cytotoxicity measurement. ¹H NMR spectra were recorded in CDCl₃ using a Varian Mercury 500 MHz spectrometer. Electrospray ionization mass spectrometry (ESI-MS, Thermo LCQdeca mass spectrometer) and Thermo Fisher Scientific LTQ-XL Orbitrap mass spectrometer were used to determine the mass and molecular formula of the compounds. Reversed phase high performance liquid chromatography (HPLC) purification was performed on an Varian HPLC system equipped with µ-bonapack C18 column (4.6 mm×150 mm, Waters Associates, Inc.) using acetonitrile and water (50/50, v/v) as mobile phase.

### Synthesis of cis,trans,cis-PtCl₂(OCOCH₂CH₂CH₂COOH)₂(NH₃)₂ prodrug.

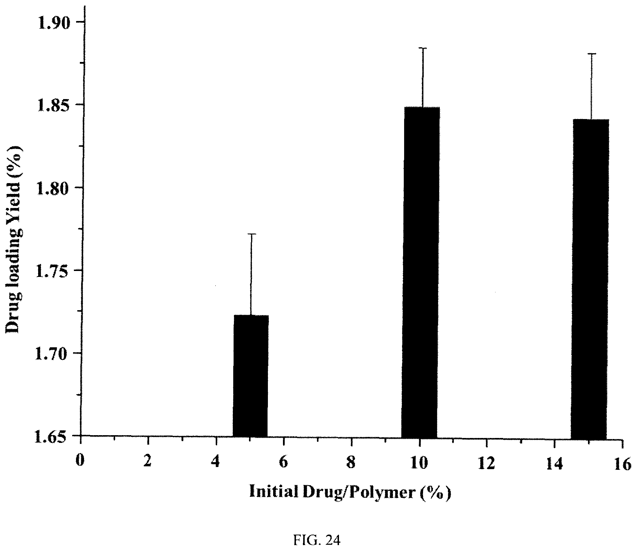

*cis,trans,cis*-PtCl₂(OH)₂(NH₃)₂ was first synthesized following a previously published protocol, (R. Kuroda, et al. X-ray and NMR studies of trans-dihydroxo-platinum(IV) antitumor complexes, J Inorg Biochem 22 (1984) 103-17; M.D. Hall, et al. The cellular distribution and oxidation state of platinum(II) and platinum(IV) antitumour complexes in cancer cells, J Biol Inorg Chem 8 (2003) 726-32) which was then used to prepare *cis,trans,cis-*PtCl₂(OCOCH₂CH₂CH₂ COOH)₂(NH₃)₂. Briefly, an excess of glutaric anhydride was added to an methylene chloride (MC) solution containing 100 mg (0.3 mmol) of PtCl₂(OH)₂(NH₃)₂ under reflux condition in the presence of catalytic amount of triethylamine (TEA). After 12 h of reaction, cold water was added to hydrolyze excess glutaric anhydride. The reaction mixture was kept at 2°C for 16 hrs. The MC was then removed from the reaction mixture under reduced pressure resulting in a white residue. The residue was purified by washing with water, ethanol, and ether in that order. The final production yield was about 45%. The mass and molecular formula of *cis,trans,cis-*PtCl₂(OCOCH₂CH₂CH₂COOH)₂(NH₃)₂ were then determined by HR-ESI-FT-MS (orbit-trap-MS, negative) m/z 560.97 [M-H]⁻, 596.83 [M+Cl]⁺. Calcd for C₁₀H₂₀Cl₂N₂O₈Pt : 561.02. Found: 561.97 (see Figure 24).

### Synthesis of Ptxl-Pt(IV) conjugate.

*cis,trans,cis*-PtCl₂(OH)₂(NH₃)₂ (10 µmol) and Ptxl (6 µmol) were dissolved in 200 µL dry MC. *N, N*-dimethylaminopyridine (DMAP, 0.57 µmol) and *N, N-*dicyclohexylcarbodiimide (DCC, 50 µmol) dissolved in 100 µL of dry MC were then added to this solution. The mixture solution was stirred at room temperature for 24 h. The reaction was monitored by HPLC using 50/50 (v/v) acetonitrile/water as an eluent (product retention time = 4.5 min). The complete disappearance of the starting paclitaxel (retention time = 5.5 min) occurred after 24 h of reaction. Solvent was concentrated and the byproduct dicyclohexylurea (DCU) was removed by filtration. The remaining solvent was completely removed and the residue was suspended in ethyl acetate and kept at 4 °C, during the process additional DCU precipitates out to form crystals which were further removed by filtration. The washing process was repeated three times to completely remove DCU. Finally, Ptxl-Pt(IV) conjugate was precipitated in hexane to obtain yellowish white powder. The final product was purified by HPLC with a recovery yield of 55%. ¹H NMR (CDCl₃, *δ* ppm) was carried out to characterize the produced Ptxl-Pt(IV) conjugate: 1.14 (s, 3H), 1.25 (s, 3H), 1.69 (s, 3H), 1.7-2.06 (broad, 9H), 2.16-2.27 (br, 4H), 2.3-2.7 (br, 9H), 2.9 (d, 1H), 3.2-3.6 (br,14H), 4.32 (d, 1H), 4.48 (t, 1H), 5.0 (d, 1H), 5.5 (d, 1H), 5.69 (d, 1H), 6.2-6.3 (br, 2H), 7.09 (d, 1H), 7.3-7.5(m, 10H), 8.13 (d, 2H), 8.6 (-NH), 11.0 (-COOH). The mass and molecular formula of Ptxl-Pt(IV) conjugate were determined by HR-ESI-FT-MS (orbit-trap-MS, negative) m/z 1395.32 [M-H]⁻, Calcd for C₅₇H₆₉Cl₂N₃O₂₁Pt : 1396.34. Found: 1396.32.

### Preparation and characterization of Ptxl-Pt(IV) drug conjugates loaded nanoparticles.

Ptxl-Pt(IV) conjugates were loaded into lipid-coated polymeric nanoparticles through a nanoprecipitation process. Typically, 0.12 mg of lecithin (Alfa® Aesar Co.) and 0.259 mg of 1,2-distearoyl-*sn*-glycero-3-phosphoethanolamine-N-[carboxy(polyethylene glycol)-2000] (DSPE-PEG-COOH, Avinti® Polar lipids Inc.) were dissolved in 4% ethanol aqueous solution and heated at 68 °C for three minutes. Then 1 mg of poly(lactic-co-glycolic acid) (PLGA, *Mₙ*=40 kDa) and calculated amount of Ptxl-Pt(IV) conjugates dissolved in acetonitrile were added drop-wise into the lipid solution under heating and stirring. After the addition of PLGA and Ptxl-Pt(IV) conjugate solution, the mixture was vortexed for 3 min followed by the addition of 1 mL of water. The resulting solution was stirred at room temperature for 2 h and washed with Amicon Ultra centrifugal filter (Millipore, Billerica, MA) with a molecular weight cutoff of 10 kDa. Finally, 1 mL of Ptxl-Pt(IV) conjugates loaded nanoparticles were collected. The nanoparticle size was obtained from three repeat measurements using a dynamic light scattering (Malvern Zetasizer, ZEN 3600) with backscattering angle of 173°. The morphology and particle size were further characterized using scanning electron microscopy (SEM). Samples for SEM were prepared by dropping 5 µL of nanoparticle solutions onto a polished silicon wafer. After drying the droplet at room temperature overnight, the sample was coated with chromium and then imaged by SEM. Drug loading yield of the nanoparticles was determined by using HPLC.

### Cellular viability assay.

Cytotoxicity of free Ptxl-Pt(IV) conjugates and Ptxl-Pt(IV) conjugates loaded nanoparticles were assessed against A2780 ovarian carcinoma cell lines using the ATP assay. First, cells were seeded to 10% confluency (∼5x10³/well) in 96-well plates and incubated for 24 h. Prior to the experiment, the culture medium was replaced with 150 µL fresh medium and cells were incubated with different concentration of free Ptxl-Pt(IV) conjugates and Ptxl-Pt(IV) conjugates loaded nanoparticles for 24 h, followed by washing the cells with PBS to remove excess drugs or nanoparticles. The cells were then incubated in fresh medium for 72 h and measured by ATP assay following a protocol provided by the manufacturer. Fresh culture medium was used as a negative control in this study.

### Results

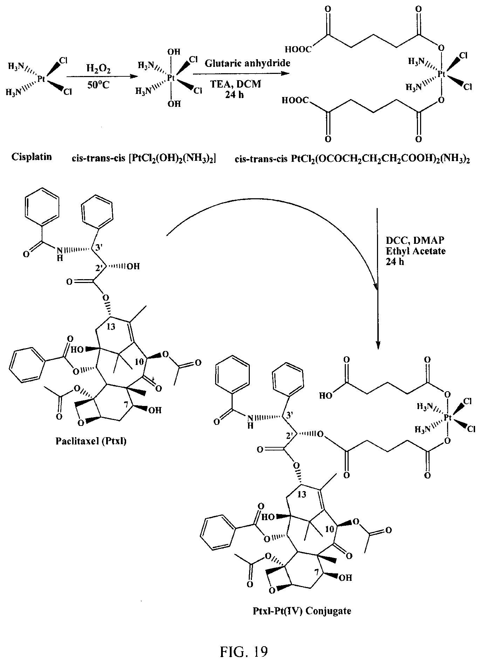

Figure 19 illustrates the synthesis scheme of Ptxl-Pt(IV) conjugate. We started the synthesis with the oxidation of cisplatin to form dihydroxy cisplatin, a Pt(IV) prodrug, which was later conjugated to Ptxl via a glutaric acid linker. In order to conjugate dihydroxy cisplatin with Ptxl, one can choose to first activate dihydroxy cisplatin with glutaric anhydride, followed by conjugating the resulting organo platinum complex to Ptxl. Alternatively, the conjugation can be carried out in a reverse order, where glutaric anhydride is reacted with Ptxl first and then conjugated to dihydroxy cisplatin. The difference between these two synthetic routes is that the former involves the conjugation of an organic compound with an organo platinum complex, while the latter involves a reaction between an organic compound with a dihydroxy platinum complex. Given the high flexibility to select proper reaction solvent for an organo platinum complex and Ptxl as compared to a dihydroxy platinum complex and Ptxl, we chose the first route to synthesize Ptxl-Pt(IV) hydrophobic-hydrophilic drug conjugates as shown in Figure 19.

As discussed in previous paragraph we converted Pt(IV) complex to Carboxyl functionalized organo Pt complex by reacting with GA (Supporting information Figure 24). Taking an advantage of the steric hindrance structural chemistry of Ptxl, we selectively reacted its 2' hydroxyl group (2'-OH) to a carboxyl moiety of Pt(IV) organo Pt complex. Among three -OH groups in Ptxl, it has been reported that the tertiary hydroxyl group is highly hindered and unreactive. The secondary hydroxyl group at 7 position (7-OH) is less reactive than that at 2' position. Typically, one has to protect the 2'-OH in order to make any modification to the 7-OH group.

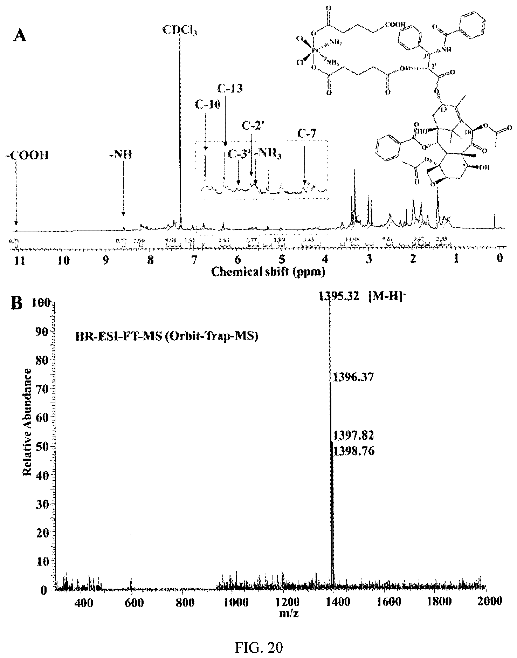

The formation of Ptxl-Pt(IV) hydrophobic and hydrophilic conjugate was first confirmed by ¹H-NMR spectroscopy with all characteristic peaks and their integration values of Ptxl and Pt(IV), respectively, as indicated in Figure 20A. The reaction at 2'-OH was confirmed due to the significant downfield shifting of the protons at the C-2' from δ 4.7 to δ 5.7 ppm. This shifting further confirms esterification between Ptxl and GA functionalized Pt(IV) thereby confirming the conjugation of Ptxl and Pt(IV) with hydrolysable linker. However, the protons at C-7 position of Ptxl remained intact at δ 4.4 ppm during the conjugation, this further indicated the Ptxl-Pt(IV) reaction only occurred at the 2'-OH group as a downfield shifting of C-7 proton would have appeared if 7-OH reaction had happened. The resulting compound 2 was further examined by high resolution mass spectroscopy to determine its mass and molecular formula. As shown in Figure 20B, the results were consistent with the expected formula of Ptxl-Pt(IV) conjugate. However, one might expect two molecules of Ptxl to attach to GA functionalized Pt(IV) due to presence of two -COOH group. Such conjugation was not observed likely because one of the GA moiety at the axial position became sterically hindered after one Ptxl was attached. The 1:1 conjugation was further confirmed by the corresponding molecular formula for Ptxl-Pt(IV) and the appearance of -COOH proton resonance at δ 11.0 ppm.

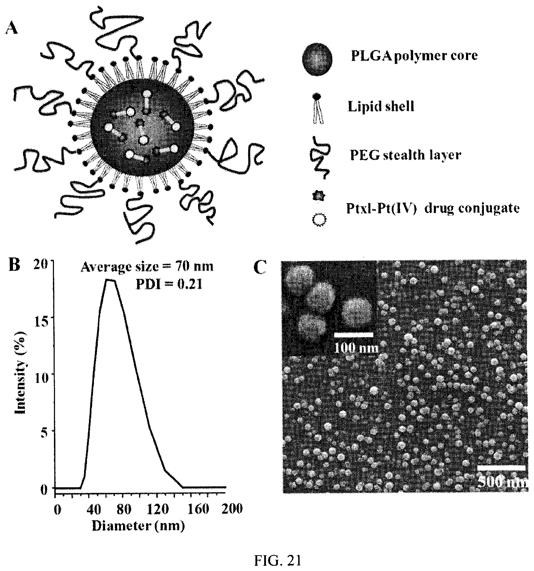

Upon completion of the conjugate synthesis and characterization, the Ptxl-Pt(IV) compound was subsequently loaded into a recently developed lipid-coated polymeric nanoparticleas demonstrated in Figure 21A to confirm whether co-encapsulation of hydrophobic and hydrophilic drugs can be accomplished using this pre-conjugation approach. Based on a previously published protocol (L. Zhang, et al. Self-assembled lipid--polymer hybrid nanoparticles: a robust drug delivery platform, ACS Nano 2 (2008) 1696-702), the Ptxl-Pt(IV) conjugates were mixed with poly(lactic-co-glycolic acid) (PLGA, Mn=40,000) in an acetonitrile solution, which was then added drop-wise in aqueous solution containing lipid and lipid-polyethylene glycol conjugates to prepare lipid-coated PLGA nanoparticles (Figure 21A). To quantify the loading yield of Ptxl-Pt(IV) conjugates, the nanoparticles were dissolved in organic solvents to free all encapsulated drugs. The solution was then analyzed by high performance liquid chromatography (HPLC). An initial Ptxl-Pt(IV) conjugate input of 10 wt% of the total polymeric nanoparticle weight yielded a final loading of 1.86% (wt/wt), or 18.6µg per 1mg of polymer (Figure 24), which is comparable with published data on nanoparticle drug loading (J.M. Chan, et al. PLGA-lecithin-PEG core-shell nanoparticles for controlled drug delivery, Biomaterials 30 (2009) 1627-34). Figure 21A shows a schematic representation of Ptxl-Pt(IV) conjugate loaded nanoparticles, which are spherical particles with unimodal size distribution with an average hydrodynamic diameter of 70 nm and a PDI of 0.21 as shown by dynamic light scattering (DLS) measurements (Figure 21B). SEM images further showed that the resulting Ptxl-Pt(IV) conjugates loaded nanoparticles had an unimodal size distribution with an average diameter of 70 nm (Figure 21C), which was consistent with the findings from DLS (Figure 21B).

After having demonstrated the loading of Ptxl-Pt(IV) conjugate, we next evaluated the in-vitro cellular cytotoxicity of Ptxl-Pt(IV) against A2780 human ovarian cancer cells as shown in Figure 22. The cells were incubated with free Pxtl-Pt(IV) conjugates and Pxtl-Pt(IV) conjugates in nanoparticles at different concentrations for 4hrs followed by PBS washing and incubation in fresh media for 72hrs before ATP cell viability assay (Figure 22A). It was observed that the Ptxl-Pt(IV) showed less toxicity as compared to that of Ptxl-Pt(IV) loaded nanoparticles. This reduced toxicity could be attributed to several factors. Firstly, the conjugation of a hydrophobic Ptxl and a hydrophilic Cisplatin gives rise to a large amphiphilic molecule that is structurally similar to phospholipids. The amphiphilic conjugate is more likely to be anchored in the lipid bilayer, resulting in less efficient drug delivery. Secondly, the cytoplasmic pH of cancer cells, which is approximately 6.8 to 7.1, cannot efficiently break the ester bond that connects the two drug molecules. In the conjugate form Ptxl and Pt(IV) cannot freely interact with their molecular targets. Therefore a slow hydrolysis rate will significantly compromise the conjugate's potency.

Cytotoxicity of the Pxtl-Pt(IV) conjugate-loaded nanoparticles provides evidence that both membrane diffusion and conjugate hydrolysis issues can be overcome by nanoparticle delivery. As shown in Figure 22A, large toxicity difference was observed between the free Ptxl-Pt(IV) and Ptxl-Pt(IV) loaded NPs system. Such difference can be easily observed from the microscopic images of the cells after the treatment with free Ptxl-Pt(IV) and Ptxl-Pt(IV) loaded NPs as shown in Figure 22B and C, respectively. The number of viable cells were significantly reduced after the treatment with Ptxl-Pt(IV) loaded NPs, Figure 2C. It has been well studied that nanoparticles below 100nm in size are taken up by cells through endocytic uptake. Upon contact with the nanoparticles the cell membranes fold inward and engulf the particles in endocytic vesicles. This process allows the drug conjugates to efficiently enter the cytoplasm without relying on passive diffusion through the lipid bilayers, which is highly unfavorable to large amphiphilic molecules. Another benefit of the endocytic uptake mechanism is that the endo-lysomal environments provides a more acidic medium which can accelerate the hydrolysis of the ester linker in the Pxtl-Cisplatin conjugate. As endosomes matures into lysosomes, their pH can drop to ∼5.5. The excess protons speed up the drug release that unblocks the functional 2'-OH of the Ptxl and relieves the Pt(IV) which reduced to Cisplatin in intracellular environment. In addition, the degradation of the PLGA polymers into lactic acid will further lower the pH value surrounding the nanoparticles, resulting in even faster drug release. The enhanced toxicity in the nanoparticle formulation of the Pxtl-Pt(IV) has significant implications as it addresses common issues in drug conjugates. Additionally, the strategy adds applicability to the fast-growing nanoparticle platforms and could potentially address the side effects associated with premature drug release in the circulation as the drug conjugates are much less potent without the vehicle.

### Conclusions

In conclusion, we have demonstrated the conjugation of hydrophobic Ptxl and hydrophilic cisplatin with a hydrolysable ester linker and subsequently encapsulated the compound into a lipid-coated polymeric nanoparticle. The cytotoxicity of the resulting Ptxl-Pt(IV) conjugates against ovarian cancer cells was compared to the corresponding free Ptxl and cisplatin drug mixtures after the conjugates were hydrolyzed. The efficacy of Ptxl-Pt(IV) was significantly improved after being encapsulated into drug delivery nanoparticles. This work provides a new approach to load hydrophobic and hydrophilic drug to the same drug delivery vehicle without adding complexity to the nanoparticle structure. We demonstrate that prodrug conjugates and nanoparticulate systems can complement each other as an excellent combinatorial drug delivery platform.

**TABLE 2 - Exemplary Cancers and Tumors**

| | |
|---|---|
| | adenocarcinoma, pancreatic |
| | adenocarcinoma, papillary, bladder |
| ackerman tumor | adenocarcinoma, pleomorphic |
| adenocarcinoid, malignant, appendiceal | adenocarcinoma, polymorphous low-grade |
| adenocarcinoma variant, gastric cancer | adenocarcinoma, proximal jejunum |
| adenocarcinoma, alpha-fetoprotein-producing, esophageal | adenocarcinoma, rete testis |
| adenocarcinoma, apocrine | adenocarcinoma, small bowel |
| adenocarcinoma, appendiceal | adenocarcinoma, thymus |
| adenocarcinoma, bartholin gland | adenocarcinoma, unknown primary site |
| adenocarcinoma, bladder | adenocarcinoma, urachal |
| adenocarcinoma, clear cell | adenocarcinoma, urethral |
| adenocarcinoma, colloid | adenocarcinoma, vaginal |
| adenocarcinoma, ductal type | adenomyoepithelioma, malignant, breast |
| adenocarcinoma, eccrine | adenosarcoma, Müllerian |
| adenocarcinoma, endometrioid primary, in colorectal endometriosis | adrenogenital syndrome /testicular tumor |
| | ameloblastoma, desmoplastic |
| adenocarcinoma, esophagus | ameloblastoma, malignant |
| adenocarcinoma, fallopian tube | amyloid |
| adenocarcinoma, fetal pulmonary | angioblastoma, giant cell |
| adenocarcinoma, gall bladder | angioendothelioma, malignant, endovascular papillary |
| adenocarcinoma, hepatoid | angioendotheliomatosis, malignant |
| adenocarcinoma, in situ, cervix | angiomyxoma, malignant, aggressive, scrotum |
| adenocarcinoma, intra-extrahepatic, bile ducts | angiomyxoma, malignant, aggressive, scrotum |
| adenocarcinoma, lacrimal gland | angiosarcoma |
| adenocarcinoma, large bowel | angiosarcoma, cardiac |
| adenocarcinoma, low-grade, extraosseous endolymphatic sac | angiosarcoma, pulmonary artery |
| adenocarcinoma, mucinous | angiosarcoma, Wilson-Jones |
| adenocarcinoma, mucinous, prostate | askin tumor |
| adenocarcinoma, mucinous, stomach | astroblastoma |
| adenocarcinoma, oncocytic | astrocytic neoplasm |
| astrocytoma, anaplastic | carcinoma, adenosquamous |
| astrocytoma, gemistocytic | carcinoma, adenosquamous, liver |
| astrocytoma, pilocytic | carcinoma, adenosquamous, pancreatic |
| astrocytoma, thalamic glioma | carcinoma, adrenocortical |
| blastoma, pleuropulmonary (PPB) | carcinoma, ameloblastic |
| blastoma, pulmonary | carcinoma, anal |
| borderline tumor, malignant, ovary | carcinoma, anaplastic |
| Buschke-Lowenstein tumor giant condyloma | carcinoma, anaplastic, thymic |
| calcifying epithelial odontogenic tumor (CEOT) | carcinoma, anaplastic, thyroid |
| carcinamitosis, peritoneal | carcinoma, apocrine |
| carcinoid, malignant | carcinoma, basal cell, perianal |
| carcinoid, malignant, atypical | carcinoma, basal cell, vulva |
| carcinoid, malignant, bronchopulmonary, atypical | carcinoma, basaloid squamous cell, esophageal |
| carcinoid, malignant, bronchopulmonary, typical | carcinoma, basaloid squamous cell, NOS |
| carcinoid, malignant, colorectal | carcinoma, basaloid, lung |
| carcinoid, malignant, gastric | carcinoma, bile duct |
| carcinoid, malignant, gastrointestinal, appendix | carcinoma, biliary tract |
| carcinoid, malignant, goblet cell | carcinoma, bronchioalveolar (BAC) |
| carcinoid, malignant, lung | carcinoma, bronchogenic small cell undifferentiated |
| carcinoid, malignant, pulmonary | carcinoma, choroid plexus |
| carcinoid, malignant, rectal | carcinoma, ciliated cell |
| carcinoid, malignant, renal | carcinoma, clear cell, bladder |
| carcinoid, malignant, small bowel | carcinoma, clear cell, eccrine |
| carcinoid, malignant, thymic | carcinoma, clear cell, odontogenic |
| carcinoma, acinar cell (ACC) | carcinoma, clear cell, thymic |
| carcinoma, acinic cell | carcinoma, collecting duct (CDC) |
| carcinoma, adenoid basal, uterine cervix | carcinoma, collecting duct, kidney |
| carcinoma, adenoid cystic (AdCC) | carcinoma, cribriform |
| carcinoma, adenoid cystic, breast (ACCB) | carcinoma, cribriform, breast |
| carcinoma, adenoid cystic, breast, metastatic (ACC-M) | carcinoma, cystic |
| carcinoma, duodenal | carcinoma, papillary, breast |
| carcinoma, epithelial-myoepithelial (EMC) | carcinoma, parathyroid |
| carcinoma, gall bladder | carcinoma, parietal cell |
| carcinoma, giant cell | carcinoma, penile |
| carcinoma, hepatocellular | carcinoma, pilomatrix |
| carcinoma, Hurthle cell | carcinoma, pituitary |
| carcinoma, Hurthle cell, thyroid | carcinoma, plasmacytoid urothelial, bladder |
| carcinoma, insular | carcinoma, poorly differentiated, neuroendocrine (PDNEC) |
| carcinoma, insular, thyroid | carcinoma, primary intraosseous |
| carcinoma, islet cell | carcinoma, primary peritoneal, extra-ovarian (EOPPC) |
| carcinoma, large cell, neuroendocrine (LCNEC) | carcinoma, renal cell (RCC), poorly differentiated |
| carcinoma, lymphoepithelioma-like, thymic | carcinoma, renal cell (RCC), chromophobic (ChC) |
| carcinoma, male breast | carcinoma, renal cell (RCC), clear cell (CCC) |
| carcinoma, medullary thyroid | carcinoma, renal cell (RCC), collecting duct (CDC) |
| carcinoma, meibomian | carcinoma, renal cell (RCC), papillary (PC) |
| carcinoma, merkel cell (MCC) | carcinoma, renal cell (RCC), sarcomatoid |
| carcinoma, metaplastic, breast | carcinoma, sarcomatoid, colon |
| carcinoma, microcystic adnexal | carcinoma, sarcomatoid, thymic |
| carcinoma, mixed acinar, endocrine | carcinoma, sebaceous |
| carcinoma, moderately differentiated, neuroendocrine | carcinoma, serous ovarian, papillary(PsOC) |
| carcinoma, mucinous, bronchioloalveolar, lung | carcinoma, signet-ring cell |
| carcinoma, mucinous, eccrine | carcinoma, small cell |
| carcinoma, mucoepidermoid | carcinoma, small cell undifferentiated, prostate |
| carcinoma, mucoepidermoid, bronchus | carcinoma, small cell undifferentiated, prostrate (SCUUP) |
| carcinoma, nasopharyngeal/caucasians (NPC) | carcinoma, small cell, anorectal neuroendocrine |
| carcinoma, neuroendocrine | carcinoma, small cell, colorectal |
| carcinoma, neuroendocrine, lung | carcinoma, small cell, esophageal |
| carcinoma, non-small cell w/neuroendocrine features, lung | carcinoma, small cell, extrapulmonary |
| carcinoma, odontogenic | carcinoma, small cell, gastrointestinal tract |
| carcinoma, papillary | carcinoma, small cell, neuroendocrine (oat cell) (SCNC) |
| carcinoma, small cell, pancreatic | carcinoma, tubal |
| carcinoma, small cell, renal | carcinoma, tubular, breast |
| carcinoma, small cell, stomach | carcinoma, undifferentiated, nasopharyngeal type (UCNT) |
| carcinoma, small cell, thymic | carcinoma, undifferentiated, primary sinonasal nasopharyngea |
| carcinoma, small intestine | carcinoma, undifferentiated, sinonasal (SNUC) |
| carcinoma, squamous cell, adnexal ductal cyst | carcinoma, undifferentiated, thymic |
| carcinoma, squamous cell, atypical | carcinoma, undifferentiated, w/lymphoid stroma |
| carcinoma, squamous cell, breast | carcinoma, vaginal |
| carcinoma, squamous cell, diffuse pagetoid, esophagus | carcinoma, verrucous |
| carcinoma, squamous cell, esophageal | carcinoma, w/ spindle cell metaplasia, breast |
| carcinoma, squamous cell, keratinizing, thymic (KTSC) | carcinoma, w/metaplasia, osteo-chondroid variant, breast |
| carcinoma, squamous cell, laryngeal | carcinoma, w/sarcomatous metaplasia, breast |
| carcinoma, squamous cell, lymphoepithelioma-like | carcinoma, well differentiated, neuroendocrine (WDNEC) |
| carcinoma, squamous cell, nasopharynx | carcinoma, well differentiated, thymic (WDTC) |
| carcinoma, squamous cell, nonkeratinizing | carcinosarcoma |
| carcinoma, squamous cell, oral cavity | carcinosarcoma, uterine |
| carcinoma, squamous cell, ovarian | cartilage tumor |
| carcinoma, squamous cell, stomach | cartilaginous tumor, larynx |
| carcinoma, squamous cell, subungual (SCC) | chemodectoma, malignant |
| carcinoma, squamous cell, thymic | chloroma |
| carcinoma, squamous cell, thyroglossal duct cyst (TGDC) | cholangio-carcinoma |
| carcinoma, squamous cell, thyroid | cholangitis, primary sclerosing |
| carcinoma, squamous cell, urethra | chondroblastoma |
| carcinoma, squamous cell, vagina | chondroid syringoma, malignant (MCS) |
| carcinoma, squamous cell, vulvar | chondroma, malignant, pulmonary (in Carney's triad) |
| carcinoma, terminal duct | chondrosarcoma |
| carcinoma, testicular | chondrosarcoma, acral synovial |
| carcinoma, transitional cell | chondrosarcoma, classic, primary intradural |
| carcinoma, transitional cell, prostate | chondrosarcoma, clear cell |
| carcinoma, trichilemmal | chondrosarcoma, clear cell, larynx |
| chondrosarcoma, dural-based | dermatofibrosarcoma protuberans, fibrosarcomatous variant |
| chondrosarcoma, intracranial | dermatofibrosarcoma protuberans, NOS |
| chondrosarcoma, mesenchymal | dermatofibrosarcoma protuberans, pigmented |
| chondrosarcoma, mysoid, extraskeletal | desmoplastic, small round cell (DSRCT) |
| chordoma | dysembryoplastic neuroepithelial tumor (DNT) |
| chordoma, clivus | dysgerminoma |
| chordoma, familial | dysgerminoma, ovarian |
| chordoma, intracranial cavity | eccrine poroma, malignant |
| chordoma, NOS | eccrine spiradenoma, malignant |
| chordoma, perifericum | ectomesenchymoma, malignant |
| chordoma, sacrum | emlanoma, malignant, placenta |
| chordoma, skull base | endocrine tumor, pancreatic |
| chordoma, vertebrae | endodermal sinus tumor |
| choriocarcinoma | endometrioid tumor, ovary |
| choriocarcinoma, esophagus | ependymoma |
| choriocarcinoma, gastric | epithelial cancer, ovarian (EOC) |
| choriocarcinoma, ovary | epithelial tumor, appendiceal |
| choriocarcinoma, stomach | epithelial tumor, oral cavity |
| choriocarcinoma/male, primary, pulmonary | epithelioma cuniculatum |
| cutaneous malignant tumor | erythroleukemia |
| cylindroma, malignant | esthesioneuroblastoma |
| cylindroma, malignant, apocrine | fibrosarcoma |
| cystadenocarcinoma, acinar cell | fibrous histiocytoma, malignant |
| cystadenocarcinoma, mucinous | fibrous histiocytoma, malignant (MFH) |
| cystadenocarcinoma, pancreatic | fibrous histiocytoma, malignant, angiomatoid |
| cystadenocarcinoma, serous | fibrous histiocytoma, malignant, intracerebral |
| cystic-pseudopapillary tumor/ pancreas | fibrous histiocytoma, malignant, renal |
| cystosarcoma phyllodes, malignant, breast | fibrous tissue tumor, malignant |
| cystosarcoma phylloides | fibrous tumor, solitary, malignant |
| dermatofibrosarcoma protuberans (DFSP) | fibroxanthoma, atypical |
| follicular tumor | hemangiosarcoma |
| ganglioneuroblastoma | hepatoblastoma |
| gastrointestinal autonomic nerve tumor | hereditary non-polyposis colorectal cancer (HNPCC) |
| germ cell tumor | hidradenoma papilliferum, malignant |
| germ cell tumor, intracranial (GCTs) | histiocytoma |
| germ cell tumor, ovarian | histiocytosis, malignant |
| germ cell tumor, testicular (GCTS) | Hodgkin's disease |
| germinoma (seminoma) | Hodgkin's disease, bladder |
| germinoma, pineal | Hodgkin's disease, blood |
| gestational trophoblastic tumor | Hodgkin's disease, bone |
| giant cell tumor, nonendocrine | Hodgkin's disease, bone marrow |
| glioblastoma multiforme, spinal chord | Hodgkin's disease, breast |
| glioblastoma, giant cell | Hodgkin's disease, cardiovascular system |
| glioma | Hodgkin's disease, central nervous system |
| glioma, optic nerve | Hodgkin's disease, connective tissue disease |
| glomangiosarcoma | Hodgkin's disease, endocrine system |
| glomus tumor, malignant | Hodgkin's disease, gastrointestinal tract |
| glucagonoma syndrome | Hodgkin's disease, genitourinary |
| granular cell tumor, malignant | Hodgkin's disease, head & neck |
| granular cell tumor, malignant, larynx | Hodgkin's disease, kidney |
| granulosa cell tumor, ovary | Hodgkin's disease, lung |
| granulosa tumor, stromal cell | Hodgkin's disease, muscle |
| gynandroblastoma | Hodgkin's disease, neurological system |
| hamartoma, mesenchymal, liver (MHL) | Hodgkin's disease, prostate |
| hemangioendothelioma | Hodgkin's disease, reproductive system |
| hemangioendothelioma, epithelioid | Hodgkin's disease, respiratory system |
| hemangioendothelioma, spindle cell | Hodgkin's disease, skin |
| hemangioendothelioma, thyroid | Hodgkin's disease, testis |
| hemangioendotheliomas, epithelioid, pulmonary (PEH) | Hodgkin's disease, thymus |
| hemangiopericytoma (HEPC) | Hodgkin's disease, thyroid |
| hypokalemia & achlorhydria syndrome, well differentiated | leukemia, acute undifferentiated (AUL) |
| inflammatory myofibroblastic tumor (IMT) | leukemia, adult T-cell |
| inflammatory myofibroblastic tumor (IMT), pulmonary | leukemia, basophilic |
| insular papillary cancer, thyroid | leukemia, central nervous system |
| insulinoma, malignant | leukemia, chronic lymphocytic (CLL) |
| islet cell tumor, nonfunctioning | leukemia, chronic myelogenous (CML) |
| islet cell, pancreatic | leukemia, cutis |
| Krukenberg | leukemia, eosinophilic |
| Langerhans Cell Histiocytosis (LCH) | leukemia, extramedullary |
| leiomyoblastoma | leukemia, hairy cell (HCL) |
| leiomyomatosis, intravenous | leukemia, Hodgkin's cell |
| leiomyosarcoma | leukemia, lymphoblastic, t-cell, acute (ALL) |
| leiomyosarcoma, adrenal | leukemia, prolymphocytic, t-cell |
| leiomyosarcoma, epithelioid, gastric leiomyosarcoma, gastric | leukemia, promyelocytic |
| epithelioid | Leydig cell tumor (LCT) |
| leiomyosarcoma, esophagus | lipoastrocytoma |
| leiomyosarcoma, lung | lipoblastoma |
| leiomyosarcoma, oral cavity | liposarcoma |
| leiomyosarcoma, pancreas | liposarcoma, larynx |
| leiomyosarcoma, primary bone (PLMSB) | liposarcoma, myxoid |
| leiomyosarcoma, renal | liposarcoma, pleomorphic |
| leiomyosarcoma, superficial perineal | liposarcoma, primary mesenteric |
| leiomyosarcoma, uterine | liposarcoma, renal |
| leiomyosarcoma, vulva | liposarcoma, well-differentiated |
| leukemia, acute erythroblastic (FAB M6) | low malignant potential tumor, ovary (LMP) |
| leukemia, acute lymphocytic (ALL) | lymphoepithelioma, parotid gland |
| leukemia, acute monocytic | lymphoma, adrenal |
| leukemia, acute myeloid (AML) | lymphoma, angiocentric |
| leukemia, acute nonlymphocytic (ANLL) | lymphoma, angiotropic large cell |
| leukemia, acute nonlymphoblastic | lymphoma, B-cell |
| lymphoma, B-cell, low grade, liver | lymphoma, large cell, anaplastic |
| lymphoma, B-cell, salivary gland | lymphoma, larynx |
| lymphoma, bladder | lymphoma, lung |
| lymphoma, bone | lymphoma, lymphoblastic (LBL) |
| lymphoma, breast | lymphoma, MALT |
| lymphoma, breast, MALT-type | lymphoma, mantle cell |
| lymphoma, Burkitt's | lymphoma, mediterranean |
| lymphoma, cardiovascular system | lymphoma, muscle |
| lymphoma, central nervous system | lymphoma, nasal |
| lymphoma, cervix | lymphoma, neurological system |
| lymphoma, chest wall | lymphoma, non-Hodgkin's (NHL) |
| lymphoma, colorectal mucosa associated lymphoid tumor | lymphoma, non-Hodgkin's, breast |
| lymphoma, cutaneous B cell | lymphoma, non-Hodgkin's, extranodal localization |
| lymphoma, cutaneous T cell (CTCL) | lymphoma, non-Hodgkin's, larynx |
| lymphoma, diffuse large cell | lymphoma, non-Hodgkin's, pulmonary |
| lymphoma, duodenal | lymphoma, non-Hodgkin's, testis |
| lymphoma, endocrine | lymphoma, ocular |
| lymphoma, esophageal | lymphoma, oral |
| lymphoma, follicular | lymphoma, orbital |
| lymphoma, gall bladder | lymphoma, ovary |
| lymphoma, gastrointestinal tract | lymphoma, pancreatic lymphoma, pancreas |
| lymphoma, genital tract | lymphoma, paranasal sinus |
| lymphoma, head & neck | lymphoma, penile |
| lymphoma, heart | lymphoma, peripheral nervous system |
| lymphoma, hepatobilliary | lymphoma, pharynx |
| lymphoma, HIV-associated | lymphoma, pituitary |
| lymphoma, intravascular | lymphoma, primary breast |
| lymphoma, Ki-1 positive, anaplastic, large cell | lymphoma, primary central nervous system |
| lymphoma, kidney | lymphoma, primary lung |
| lymphoma, large bowel | lymphoma, prostate |
| lymphoma, pulmonary | melanoma, central nervous system |
| lymphoma, renal | melanoma, cervix |
| lymphoma, respiratory system | melanoma, choroidal |
| lymphoma, scrotum | melanoma, conjunctival |
| lymphoma, skin | melanoma, desmoplastic |
| lymphoma, small bowel | melanoma, endocrine |
| lymphoma, small intestine | melanoma, esophageal |
| lymphoma, soft tissue | melanoma, gall bladder |
| lymphoma, spermatic cord | melanoma, gastrointestinal tract |
| lymphoma, stomach | melanoma, genitourinary tract |
| lymphoma, t-cell (CTCL) | melanoma, head & neck |
| lymphoma, testicular | melanoma, heart |
| lymphoma, thyroid | melanoma, intraocular |
| lymphoma, trachea | melanoma, intraoral |
| lymphoma, ureter | melanoma, kidney |
| lymphoma, urethra | melanoma, larynx |
| lymphoma, urological system | melanoma, leptomeningeal |
| lymphoma, uterus | melanoma, lung |
| lymphomatosis, intravascular | melanoma, nasal mucosa |
| MALT tumor | melanoma, oral cavity |
| medulloblastoma | melanoma, osteoid forming/ osteogenic |
| melanoma, adrenal | melanoma, ovary |
| melanoma, amelanotic | melanoma, pancreas |
| melanoma, anal | melanoma, paranasal sinuses |
| melanoma, anorectal | melanoma, parathyroid |
| melanoma, biliary tree | melanoma, penis |
| melanoma, bladder | melanoma, pericardium |
| melanoma, brain | melanoma, pituitary |
| melanoma, breast | melanoma, placenta |
| melanoma, cardiopulmonary system | melanoma, prostate |
| melanoma, pulmonary | mixed mesodermal tumor (MMT) |
| melanoma, rectum | mucosa-associated lymphoid tissue (MALT) |
| melanoma, renal pelvis | Mullerian tumor, malignant mixed, fallopian tube |
| melanoma, sinonasal | Mullerian tumor, malignant mixed, uterine cervix |
| melanoma, skeletal system | myeloma, IgM |
| melanoma, small bowel | myoepithelioma |
| melanoma, small intestine | myoepithelioma, malignant, salivary gland |
| melanoma, spinal cord | nephroblastoma |
| melanoma, spleen | neuroblastoma |
| melanoma, stomach | neuroectodermal tumor, renal |
| melanoma, testis | neuroendocrine tumor, prostate |
| melanoma, thyroid | neurofibrosarcoma |
| melanoma, ureter | nodular hidradenoma, malignant |
| melanoma, urethra | oligodendroglioma |
| melanoma, uterus | oligodendroglioma, anaplastic |
| melanoma, vagina | oligodendroglioma, low-grade |
| melanoma, vulva | osteosarcoma |
| meningioma, malignant, anaplastic | Paget's disease, extramammary (EMPD) |
| meningioma, malignant, angioblastic | Paget's disease, mammary |
| meningioma, malignant, atypical | pancreatoblastoma |
| meningioma, malignant, papillary | paraganglioma, malignant |
| mesenchymal neoplasm, stromal | paraganglioma, malignant, extra-adrenal |
| mesenchymoma | paraganglioma, malignant, gangliocytic |
| mesoblastic nephroma | paraganglioma, malignant, laryngeal |
| mesothelioma, malignant | peripherial nerve sheath tumor, malignant (MPNST) |
| mesothelioma, malignant, pleura | pheochromocytoma, malignant |
| mesothelioma, papillary | phyllodes tumor, malignant, breast |
| mesothelioma/tunica vaginalis, malignant (MMTV) | pilomatrixoma, malignant |
| microadenocarcinoma, pancreatic | plasmacytoma, extramedullary (EMP) |
| mixed cell tumor, pancreatic | plasmacytoma, laryngeal |
| plasmacytoma, solitary | sarcoma, bladder |
| pleomorphic adenoma, malignant | sarcoma, botryoides |
| pleomorphic xanthoastrocytoma (PXA) | sarcoma, central nervous system |
| plexiform fibrohistiocytic tumor | sarcoma, clear cell, kidney |
| polyembryoma | sarcoma, clear cell, soft parts |
| polypoid glottic tumor | sarcoma, dendritic cell, follicular |
| primary lesions, malignant, diaphragm | sarcoma, endometrial stromal (ESS) |
| primary malignant lesions, chest wall | sarcoma, epithelioid |
| primary malignant lesions, pleura | sarcoma, Ewing's (EWS) |
| primary sinonasal nasopharyngeal undifferentiated (PSNPC) | sarcoma, Ewing's, extraosseus (EOE) |
| primitive neuroectodermal tumor (PNET) | sarcoma, Ewing's, primitive neuroectodermal tumor |
| proliferating trichilemmal tumor, malignant | sarcoma, fallopian tube |
| pseudomyxoma peritonei, malignant (PMP) | sarcoma, fibromyxoid |
| raniopharyngioma | sarcoma, granulocytic |
| reticuloendothelial tumor | sarcoma, interdigitating reticulum cell |
| retiforme hemangioendothelioma | sarcoma, intracerebral |
| retinoblastoma | sarcoma, intracranial |
| retinoblastoma, trilateral | sarcoma, Kaposi's |
| rhabdoid teratoma, atypical teratoid AT/RT | sarcoma, Kaposi's, intraoral |
| rhabdoid tumor, malignant | sarcoma, kidney |
| rhabdomyosarcoma (RMS) | sarcoma, mediastinum |
| rhabdomyosarcoma, orbital | sarcoma, meningeal |
| rhabdomyosarcoma, alveolar | sarcoma, neurogenic |
| rhabdomyosarcoma, botryoid | sarcoma, ovarian |
| rhabdomyosarcoma, central nervous system | sarcoma, pituitary |
| rhabdomyosarcoma, chest wall | sarcoma, pleomorphic soft tissue |
| rhabdomyosarcoma, paratesticular (PTR) | sarcoma, primary, lung |
| sarcoma, adult prostate gland | sarcoma, primary, pulmonar (PPS) |
| sarcoma, adult soft tissue | sarcoma, prostate |
| sarcoma, alveolar soft part (ASPS) | sarcoma, pulmonary arterial tree |
| sarcoma, renal | stromal cell tumor, sex cord |
| sarcoma, respiratory tree | stromal cell, testicular |
| sarcoma, soft tissue | stromal luteoma |
| sarcoma, stromal, gastrointestinal (GIST) | stromal myosis, endolymphatic (ESM) |
| sarcoma, stromal, ovarian | stromal tumor, colorectal |
| sarcoma, synovial | stromal tumor, gastrointestinal (GIST) |
| sarcoma, synovial, intraarticular | stromal tumor, gonadal (sex cord) (GSTS) |
| sarcoma, synovial, lung | stromal tumor, ovary |
| sarcoma, true | stromal tumor, small bowel |
| sarcoma, uterine | struma ovarii |
| sarcoma, vaginal | teratocarcinosarcoma, sinonasal (SNTCS) |
| sarcoma, vulvar | teratoma, immature |
| sarcomatosis, meningeal | teratoma, intramedullary spine |
| sarcomatous metaplasia | teratoma, mature |
| schwannoma, malignant | teratoma, pericardium |
| schwannoma, malignant, cellular, skin | teratoma, thyroid gland |
| schwannoma, malignant, epithelioid | thecoma stromal luteoma |
| schwannoma, malignant, esophagus | thymoma, malignant |
| schwannoma, malignant, nos | thymoma, malignant, medullary |
| Sertoli cell tumor, large cell, calcifying | thyroid/brain, anaplastic |
| sertoli-Leydig cell tumor (SLCT) | trichoblastoma, skin |
| small cell cancer, lungsmall cell lung cancer (SCLC) | triton tumor, malignant, nasal cavity |
| solid-pseudopapillary tumor, pancreas | trophoblastic tumor, fallopian tube |
| somatostinoma | trophoblastic tumor, placental site |
| spindle cell tumor | urethral cancer |
| spindle epithelial tumour w/thymus-like element | vipoma (islet cell) |
| spiradenocylindroma, kidney | vulvar cancer |
| squamous neoplasm, papillary | Waldenstrom's macroglobullinemia |
| steroid cell tumor | Wilms' tumor Nephroblastoma |
| Stewart-Treves syndrome | Wilms' tumor, lung |

**TABLE 3 - Exemplary Cancer Medications**

| | |
|---|---|
| | Cabazitaxel |
| | Campath (Alemtuzumab) |
| Abiraterone Acetate | Camptosar (Irinotecan Hydrochloride) |
| Abitrexate (Methotrexate) | Capecitabine |
| Adriamycin (Doxorubicin Hydrochloride) | Carboplatin |
| Adrucil (Fluorouracil) | Cerubidine (Daunorubicin Hydrochloride) |
| Afinitor (Everolimus) | Cervarix (Recombinant HPV Bivalent Vaccine) |
| Aldara (Imiquimod) | Cetuximab |
| Aldesleukin | Chlorambucil |
| Alemtuzumab | Cisplatin |
| Alimta (Pemetrexed Disodium) | Clafen (Cyclophosphamide) |
| Aloxi (Palonosetron Hydrochloride) | Clofarabine |
| Ambochlorin (Chlorambucil) | Clofarex (Clofarabine) |
| Amboclorin (Chlorambucil) | Clolar (Clofarabine) |
| Aminolevulinic Acid | Cyclophosphamide |
| Anastrozole | Cyfos (Ifosfamide) |
| Aprepitant | Cytarabine |
| Arimidex (Anastrozole) | Cytarabine, Liposomal |
| Aromasin (Exemestane) | Cytosar-U (Cytarabine) |
| Arranon (Nelarabine) | Cytoxan (Cyclophosphamide) |
| Arsenic Trioxide | Dacarbazine |
| Arzerra (Ofatumumab) | Dacogen (Decitabine) |
| Avastin (Bevacizumab) | Dasatinib |
| Azacitidine | Daunorubicin Hydrochloride |
| Bendamustine Hydrochloride | Decitabine |
| Bevacizumab | Degarelix |
| Bexarotene | Denileukin Diftitox |
| Bexxar (Tositumomab and I 131 Iodine Tositumomab) | Denosumab |
| Bleomycin | DepoCyt (Liposomal Cytarabine) |
| Bortezomib | DepoFoam (Liposomal Cytarabine) |
| Dexrazoxane Hydrochloride | Fulvestrant |
| Docetaxel | Gardasil (Recombinant HPV Quadrivalent Vaccine) |
| Doxorubicin Hydrochloride | Gefitinib |
| Efudex (Fluorouracil) | Gemcitabine Hydrochloride |
| Elitek (Rasburicase) | Gemtuzumab Ozogamicin |
| Ellence (Epirubicin Hydrochloride) | Gemzar (Gemcitabine Hydrochloride) |
| Eloxatin (Oxaliplatin) | Gleevec (Imatinib Mesylate) |
| Eltrombopag Olamine | Halaven (Eribulin Mesylate) |
| Emend (Aprepitant) | Herceptin (Trastuzumab) |
| Epirubicin Hydrochloride | HPV Bivalent Vaccine, Recombinant |
| Erbitux (Cetuximab) | HPV Quadrivalent Vaccine, Recombinant |
| Eribulin Mesylate | Hycamtin (Topotecan Hydrochloride) |
| Erlotinib Hydrochloride | Ibritumomab Tiuxetan |
| Etopophos (Etoposide Phosphate) | Ifex (Ifosfamide) |
| Etoposide | Ifosfamide |
| Etoposide Phosphate | Ifosfamidum (Ifosfamide) |
| Everolimus | Imatinib Mesylate |
| Evista (Raloxifene Hydrochloride) | Imiquimod |
| Exemestane | Ipilimumab |
| Fareston (Toremifene) | Iressa (Gefitinib) |
| Faslodex (Fulvestrant) | Irinotecan Hydrochloride |
| Femara (Letrozole) | Istodax (Romidepsin) |
| Filgrastim | Ixabepilone |
| Fludara (Fludarabine Phosphate) | Ixempra (Ixabepilone) |
| Fludarabine Phosphate | Jevtana (Cabazitaxel) |
| Fluoroplex (Fluorouracil) | Keoxifene (Raloxifene Hydrochloride) |
| Fluorouracil | Kepivance (Palifermin) |
| Folex (Methotrexate) | Lapatinib Ditosylate |
| Folex PFS (Methotrexate) | Lenalidomide |
| Folotyn (Pralatrexate) | Letrozole |
| Leucovorin Calcium | Ofatumumab |
| Leukeran (Chlorambucil) | Oncaspar (Pegaspargase) |
| Leuprolide Acetate | Ontak (Denileukin Diftitox) |
| Levulan (Aminolevulinic Acid) | Oxaliplatin |
| Linfolizin (Chlorambucil) | Paclitaxel |
| LipoDox (Doxorubicin Hydrochloride Liposome) | Palifermin |
| Liposomal Cytarabine | Palonosetron Hydrochloride |
| Lupron (Leuprolide Acetate) | Panitumumab |
| Lupron Depot (Leuprolide Acetate) | Paraplat (Carboplatin) |
| Lupron Depot-Ped (Leuprolide Acetate) | Paraplatin (Carboplatin) |
| Lupron Depot-3 Month (Leuprolide Acetate) | Pazopanib Hydrochloride |
| Lupron Depot-4 Month (Leuprolide Acetate) | Pegaspargase |
| Matulane (Procarbazine Hydrochloride) | Pemetrexed Disodium |
| Methazolastone (Temozolomide) | Platinol (Cisplatin) |
| Methotrexate | Platinol-AQ (Cisplatin) |
| Methotrexate LPF (Methotrexate) | Plerixafor |
| Mexate (Methotrexate) | Pralatrexate |
| Mexate-AQ (Methotrexate) | Prednisone |
| Mozobil (Plerixafor) | Procarbazine Hydrochloride |
| Mylosar (Azacitidine) | Proleukin (Aldesleukin) |
| Mylotarg (Gemtuzumab Ozogamicin) | Prolia (Denosumab) |
| Nanoparticle Paclitaxel (Paclitaxel Albumin-stabilized Nanoparticle | Promacta (Eltrombopag Olamine) |
| Formulation) | Provenge (Sipuleucel-T) |
| Nelarabine | Raloxifene Hydrochloride |
| Neosar (Cyclophosphamide) | Rasburicase |
| Neupogen (Filgrastim) | Recombinant HPV Bivalent Vaccine |
| Nexavar (Sorafenib Tosylate) | Recombinant HPV Quadrivalent Vaccine |
| Nilotinib | Revlimid (Lenalidomide) |
| Nolvadex (Tamoxifen Citrate) | Rheumatrex (Methotrexate) |
| Nplate (Romiplostim) | Rituxan (Rituximab) |
| Rituximab | Tositumomab and I 131 Iodine Tositumomab |
| Romidepsin | Totect (Dexrazoxane Hydrochloride) |
| Romiplostim | Trastuzumab |
| Rubidomycin (Daunorubicin Hydrochloride) | Treanda (Bendamustine Hydrochloride) |
| Sclerosol Intrapleural Aerosol (Talc) | Trisenox (Arsenic Trioxide) |
| Sipuleucel-T | Tykerb (Lapatinib Ditosylate) |
| Sorafenib Tosylate | Vandetanib |
| Sprycel (Dasatinib) | Vectibix (Panitumumab) |
| Sterile Talc Powder (Talc) | Velban (Vinblastine Sulfate) |
| Steritalc (Talc) | Velcade (Bortezomib) |
| Sunitinib Malate | Velsar (Vinblastine Sulfate) |
| Sutent (Sunitinib Malate) | VePesid (Etoposide) |
| Synovir (Thalidomide) | Viadur (Leuprolide Acetate) |
| Talc | Vidaza (Azacitidine) |
| Tamoxifen Citrate | Vinblastine Sulfate |
| Tarabine PFS (Cytarabine) | Vincasar PFS (Vincristine Sulfate) |
| Tarceva (Erlotinib Hydrochloride) | Vincristine Sulfate |
| Targretin (Bexarotene) | Vorinostat |
| Tasigna (Nilotinib) | Votrient (Pazopanib Hydrochloride) |
| Taxol (Paclitaxel) | Wellcovorin (Leucovorin Calcium) |
| Taxotere (Docetaxel) | Xeloda (Capecitabine) |
| Temodar (Temozolomide) | Xgeva (Denosumab) |
| Temozolomide | Yervoy (Ipilimumab) |
| Temsirolimus | Zevalin (Ibritumomab Tiuxetan) |
| Thalidomide | Zinecard (Dexrazoxane Hydrochloride) |
| Thalomid (Thalidomide) | Zoledronic Acid |
| Toposar (Etoposide) | Zolinza (Vorinostat) |
| Topotecan Hydrochloride | Zometa (Zoledronic Acid) |
| Toremifene | Zytiga (Abiraterone Acetate) |
| Torisel (Temsirolimus) | |

**TABLE 4 - Exemplary Ocular Diseases and Conditions**

| | | | |
|---|---|---|---|
| | | • | keratoconjunctivitis sicca (dry eye syndrome) |
| ***Examples of "back of the eye" diseases include*** | | • | iridocyclitis |
| • | macular edema such as angiographic cystoid macular | • | iritis |
| | edema | • | scleritis |
| • | retinal ischemia and choroidal neovascularization | • | episcleritis |
| • | macular degeneration | • | corneal edema |
| • | retinal diseases (e.g., diabetic retinopathy, diabetic | • | scleral disease |
| | retinal edema, retinal detachment); inflammatory | • | ocular cicatrcial pemphigoid |
| | diseases such as uveitis (including panuveitis) or | • | pars planitis |
| | choroiditis (including multifocal choroiditis) of | • | Posner Schlossman syndrome |
| | unknown cause (idiopathic) or associated with a | • | Behcet's disease |
| | systemic (e.g., autoimmune) disease; episcleritis or | • | Vogt-Koyanagi-Harada syndrome |
| | scleritis | • | hypersensitivity reactions |
| • | Birdshot retinochoroidopathy | • | conjunctival edema |
| • | vascular diseases (retinal ischemia, retinal vasculitis, | | |
| | choroidal vascular insufficiency, choroidal | • | conjunctival venous congestion |
| | thrombosis) | • | periorbital cellulitis; acute dacryocystitis |
| • | neovascularization of the optic nerve | • | non-specific vasculitis |
| • | optic neuritis | • | sarcoidosis |
| ***Examples of*** "***front***-***of***-***eye***" ***diseases include:*** | | | |
| • | blepharitis | | |
| • | keratitis | | |
| • | rubeosis iritis | | |
| • | Fuchs' heterochromic iridocyclitis | | |
| • | chronic uveitis or anterior uveitis | | |
| • | conjunctivitis | | |
| • | allergic conjunctivitis (including seasonal or perennial, vernal, atopic, and giant papillary) | | |

**TABLE 5 - Exemplary Ocular Medications**

| |
|---|
| Atropine |
| Brimondine (Alphagan) |
| Ciloxan |
| Erythromycin |
| Gentamicin |
| Levobunolol (Betagan) |
| Metipranolol (Optipranolol) |
| Optivar |
| Patanol |
| PredForte |
| Proparacaine |
| Timoptic |
| Trusopt |
| Visudyne (Verteporfin) |
| Voltaren |
| Xalatan |

**TABLE 6 - Exemplary Diseases and Conditions affecting the Lungs**

| | |
|---|---|
| Acute Bronchitis | Pulmonary Arterial Hypertension |
| Acute Respiratory Distress Syndrome (ARDS) | Pulmonary Fibrosis |
| Asbestosis | Pulmonary Vascular Disease |
| Asthma | Respiratory Syncytial Virus |
| Bronchiectasis | Sarcoidosis |
| Bronchiolitis | Severe Acute Respiratory Syndrome |
| Bronchopulmonary Dysplasia | Silicosis |
| Byssinosis | Sleep Apnea |
| Chronic Bronchitis | Sudden Infant Death Syndrome |
| Coccidioidomycosis (Cocci) | Tuberculosis |
| COPD | |
| Cystic Fibrosis | |
| Emphysema | |
| Hantavirus Pulmonary Syndrome | |
| Histoplasmosis | |
| Human Metapneumovirus | |
| Hypersensitivity Pneumonitis | |
| Influenza | |
| Lung Cancer | |
| Lymphangiomatosis | |
| Mesothelioma | |
| Nontuberculosis Mycobacterium | |
| Pertussis | |
| Pneumoconiosis | |
| Pneumonia | |
| Primary Ciliary Dyskinesia | |
| Primary Pulmonary Hypertension | |

**TABLE 7 - Exemplary Lung/Respiratory disease medications:**

| | |
|---|---|
| | Curosurf |
| | Daliresp (roflumilast) |
| Accolate | Dulera (mometasone furoate + formoterol fumarate dihydrate) |
| Accolate | DuoNeb (albuterol sulfate and ipratropium bromide) |
| Adcirca (tadalafil) | Dynabac |
| Aldurazyme (laronidase) | Flonase Nasal Spray |
| Allegra (fexofenadine hydrochloride) | Flovent Rotadisk |
| Allegra-D | Foradil Aerolizer (formoterol fumarate) inhalation powder) |
| Alvesco (ciclesonide) | Infasurf |
| Astelin nasal spray | Invanz |
| Atrovent (ipratropium bromide) | Iressa (gefitinib) |
| Augmentin (amoxicillin/clavulanate) | Ketek (telithromycin) |
| Avelox I.V. (moxifloxacin hydrochloride) | Letairis (ambrisentan) |
| Azmacort (triamcinolone acetonide) Inhalation Aerosol | Metaprotereol Sulfate Inhalation Solution, 5% |
| Biaxin XL (clarithromycin extended-release tablets) | Nasacort AQ (triamcinolone acetonide) Nasal Spray |
| Breathe Right | Nasacort AQ (triamcinolone acetonide) Nasal Spray |
| Brovana (arformoterol tartrate) | NasalCrom Nasal Spray |
| Cafcit Injection | OcuHist |
| Cayston (aztreonam for inhalation solution) | Omnicef |
| Cedax (ceftibuten) | Patanase (olopatadine hydrochloride) |
| Cefazolin and Dextrose USP | Priftin |
| Ceftin (cefuroxime axetil) | Proventil HFA Inhalation Aerosol |
| Cipro (ciprofloxacin HCl) | Pulmozyme (dornase alfa) |
| Clarinex | Pulmozyme (dornase alfa) |
| Claritin RediTabs (10 mg loratadine rapidly-disintegrating tablet) | Qvar (beclomethasone dipropionate) |
| Claritin Syrup (loratadine) | Raxar (grepafloxacin) |
| Claritin-D 24 Hour Extended Release Tablets (10 mg loratadine, | Remodulin (treprostinil) |
| 240 mg pseudoephedrine sulfate) | RespiGam (Respiratory Syncitial Virus Immune Globulin |
| Clemastine fumarate syrup | Intravenous) |
| Covera-HS (verapamil) | Rhinocort Aqua Nasal Spray |
| Sclerosol Intrapleural Aerosol | Zosyn (sterile piperacillin sodium/tazobactam sodium) |
| Serevent | Zyflo (Zileuton) |
| Singulair | Zyrtec (cetirizine HCl) |
| Spiriva HandiHaler (tiotropium bromide) | |
| Synagis | |
| Tavist (clemastine fumarate) | |
| Tavist (clemastine fumarate) | |
| Teflaro (ceftaroline fosamil) | |
| Tequin | |
| Tikosyn Capsules | |
| Tilade (nedocromil sodium) | |
| Tilade (nedocromil sodium) | |
| Tilade (nedocromil sodium) | |
| Tobi | |
| Tracleer (bosentan) | |
| Tri-Nasal Spray (triamcinolone acetonide spray) | |
| Tripedia (Diptheria and Tetanus Toxoids and Acellular Pertussis | |
| Vaccine Absorbed) | |
| Tygacil (tigecycline) | |
| Tyvaso (treprostinil) | |
| Vancenase AQ 84 mcg Double Strength | |
| Vanceril 84 mcg Double Strength (beclomethasone dipropionate, 84 | |
| mcg) Inhalation Aerosol | |
| Ventolin HFA (albuterol sulfate inhalation aerosol) | |
| Visipaque (iodixanol) | |
| Xolair (omalizumab) | |
| Xopenex | |
| Xyzal (levocetirizine dihydrochloride) | |
| Zagam (sparfloxacin) tablets | |
| Zemaira (alphal-proteinase inhibitor) | |

**TABLE 8 - Exemplary Diseases and Conditions affecting the Heart:**

| |
|---|
| Heart attack |
| Atherosclerosis |
| High blood pressure |
| Ischemic heart disease |
| Heart rhythm disorders |
| Tachycardia |
| Heart murmurs |
| Rheumatic heart disease |
| Pulmonary heart disease |
| Hypertensive heart disease |
| Valvular heart disease |
| Infective endocarditis |
| Congenital heart diseases |
| Coronary heart disease |
| Atrial myxoma |
| HOCM |
| Long QT syndrome |
| Wolff Parkinson White syndrome |
| Supraventricular tachycardia |
| Atrial flutter |
| Constrictive pericarditis |
| Atrial myxoma |
| Long QT syndrome |
| Wolff Parkinson White syndrome |
| Supraventricular tachycardia |
| Atrial flutter |

**TABLE 9 - Exemplary Heart Medications**

| | |
|---|---|
| | nadolol, Corgard |
| | niacin and lovastatin, Advicor |
| ACE Inhibitors | niacin, Niacor, Niaspan, Slo-Niacin |
| acetylsalicylic acid, Aspirin, Ecotrin | nitroglycerin, Nitro-Bid, Nitro-Dur, Nitrostat, Transderm- |
| alteplase, Activase, TPA | Nitro, Minitran, Deponit, Nitrol |
| anistreplase-injection, Eminase | oxprenolol-oral |
| Aspirin and Antiplatelet Medications | pravastatin, Pravachol |
| atenolol, Tenormin | pravastatin/buffered aspirin-oral, Pravigard PAC |
| atorvastatin, Lipitor | propranolol, Inderal, Inderal LA |
| benazepril, Lotensin | quinapril hcl/hydrochlorothiazide-oral, Accuretic |
| Beta Blockers | quinapril, Accupril |
| Bile Acid Sequestrants | ramipril, Altace |
| Calcium Channel Blockers | reteplase-injection, Retavase |
| captopril and hydrochlorothiazide, Capozide | simvastatin, Zocor |
| captopril, Capoten | Statins |
| clopidogrel bisulfate, Plavix | streptokinase-injection, Kabikinase, Streptase |
| colesevelam, Welchol | torsemide-oral, Demadex |
| dipyridamole-oral, Persantine | trandolapril, Mavik |
| enalapril and hydrochlorothiazide, Vaseretic | |
| enalapril, Vasotec | |
| ezetimibe and simvastatin, Vytorin | |
| Fibrates | |
| fluvastatin, Lescol | |
| fosinopril sodium, Monopril | |
| lisinopril and hydrochlorothiazide, Zestoretic, Prinzide | |
| lisinopril, Zestril, Prinivil | |
| lovastatin, Mevacor, Altocor | |
| magnesium sulfate-injection | |
| metoprolol, Lopressor, Toprol XL | |
| moexipril-oral, Univasc | |

**TABLE 10 - Exemplary Bacterial, Viral, Fungal and Parasitic Conditions**

| ***Bacterial Infections caused by:*** | |
|---|---|
| • | Borrelia species |
| • | Streptococcus pneumoniae |
| • | Staphylococcus aureus |
| • | Mycobacterium tuberculosis |
| • | Mycobacterium leprae |
| • | Neisseria gonorrheae |
| • | Chlamydia trachomatis |
| • | Pseudomonas aeruginosa |
| | |

| ***Viral Infections caused by:*** | |
|---|---|
| • | Herpes simplex |
| • | Herpes zoster |
| • | cytomegalovirus |
| | |

| ***Fungal Infections caused by:*** | |
|---|---|
| • | Aspergillus fumigatus |
| • | Candida albicans |
| • | Histoplasmosis capsulatum |
| • | Cryptococcus species |
| • | Pneumocystis carinii |
| | |

| ***Parasitic Infections caused by:*** | |
|---|---|
| • | Toxoplasmosis gondii |
| • | Trypanosome cruzi |
| • | Leishmania species |
| • | Acanthamoeba species |
| • | Giardia lamblia |
| • | Septata species |
| • | Dirofilaria immitis |

## Claims

1. A method for nanoencapsulation of a plurality of drugs comprising:
separately linking each of the plurality of drugs with a corresponding polymer backbone with nearly 100% loading efficiency by forming the corresponding polymer backbone by ring opening polymerization beginning with the corresponding drug, wherein each of the corresponding polymer backbones has the same or similar physicochemical properties and has approximately the same chain length;
mixing the plurality of linked drugs and polymers at selectively predetermined ratios at selectively and precisely controlled drug ratios; and
synthesizing the mixed plurality of linked drugs and polymers into a nanoparticle.

2. The method of claim 1, wherein the plurality of drugs are independently selected from the group consisting of an antibiotic, antimicrobial, growth factor, chemotherapeutic agent, and combinations thereof.

3. The method of claims 1 to 2, wherein the plurality of drugs comprise an anticancer chemotherapeutic agent.

4. The method of claim 1 to 2, wherein the plurality of drugs are independently selected from the group consisting of doxorubicin, camptothecin, gemcitabine, carboplatin, oxaliplatin, epirubicin, idarubicin, carminomycin, daunorubicin, aminopterin, methotrexate, methopterin, dichloromethotrexate, mitomycin C, porfiromycin, 5-fluorouracil, 6-mercaptopurine, cytosine arabinoside, podophyllotoxin, etoposide, etoposide phosphate, melphalan, vinblastine, vincristine, leurosidine, vindesine, estramustine, cisplatin, cyclophosphamide, paclitaxel, leurositte, 4-desacetylvinblastine, epothilone B, docetaxel, maytansinol, epothilone A, combretastatin, pharmaceutically active analogs thereof, and pharmaceutically acceptable salts thereof.

5. The method of claim 1, wherein the polymer backbone is a stimuli-sensitive linker.

6. The method of claim 5, wherein the linker is a pH-sensitive linker.

7. The method of claim 5, wherein the stimuli-sensitive linker is selected from the group consisting of C₁-C₁₀ straight chain alkyl, C₁-C₁₀ straight chain O-alkyl, C₁-C₁₀ straight chain substituted alkyl, C₁-C₁₀ straight chain substituted O-alkyl, C₄-C₁₃ branched chain alkyl, C₄-C₁₃ branched chain O-alkyl, C₂-C₁₂ straight chain alkenyl, C₂-C₁₂ straight chain O-alkenyl, C₃-C₁₂ straight chain substituted alkenyl, C₃-C₁₂ straight chain substituted O-alkenyl, polyethylene glycol, polylactic acid, polyglycolic acid, poly(lactide-co-glycolide), polycarprolactone, polycyanoacrylate, ketone, aryl, aralkyl, heterocyclic, and combinations thereof.

8. The method of claims 1 to 7, wherein said nanoparticle is a lipid-polymer hybrid nanoparticle with a polymeric core consisting of the plurality of linked drugs and polymers.

9. The method of claim 8, wherein said nanoparticle is a lipid-coated polymeric nanoparticle.

10. The method of claims 1 to 9, wherein the nanoparticle is about 1 nm to about 10 µm in diameter.

11. The method of claim 10, wherein the nanoparticle is about 30 nm to about 300 nm in diameter.

## Patentansprüche

1. Verfahren zur Nanoeinkapselung mehrerer Arzneimittel, umfassend:
separates Verbinden jedes der mehreren Arzneimittel mit einem entsprechenden Polymergerüst mit nahezu 100 % Beladungseffizienz durch Bilden des entsprechenden Polymergerüsts durch Ringöffnungspolymerisation, die mit dem entsprechenden Arzneimittel beginnt, worin jedes der entsprechenden Polymergerüste die gleichen oder ähnliche physikochemischen Eigenschaften aufweist und ungefähr die gleiche Kettenlänge aufweist;
Mischen der mehreren verbundenen Arzneimittel und Polymere in ausgewählten vorherbestimmten Verhältnissen unter ausgewählten und genau kontrollierten Arzneimittelverhältnissen; und
Synthetisieren der gemischten mehreren verbundenen Arzneimittel und Polymere in ein Nanoteilchen.

2. Verfahren nach Anspruch 1, worin die mehreren Arzneimittel unabhängig ausgewählt sind aus der Gruppe, bestehend aus einem Antibiotikum, antimikrobiellen Mittel, einem Wachstumsfaktor, einem chemotherapeutischen Mittel und Kombinationen davon.

3. Verfahren nach Anspruch 1 oder 2, worin die mehreren Arzneimittel ein Antkrebschemotherapiemittel umfassen.

4. Verfahren nach Anspruch 1 bis 2, worin die mehreren Arzneimittel unabhängig ausgewählt sind aus der Gruppe, bestehend aus Doxorubicin, Camptothecin, Gemcitabin, Carboplatin, Oxaliplatin, Epirubicin, Idarubicin, Carminomycin, Daunorubicin, Aminopterin, Methotrexat, Methopterin, Dichlormethotrexat, Mitomycin C, Porfiromycin, 5-Fluoruracil, 6-Mercaptopurin, Cytosinarbinosid, Podophyllotoxin, Etoposid, Etoposidphosphat, Melphalan, Vinblastin, Vincristin, Leurosidin, Vindesin, Estramustin, Cisplatin, Cyclophospamid, Paclitaxel, Leurositte, 4-Desacetylvinblastin, Epothilon B, Docetaxel, Maytansinol, Epothilon A, Combretastatin, pharmazeutisch aktiven Analogen davon und pharmazeutisch verträgliche Salze davon.

5. Verfahren nach Anspruch 1, worin das Polymergerüst ein Stimulisensitiver Linker ist.

6. Verfahren nach Anspruch 5, worin der Linker ein pH-sensitiver Linker ist.

7. Verfahren nach Anspruch 5, worin der Stimuli-sensitive Linker ausgewählt ist aus der Gruppe, bestehend aus geradkettigem C₁-C₁₀-Alkyl, geradkettigem C₁-C₁₀-O-Alkyl, geradkettigem substituiertem C₁-C₁₀-Alkyl, geradkettigem substituiertem C₁-C₁₀-O-Alkyl, verzweigtkettigem C₄-C₁₃-Alkyl, verzweigtkettigem C₄-C₁₃-O-Alkyl, geradkettigem C₂-C₁₂-Alkenyl, geradkettigem C₂-C₁₂-O-Alkenyl, geradkettigem substituiertem C₃-C₁₂-Alkenyl, geradkettigem substituiertem C₃-C₁₂-O-Alkenyl, Polyethylenglykol, Polymilchsäure, Polyglykolsäure, Poly(lactid-co-glycolid), Polycaprolacton, Polycyanacrylat, Keton, Aryl, Aralkyl, heterozyklisch und Kombinationen davon.

8. Verfahren nach den Ansprüchen 1 bis 7, worin das Nanoteilchen ein Lipid-Polymer-Hybrid-Nanoteilchen mit einem polymeren Kern, bestehend aus den mehreren verbundenen Arzneimitteln und Polymeren, ist.

9. Verfahren nach Anspruch 8, worin das Nanoteilchen ein Lipdbeschichtetes polymeres Nanoteilchen ist.

10. Verfahren nach einem der Ansprüche 1 bis 9, worin das Nanoteilchen einen Durchmesser von etwa 1 nm bis etwa 10 µm aufweist.

11. Verfahren nach einem der Ansprüche 1 bis 9, worin das Nanoteilchen einen Durchmesser von etwa 30 nm bis etwa 300 nm aufweist.

## Revendications

1. Procédé pour la nanoencapsulation d'une pluralité de médicaments comprenant:
la liaison séparément de chacun de la pluralité de médicaments avec un squelette polymère correspondant avec sensiblement 100 % d'efficacité de chargement par formation du squelette polymère correspondant par polymérisation par ouverture de cycle commençant avec le médicament correspondant, où chacun des squelettes polymères correspondants a des propriétés physicochimiques identiques ou similaires et a approximativement la même longueur de chaîne;
le mélange de la pluralité de médicaments et de polymères liés à des rapports sélectivement prédéterminés à des rapports de médicaments sélectivement et précisément contrôlés; et
la synthèse de la pluralité mélangée de médicaments et de polymères liés en une nanoparticule.

2. Procédé selon la revendication 1 où la pluralité de médicaments est choisie indépendamment dans le groupe consistant en un antibiotique, un antimicrobien, un facteur de croissance, un agent chimiothérapeutique et leurs combinaisons.

3. Procédé selon les revendications 1 à 2 où la pluralité de médicaments comprend un agent chimiothérapeutique anticancéreux.

4. Procédé selon les revendications 1 à 2 où la pluralité de médicaments est choisie indépendamment dans le groupe consistant en la doxorubicine, la camptothécine, la gemcitabine, le carboplatine, l'oxaliplatine, l'épirubicine, l'idarubicine, la carminomycine, la daunorubicine, l'aminoptérine, le méthotrexate, la méthoptérine, le dichlorométhotrexate, la mitomycine C, la porfiromycine, le 5-fluorouracile, la 6-mercaptopurine, l'arabinoside de cytosine, la podophyllotoxine, l'étoposide, l'étoposide phosphate, le melphalan, la vinblastine, la vincristine, la leurosidine, la vindésine, l'estramustine, le cisplatine, le cyclophosphamide, le paclitaxel, la leurositte, la 4-désacétylvinblastine, l'épothilone B, le docétaxel, le maytansinol, l'épothilone A, la combrétastatine, leurs analogues pharmaceutiquement actifs et leurs sels pharmaeeutiquement acceptables.

5. Procédé selon la revendication 1 où le squelette polymère est un lieur sensible aux stimuli.

6. Procédé selon la revendication 5 où le lieur est un lieur sensible au pH.

7. Procédé selon la revendication 5 où le lieur sensible aux stimuli est choisi dans le groupe consistant en C₁-C₁₀ alkyle linéaire, C₁-C₁₀ O-alkyle linéaire, C₁-C₁₀ alkyle linéaire substitué, C₁-C₁₀ O-alkyle linéaire substitué, C₄-C₁₃ alkyle ramifié, C₄-C₁₃ O-alkyle ramifié, C₂-C₁₂ alcényle linéaire, C₂-C₁₂ O-alcényle linéaire, C₃-C₁₂ alcényle linéaire substitué, C₃-C₁₂ O-alcényle linéaire substitué, le polyéthylèneglycol, le poly(acide lactique), le poly(acide glycolique), un copolymère lactide-glycolide, la polycaprolactone, un polycyanoacrylate, une cétone, un aryle, aralkyle, hétérocyclique, et leurs combinaisons.

8. Procédé selon les revendications 1 à 7 où ladite nanoparticule est une nanoparticule hybride lipide-polymère avec un noyau polymérique consistant en la pluralité de médicaments et de polymères liés.

9. Procédé selon la revendication 8 où ladite nanoparticule est une nanoparticule polymérique à enrobage lipidique.

10. Procédé selon les revendications 1 à 9 où la nanoparticule a un diamètre d'environ 1 nm à environ 10 µm.

11. Procédé selon la revendication 10 où la nanoparticule a un diamètre d'environ 30 nm à environ 300 nm.
